# EUROPEAN PATENT APPLICATION

(11) **EP 4 506 368 A1**
(43) Date of publication of application: **12.02.2025**
(21) Application number: 23784816.3
(22) Date of filing: 07.04.2023
(51) Int. Cl.: C07K 16/46, A61K 39/395, A61P 35/00, A61P 35/02, A61P 37/04, C07K 16/28, C12Q 1/06, G01N 33/574, C12N 15/13, C12P 21/08

(54) **BISPECIFIC ANTIBODY TO HUMAN TIM-3 AND HUMAN CD39, AND APPLICATIONS THEREOF**

(30) Priority: 07.04.2022 JP 2022063998
(71) Applicant: Brightpath Biotherapeutics Co., Ltd., Kawasaki-shi, Kanagawa 210-0821 (JP)
(72) Inventor: NAKAMURA, Norihiro, Kawasaki-shi, Kanagawa 210-0821 (JP); NAKAJIMA, Kanto, Kawasaki-shi, Kanagawa 210-0821 (JP); MISHIMA, Yuji, Kawasaki-shi, Kanagawa 210-0821 (JP); MATSUMOTO Noriko, Kawasaki-shi, Kanagawa 210-0821 (JP)
(74) Representative: Plasseraud IP
(86) International application number: PCT/JP2023/014336
(87) International publication number: WO 2023/195532

(57) **Abstract**

The purpose of the present invention is to produce an antibody or antibody derivative having a higher immune checkpoint inhibitory effect. More specifically, the purpose of the present invention is to produce an antibody or antibody derivative having a higher immune checkpoint inhibitory effect, and having an action of enhancing immune activity against cancer cells, and a cancer treatment effect. The present invention has demonstrated that the purpose can be achieved by providing a heterodimeric antibody or antibody derivative having a binding ability to TIM-3 antigen and a binding ability to CD39 antigen. More specifically, the present invention provides a heterodimeric antibody or antibody derivative having a binding ability to TIM-3 antigen and a binding ability to CD39 antigen, and having an action of enhancing immune activity against cancer cells.

## Description

### Technical field

The present invention relates to a heterodimeric antibody or antibody derivative having a binding ability to both TIM-3 antigen expressed on dendritic cells, macrophage, NK cells, activated T cells, cancer cells and the like and CD39 antigen expressed on various immune cells and non-immune cells (endothelial cells, fibroblast cells and the like), and more particularly to an antibody or antibody derivative having a binding ability to both the TIM-3 antigen and the CD39 antigen and having an ability to enhance immune activity against cancer cells, and a composition and a method for the treatment of cancer and the diagnosis of cancer that comprise the antibody or antibody derivative.

### Background Art

Cancer cells actively utilize the immunosuppressive function of immune checkpoint molecules, in addition to regulatory T cells (Treg) and myeloid-derived suppressor cells (MDSC), to avoid attacks from the immune system. Various immune checkpoint molecules and their ligands have been identified.

Immune checkpoint inhibitors inhibit the binding between immune checkpoint molecules and their ligands, to inhibit the transmission of immunosuppressive signals, thereby releasing the suppression of T cell activation by immune checkpoint molecules on cancer cells, and can reactivate the immune function and generate an immune response against the cancer cells. By utilizing this mechanism, immune checkpoint inhibitors treat cancer through a different approach from conventional anticancer drugs and molecular target drugs.

So far, several immune checkpoint inhibitors have been approved as pharmaceuticals of an inhibitor of PD-1/PD-L1 including nivolumab and pembrolizumab (anti-PD-1 antibodies), atezolizumab, and durvalumab (anti-PD-L1 antibodies), as well as pharmaceuticals of an inhibitor of CTLA-4/B7 (CD80/CD86) including ipilimumab (anti-CTLA-4 antibody). Drugs comprising these antibodies as a primary component demonstrate high efficacy in some cancer types when used alone or in combination with other anticancer agents.

However, the response rate of existing immune checkpoint inhibitors is reported to be approximately 5-30%. It has been revealed that the individual differences in the immune status of the patient's cancer relate to the effectiveness of immune checkpoint inhibitors. The individual differences in cancer immune status are considered to be influenced by the genetic abnormalities of cancer cells as a main cause, in combination with factors such as the patient's immune constitution (genetic background, such as HLA type) and various environmental factors (such as intestinal bacteria, smoking, ultraviolet radiation, diet, stress), and, as a result, the existing immune checkpoint inhibitors are unable to address certain patients.

TIM-3 (also known as HAVCR2) is recognized as one of the immune checkpoint molecules. TIM-3 belongs to the T cell Ig and mucin domain-containing molecule superfamily. TIM-3 is an essential regulatory molecule in T cell tolerance and plays a crucial role in autoimmunity and T cell exhaustion during chronic viral infections (Non Patent Literature 1).

TIM-3 is a cell-surface receptor that is involved in regulation of innate immunity and adaptive immune responses, which is known as a molecule that is generally considered to be suppressive, and TIM-3 is expressed on macrophage, NK cells, Th1 cells (type 1 T helper cells), dendritic cells, CD8+ T cells and other lymphocyte subsets. TIM-3 interacts with a lot of ligands such as Phosphatidylserine (PS), CEACAM1 (Carcinoembryonic antigen-related cell adhesion molecule 1), Galectin-9, and HMGB1 (High mobility group box 1), and it is considered that the function of TIM-3 may vary depending on cells and binding ligands. Among them, the TIM-3-Galectin-9 pathway negatively regulates Th1 cell (type 1 T helper cell) immunity to enhance immune tolerance for Th1 cells (Non Patent Literature 2), and therefore inhibition of TIM-3 is expected to enhance innate and adaptive immunity.

For adaptive immune responses, TIM-3 is reported to inhibit T cell receptor (TCR)-induced signal transmission in CD8+ T cells to suppress cytokine production (Non Patent Literatures 3 and 4). Binding of TIM-3 to Galectin-9 is reported to suppress T cell responses to induce apoptosis of antigen-specific T cells (Non

### Patent Literatures 3 and 5).

For cancer immunity, TIM-3 is highly expressed on tumor-infiltrating T cells, and recognized as one of the markers representing an exhaustion state of T cells as described above. The TIM-3 antibody has been found to exhibit a synergistic antitumor effect when combined with the PD-1 antibody in syngeneic mouse models.

TIM-3 is also expressed on immune cells other than T cells, and recently, it has been indicated that TIM-3 expressed on dendritic cells may play an essential role in regulation of the function of dendritic cells by regulating activation of inflammasome, and negatively regulate antitumor immunity (Non

### Patent Literature 6).

CD39 (also known as Ectonucleoside Triphosphate Diphosphohydrolase 1 (ENTPD1)) is recognized as a substance considered to function as another immune checkpoint molecule. CD39 is a transmembrane protein, having two transmembrane domains and one extracellular domain. CD39 has the enzymic activity of binding to extracellular ATP (eATP) and hydrolyzing the ATP into AMP (Non Patent Literature 7).

CD39 is expressed on various immune cells and non-immune cells (endothelial cells, fibroblast cells and the like), and expression of CD39 has been shown to increase in intratumor immune cells. On the other hand, ATP released from stressed cells or dying cells provides inflammatory signals essential for effective innate immunity and adaptive immune responses, and adenosine generated by extracellular hydrolysis of eATP, generated mainly through cascades of CD39 and CD73 and released out of cells, suppresses immune responses (Non Patent Literature 8).

The release of ATP from dying tumor cells is a characteristic of immunological cell death, and has been extensively demonstrated to enhance antitumor immune responses (Non Patent Literature 9). Activation of P2XR7 and NLRP3 inflammasomes and production of IL-1beta in dendritic cells are required to stimulate antitumor T cell immunity, but exhaustion of ATP generated by CD39 has been shown to disable the antitumor activity of immunogenic chemotherapy (Non Patent Literature 10).

### Citation List

### Non Patent Literature

Non Patent Literature 1: Isabel Sada-Ovalle, et al., J. Immunol., 2012, 189(12): 5896-902
Non Patent Literature 2: Sanchez-Fueyo, A., Nat. Immunol. 2003 Nov; 4(11):1093-101
Non Patent Literature 3: Clayton, KL., J. Immunol. 2014 Jan 15; 192(2) :782-91
Non Patent Literature 4: Tomkowicz, B., PLoS One. 2015 Oct 22; 10(10):e0140694
Non Patent Literature 5: Zhu, C., Nat Immunol. 2005 Dec; 6(12):1245-52
Non Patent Literature 6: Dixon, KO., Nature volume 595, pages 101-106 (2021)
Non Patent Literature 7: Kaczmarek, E., J Biol Chem. 1996 Dec 20; 271(51):33116-22
Non Patent Literature 8: Moesta, AK., Nat. Rev. Immunol. 20, pages 739-755 (2020)
Non Patent Literature 9: Kroemer, G., Annu. Rev. Immunol. 31, 51-72 (2013)
Non Patent Literature 10: Mascanfroni, ID., Nat. Immunol. 14, 1054-1063 (2013)

### Summary of Invention

### Technical problem

The present invention is directed to produce an antibody or antibody derivative having a more effective immune checkpoint inhibitory effect. More specifically, the present invention is directed to produce an antibody or antibody derivative having a more effective immune checkpoint inhibitory effect, and having an ability to enhance immune activity against cancer cells, and a cancer treatment effect.

### Solution to Problem

The present inventors have demonstrated that the above-described problem can be solved by providing a heterodimeric antibody or antibody derivative having a binding ability to TIM-3 antigen and a binding ability to CD39 antigen. More specifically, the present invention provides a heterodimeric antibody or antibody derivative having a binding ability to TIM-3 antigen and a binding ability to CD39 antigen, and having an ability to enhance immune activity against cancer cells.

More specifically, the present application provides the following embodiments to solve the problem aforementioned:
[1]: A heterodimeric antibody or antibody derivative that is a combination of two different half-molecules, one of which has a binding ability to Tim-3 antigen and the other of which has a binding ability to CD39 antigen;
[2]: The antibody or antibody derivative according to [1], wherein the antibody or antibody derivative has an ability to enhance immune activity against cancer cells;
[3]: The antibody or antibody derivative according to [1] or [2], wherein the half-molecule having the binding ability to TIM-3 antigen is a half-molecule of the antibody or a half-molecule of the antibody derivative comprising either set of the complementarity-determining regions of the heavy chain and the light chain selected from the group consisting of:
   (1-1) Complementarity-determining regions of the heavy chain, CDR1 (SYYMS, SEQ ID NO: 1), CDR2 (TISNSGGSTYYPDSVKD, SEQ ID NO: 2), and CDR3 (DPYYTNYVPMDY, SEQ ID NO: 3, and
   complementarity-determining regions of the light chain, CDR1 (KASQYVDTYVA, SEQ ID NO: 4), CDR2 (SASTRHT, SEQ ID NO: 5), and CDR3 (AQYSSSPLT, SEQ ID NO: 6);
   (1-2) Complementarity-determining regions of the heavy chain, CDR1 (DYYMD, SEQ ID NO: 9), CDR2 (YIYPNKGGTSYNQKFKG, SEQ ID NO: 10), and CDR3 (SGYGNYYTMDY, SEQ ID NO: 11), and
   complementarity-determining regions of the light chain, CDR1 (KASQSVGNNVA, SEQ ID NO: 12), CDR2 (YASNRYT, SEQ ID NO: 13), and CDR3 (QQHYSSPST, SEQ ID NO: 14);
   (1-3) Complementarity-determining regions of the heavy chain, CDR1 (DYYMD, SEQ ID NO: 17), CDR2 (YIYPNKGGTSYNQKFKG, SEQ ID NO: 18), and CDR3 (GGYYSYYSYDY, SEQ ID NO: 19), and
   complementarity-determining regions of the light chain, CDR1 (KASQSVGNNVA, SEQ ID NO: 20), CDR2 (YASNRYT, SEQ ID NO: 21), and CDR3 (QQHYSSPYT, SEQ ID NO: 22);
   (1-4) Complementarity-determining regions of the heavy chain, CDR1 (DYYMD, SEQ ID NO: 25), CDR2 (YIYPNKGGTSYNQKFKG, SEQ ID NO: 26), and CDR3 (SGYKAYYAMDY, SEQ ID NO: 27), and
   complementarity-determining regions of the light chain, CDR1 (KASQSVGNNVA, SEQ ID NO: 28), CDR2 (YASNRYT, SEQ ID NO: 29), and CDR3 (QQHYSSPYT, SEQ ID NO: 30);
   (1-5) Complementarity-determining regions of the heavy chain, CDR1 (SYYMS, SEQ ID NO: 33), CDR2 (TISNSGGSTYYPDSVKD, SEQ ID NO: 34), and CDR3 (DPYYTNYVPMDY, SEQ ID NO: 35), and
   complementarity-determining regions of the light chain, CDR1 (KASENVGTYVS, SEQ ID NO: 36), CDR2 (GASNRYT, SEQ ID NO: 37), and CDR3 (GQSYSYPLT, SEQ ID NO: 38);
[4]: The antibody or antibody derivative according to [1] or [2], wherein the half-molecule having the binding ability to CD39 antigen is a half-molecule of the antibody or a half-molecule of the antibody derivative comprising either set of the complementarity-determining regions of the heavy chain and the light chain selected from the group consisting of:
   (2-1) Complementarity-determining regions of the heavy chain, CDR1 (GFSIKDTY, SEQ ID NO: 41), CDR2 (IDPANVNT, SEQ ID NO: 42), and CDR3 (ALYGYDDDAYYFDY, SEQ ID NO: 43), and
   complementarity-determining regions of the light chain, CDR1 (ESVDNYGISF, SEQ ID NO: 44), CDR2 (AAS, SEQ ID NO: 45), and CDR3 (QQSKEVPYT, SEQ ID NO: 46);
   (2-2) Complementarity-determining regions of the heavy chain, CDR1 (GYSFTDYN, SEQ ID NO: 49), CDR2 (IDPYSGGT, SEQ ID NO: 50), and CDR3 (GLYGYDDDANYFDD, SEQ ID NO: 51), and
   complementarity-determining regions of the light chain, CDR1 (SSVSY, SEQ ID NO: 52), CDR2 (ATS, SEQ ID NO: 53), and CDR3 (QQRSSYPLT, SEQ ID NO: 54);
   (2-3) Complementarity-determining regions of the heavy chain, CDR1 (NYGVH, SEQ ID NO: 57), CDR2 (VILRRGSTDYNAAFMS, SEQ ID NO: 58), and CDR3 (TAVVAGDYFDY, SEQ ID NO: 59), and
   complementarity-determining regions of the light chain, CDR1 (KASQNVGTNVA, SEQ ID NO: 60), CDR2 (SASYRYS, SEQ ID NO: 61), and CDR3 (QQYNSYPLT, SEQ ID NO: 62);
   (2-4) Complementarity-determining regions of the heavy chain, CDR1 (NYGMN, SEQ ID NO: 65), CDR2 (WINTYTGEPTYADDFKG, SEQ ID NO: 66), and CDR3 (KGYYGYPNYYAMDY, SEQ ID NO: 67), and
   complementarity-determining regions of the light chain, CDR1 (KASQNVGTAVA, SEQ ID NO: 68), CDR2 (SASNRYT, SEQ ID NO: 69), and CDR3 (QQYSSYPIT, SEQ ID NO: 70);
   (2-5) Complementarity-determining regions of the heavy chain, CDR1 (SYGVH, SEQ ID NO: 73), CDR2 (VIWRRGSTDYNAAFMS, SEQ ID NO: 74), and CDR3 (GISTATSWFAY, SEQ ID NO: 75), and
   complementarity-determining regions of the light chain, CDR1 (KASQNVGTNVA, SEQ ID NO: 76), CDR2 (SASYRYS, SEQ ID NO: 77), and CDR3 (QQYNSYPLT, SEQ ID NO: 78);
   (2-6) Complementarity-determining regions of the heavy chain, CDR1 (SYGVH, SEQ ID NO: 81), CDR2 (VIWRRGSTDYNAAFMS, SEQ ID NO: 82), and CDR3 (VRGDAMDY, SEQ ID NO: 83), and
   complementarity-determining regions of the light chain, CDR1 (KASQNVGTNVA, SEQ ID NO: 84), CDR2 (SASYRYS, SEQ ID NO: 85), and CDR3 (QQYNSYPLT, SEQ ID NO: 86);
   (2-7) Complementarity-determining regions of the heavy chain, CDR1 (DYGMH, SEQ ID NO: 89), CDR2 (YISSGSSIIYYADTVKG, SEQ ID NO: 90), and CDR3 (KDYPYAMDY, SEQ ID NO: 91), and
   complementarity-determining regions of the light chain, CDR1 (KASQNVGTNVA, SEQ ID NO: 92), CDR2 (WASNRFT, SEQ ID NO: 93), and CDR3 (QQYSSSPYT, SEQ ID NO: 94);
[5]: The antibody or antibody derivative according to claim 3, wherein the half-molecule having the binding ability to TIM-3 antigen is the half-molecule of (1-1) or (1-5);
[6]: The antibody or antibody derivative according to [4], wherein the half-molecule having the binding ability to CD39 antigen is any one of the half-molecules of (2-1), and (2-3)-(2-7);
[7]: The antibody or antibody derivative according to [1] or [2], wherein the amino acid sequence of the heavy chain variable region VH domain of the half-molecule having the binding ability to TIM-3 antigen is selected from the group consisting of:
   (VH-1-1) the amino acid sequence of SEQ ID No: 7, or an amino acid sequence comprising one or a few amino acid substitutions (e.g., conservative substitutions), insertions, or deletions in regions of the amino acid sequence of SEQ ID No: 7 other than CDR1 (SEQ ID No: 1), CDR2 (SEQ ID No: 2), and CDR3 (SEQ ID No: 3);
   (VH-1-2) the amino acid sequence of SEQ ID No: 15, or an amino acid sequence comprising one or a few amino acid substitutions (e.g., conservative substitutions), insertions, or deletions in regions of the amino acid sequence of SEQ ID No: 15 other than CDR1 (SEQ ID NO: 9), CDR2 (SEQ ID NO: 10), and CDR3 (SEQ ID NO: 11);
   (VH-1-3) the amino acid sequence of SEQ ID No: 23, or an amino acid sequence comprising one or a few amino acid substitutions (e.g., conservative substitutions), insertions, or deletions in regions of the amino acid sequence of SEQ ID No: 23 other than CDR1 (SEQ ID NO: 17), CDR2 (SEQ ID NO: 18), and CDR3 (SEQ ID NO: 19);
   (VH-1-4) the amino acid sequence of SEQ ID No: 31, or an amino acid sequence comprising one or a few amino acid substitutions (e.g., conservative substitutions), insertions, or deletions in regions of the amino acid sequence of SEQ ID No: 31 other than CDR1 (SEQ ID NO: 25), CDR2 (SEQ ID NO: 26), and CDR3 (SEQ ID NO: 27);
   (VH-1-5) the amino acid sequence of SEQ ID No: 39, or an amino acid sequence comprising one or a few amino acid substitutions (e.g., conservative substitutions), insertions, or deletions in regions of the amino acid sequence of SEQ ID No: 39 other than CDR1 (SEQ ID NO: 33), CDR2 (SEQ ID NO: 34), and CDR3 (SEQ ID NO: 35);
[8]: The antibody or antibody derivative according to [1] or [2], wherein the amino acid sequence of the light chain variable region VL domain of the half-molecule having the binding ability to TIM-3 is selected from the group consisting of;
   (VL-1-1) the amino acid sequence of SEQ ID No: 8, or an amino acid sequence comprising one or a few amino acid substitutions (e.g., conservative substitutions), insertions, or deletions in regions the amino acid sequence of SEQ ID No: 8 other than CDR1 (SEQ ID No: 4), CDR2 (SEQ ID No: 5), and CDR3 (SEQ ID No: 6);
   (VL-1-2) the amino acid sequence of SEQ ID No: 16, or an amino acid sequence comprising one or a few amino acid substitutions (e.g., conservative substitutions), insertions, or deletions in regions the amino acid sequence of SEQ ID No: 16 other than CDR1 (SEQ ID NO: 12), CDR2 (SEQ ID NO: 13), and CDR3 (SEQ ID NO: 14);
   (VL-1-3) the amino acid sequence of SEQ ID No: 24, or an amino acid sequence comprising one or a few amino acid substitutions (e.g., conservative substitutions), insertions, or deletions in regions the amino acid sequence of SEQ ID No: 24 other than CDR1 (SEQ ID NO: 20), CDR2 (SEQ ID NO: 21), and CDR3 (SEQ ID NO: 22);
   (VL-1-4) the amino acid sequence of SEQ ID No: 32, or an amino acid sequence comprising one or a few amino acid substitutions (e.g., conservative substitutions), insertions, or deletions in regions the amino acid sequence of SEQ ID No: 32 other than CDR1 (SEQ ID NO: 28), CDR2 (SEQ ID NO: 29), and CDR3 (SEQ ID NO: 30);
   (VL-1-5) the amino acid sequence of SEQ ID No: 40, or an amino acid sequence comprising one or a few amino acid substitutions (e.g., conservative substitutions), insertions, or deletions in regions the amino acid sequence of SEQ ID No: 40 other than CDR1 (SEQ ID NO: 36), CDR2 (SEQ ID NO: 37), and CDR3 (SEQ ID NO: 38);
[9]: The antibody or antibody derivative according to [1] or [2], wherein the half-molecule having the binding ability to TIM-3 antigen of the antibody derivative is selected from the following variable regions of the half-molecules which consitute the single chain Fv antibodies (scFv):
   (1-1) an amino acid sequence in which the heavy chain variable region of (VH-1-1) and the light chain variable region of (VL-1-1) are connected via a linker;
   (1-2) an amino acid sequence in which the heavy chain variable region of (VH-1-2) and the light chain variable region of (VL-1-2) are connected via a linker;
   (1-3) an amino acid sequence in which the heavy chain variable region of (VH-1-3) and the light chain variable region of (VL-1-3) are connected via a linker;
   (1-4) an amino acid sequence in which the heavy chain variable region of (VH-1-4) and the light chain variable region of (VL-1-4) are connected via a linker;
   (1-5) an amino acid sequence in which the heavy chain variable region of (VH-1-5) and the light chain variable region of (VL-1-5) are connected via a linker.
[10]: The antibody or antibody derivative according to [1] or [2], wherein the amino acid sequence of the heavy chain variable region VH domain of the half-molecule having the binding ability to CD39 antigen is selected from the group consisting of:
   (VH-2-1) the amino acid sequence of SEQ ID No: 47, or an amino acid sequence comprising one or a few amino acid substitutions (e.g., conservative substitutions), insertions, or deletions in regions of the amino acid sequence of SEQ ID No: 47 other than CDR1 (SEQ ID NO: 41), CDR2 (SEQ ID NO: 42), and CDR3 (SEQ ID NO: 43);
   (VH-2-2) the amino acid sequence of SEQ ID No: 55, or an amino acid sequence comprising one or a few amino acid substitutions (e.g., conservative substitutions), insertions, or deletions in regions of the amino acid sequence of SEQ ID No: 55 other than CDR1 (SEQ ID NO: 49), CDR2 (SEQ ID NO: 50), and CDR3 (SEQ ID NO: 51);
   (VH-2-3) the amino acid sequence of SEQ ID No: 123, or an amino acid sequence comprising one or a few amino acid substitutions (e.g., conservative substitutions), insertions, or deletions in regions of the amino acid sequence of SEQ ID No: 123 other than CDR1 (SEQ ID NO: 57), CDR2 (SEQ ID NO: 58), and CDR3 (SEQ ID NO: 59);
   (VH-2-4) the amino acid sequence of SEQ ID No: 71, or an amino acid sequence comprising one or a few amino acid substitutions (e.g., conservative substitutions), insertions, or deletions in regions of the amino acid sequence of SEQ ID No: 71 other than CDR1 (SEQ ID NO: 65), CDR2 (SEQ ID NO: 66), and CDR3 (SEQ ID NO: 67);
   (VH-2-5) the amino acid sequence of SEQ ID No: 79, or an amino acid sequence comprising one or a few amino acid substitutions (e.g., conservative substitutions), insertions, or deletions in regions of the amino acid sequence of SEQ ID No: 79 other than CDR1 (SEQ ID NO: 73), CDR2 (SEQ ID NO: 74), and CDR3 (SEQ ID NO: 75);
   (VH-2-6) the amino acid sequence of SEQ ID No: 87, or an amino acid sequence comprising one or a few amino acid substitutions (e.g., conservative substitutions), insertions, or deletions in regions of the amino acid sequence of SEQ ID No: 87 other than CDR1 (SEQ ID NO: 81), CDR2 (SEQ ID NO: 82), and CDR3 (SEQ ID NO: 83);
   (VH-2-7) the amino acid sequence of SEQ ID No: 95, or an amino acid sequence comprising one or a few amino acid substitutions (e.g., conservative substitutions), insertions, or deletions in regions of the amino acid sequence of SEQ ID No: 95 other than CDR1 (SEQ ID NO: 89), CDR2 (SEQ ID NO: 90), and CDR3 (SEQ ID NO: 91).
[11]: The antibody or antibody derivative according to [1] or [2], wherein the amino acid sequence of the light chain variable region VL domain of the half-molecule having the binding ability to CD39 is selected from the group consisting of;
   (VL-2-1) the amino acid sequence of SEQ ID No: 48, or an amino acid sequence comprising one or a few amino acid substitutions (e.g., conservative substitutions), insertions, or deletions in regions the amino acid sequence of SEQ ID No: 48 other than CDR1 (SEQ ID NO: 44), CDR2 (SEQ ID NO: 45), and CDR3 (SEQ ID NO: 46);
   (VL-2-2) the amino acid sequence of SEQ ID No: 56, or an amino acid sequence comprising one or a few amino acid substitutions (e.g., conservative substitutions), insertions, or deletions in regions the amino acid sequence of SEQ ID No: 56 other than CDR1 (SEQ ID NO: 52), CDR2 (SEQ ID NO: 53), and CDR3 (SEQ ID NO: 54);
   (VL-2-3) the amino acid sequence of SEQ ID No: 124, or an amino acid sequence comprising one or a few amino acid substitutions (e.g., conservative substitutions), insertions, or deletions in regions the amino acid sequence of SEQ ID No: 124 other than CDR1 (SEQ ID NO: 60), CDR2 (SEQ ID NO: 61), and CDR3 (SEQ ID NO: 62);
   (VL-2-4) the amino acid sequence of SEQ ID No: 72, or an amino acid sequence comprising one or a few amino acid substitutions (e.g., conservative substitutions), insertions, or deletions in regions the amino acid sequence of SEQ ID No: 72 other than CDR1 (SEQ ID NO: 68), CDR2 (SEQ ID NO: 69), and CDR3 (SEQ ID NO: 70);
   (VL-2-5) the amino acid sequence of SEQ ID No: 80, or an amino acid sequence comprising one or a few amino acid substitutions (e.g., conservative substitutions), insertions, or deletions in regions the amino acid sequence of SEQ ID No: 80 other than CDR1 (SEQ ID NO: 76), CDR2 (SEQ ID NO: 77), and CDR3 (SEQ ID NO: 78);
   (VL-2-6) the amino acid sequence of SEQ ID No: 88, or an amino acid sequence comprising one or a few amino acid substitutions (e.g., conservative substitutions), insertions, or deletions in regions the amino acid sequence of SEQ ID No: 88 other than CDR1 (SEQ ID NO: 84), CDR2 (SEQ ID NO: 85), and CDR3 (SEQ ID NO: 86);
   (VL-2-7) the amino acid sequence of SEQ ID No: 96, or an amino acid sequence comprising one or a few amino acid substitutions (e.g., conservative substitutions), insertions, or deletions in regions the amino acid sequence of SEQ ID No: 96 other than CDR1 (SEQ ID NO: 92), CDR2 (SEQ ID NO: 93), and CDR3 (SEQ ID NO: 94).
[12]: The antibody or antibody derivative according to [1] or [2], wherein the half-molecule having the binding ability to CD39 antigen of the antibody derivative is selected from the following variable regions of the half-molecules which constitute the single chain Fv antibodies (scFv):
   (2-1) an amino acid sequence in which the heavy chain variable region of (VH-2-1) and the light chain variable region of (VL-2-1) are connected via a linker;
   (2-2) an amino acid sequence in which the heavy chain variable region of (VH-2-2) and the light chain variable region of (VL-2-2) are connected via a linker;
   (2-3) an amino acid sequence in which the heavy chain variable region of (VH-2-3) and the light chain variable region of (VL-2-3) are connected via a linker;
   (2-4) an amino acid sequence in which the heavy chain variable region of (VH-2-4) and the light chain variable region of (VL-2-4) are connected via a linker;
   (2-5) an amino acid sequence in which the heavy chain variable region of (VH-2-5) and the light chain variable region of (VL-2-5) are connected via a linker;
   (2-6) an amino acid sequence in which the heavy chain variable region of (VH-2-6) and the light chain variable region of (VL-2-6) are connected via a linker;
   (2-7) an amino acid sequence in which the heavy chain variable region of (VH-2-7) and the light chain variable region of (VL-2-7) are connected via a linker.
[13]: The antibody or antibody derivative according to [1] or [2], wherein the antibody derivative is selected from a modified antibody selected from a humanized antibody, a chimeric antibody, a single chain Fv antibody (a scFv antibody), Fab, Fab', F(ab')2, Fc effector function modified antibody, IgG1LALA, IgG1_N297A, IgG4_S228P or functional fragments thereof.
[14]: The antibody or antibody derivative according to [13], wherein the enhancement of immune activity is activation of T cells selected from the group consisting of removal of immunosuppressive signals, relieving T cell exhaustion, proliferation of T cells, increase in cytotoxicity of T cells against cancer cells, promotion of T cell cytokine secretion, enhancement of tumor infiltration of activated T cells, promotion of dendritic cell (DC) maturation, enhancement of dendritic cell-mediated activation of antigen-specific T cells, and enhancement of dendritic cell-mediated tumor cytotoxic activity of T cells.
[15]: The antibody or antibody derivative according to [13], wherein the antibody or antibody derivative induces cytotoxicity against cancer cells but not against normal cells.
[16]: The antibody or antibody derivative according to [13], wherein the cancer cell is selected from the group consisting of blood cancer, melanoma, breast cancer, lung cancer, colon cancer, gastric cancer, pancreatic cancer, and liver cancer.
[17]: A pharmaceutical composition for treating cancer, comprising a heterodimeric antibody or antibody derivative that is a combination of two different half-molecules, one of which has a binding ability to the Tim-3 antigen and the other of which has a binding ability to the CD39 antigen.
[18]: The pharmaceutical composition according to [17], wherein the cancer is selected from the group consisting of blood cancer, melanoma, breast cancer, lung cancer, colon cancer, gastric cancer, pancreatic cancer, and liver cancer.
[19]: A method for measuring cytotoxicity against cancer cells, comprising
   a step of bringing cancer cells collected from a subject into contact in vitro with a heterodimeric antibody or antibody derivative that is a combination of two different half-molecules, one of which has a binding ability to the Tim-3 antigen and the other of which has a binding ability to the CD39 antigen, and
   a step of measuring whether the cell viability of the cancer cells is decreased or a step of measuring whether the secretion of immune-activating substances is enhanced under culture conditions.
[20]: The method according to [19], wherein whether cell viability of cancer cells is decreased or whether immune cells derived from peripheral blood lymphocytes are activated, is measured in the presence of peripheral blood lymphocytes from the same subject.
[21]: The method according to [19] or [20], wherein the enhancement of cytotoxicity against the cancer cells in vitro when a heterodimeric antibody or antibody derivative that is a combination of two different half-molecules, one of which has a binding ability to the Tim-3 antigen and the other of which has a binding ability to the CD39 antigen is administered to the subject is measured based on the in vitro cytotoxicity against cancer cells collected from the subject.
[22]: The method according to [19] or [20], wherein the enhancement of cytotoxicity against cancer cells when a heterodimeric antibody or antibody derivative that is a combination of two different half-molecules, one of which has a binding ability to the Tim-3 antigen and the other of which has a binding ability to the CD39 antigen is administered to the subject is measured based on the enhancement of secretion of an immune activating substance in vitro.
[23]: A measurement kit comprising a heterodimeric antibody or antibody derivative that is a combination of two different half-molecules, one of which has a binding ability to the Tim-3 antigen and the other of which has a binding ability to the CD39 antigen, for measuring in vitro cytotoxicity against cancer cells from a subject, secretion of immune activating substances against the cancer cells, or activation of immune cells against cancer cells of the antibody or antibody derivative.

### Advantageous Effects of Invention

The antibody or antibody derivative (hereinafter simply referred to as "antibody etc.") obtained according to the present invention has a binding ability to both TIM-3 antigen, expressed on cancer cells or immune cells, and CD39 antigen, expressed on various immune cells and non-immune cells (endothelial cells, fibroblast cells and the like), suppresses the function of TIM-3 antigen as well as the function of CD39 antigen, and thus exhibits an effect to enhance immune activity against cancer cells and a cancer treatment effect.

### Brief Description of Drawings

[Figure 1] Figure 1 shows a procedure for evaluating whether an anti-human TIM-3/CD39 bispecific antibody of the present invention binds simultaneously to both targets (human TIM-3 and human CD39), by interaction analysis of surface plasmon resonance (SPR) using Biacore8K.
[Figure 2] Figure 2 shows the results of surface plasmon resonance (SPR) using Biacore8K for evaluating whether the anti-human TIM-3/CD39 bispecific antibody of the present invention binds simultaneously to both targets (human TIM-3 and human CD39).
[Figure 3-1] Figure 3-1 shows the results of flow cytometry analysis of the binding selectivity of the anti-human TIM-3/CD39 bispecific antibody of the present invention to cells expressing any or both of two antigens: human TIM-3 and human CD39.
[Figure 3-2] Figure 3-2 shows the results of flow cytometry analysis of the binding selectivity of the anti-human TIM-3/CD39 bispecific antibody of the present invention to cells expressing any or both of two antigens: human TIM-3 and human CD39.
[Figure 4-1] Figure 4-1 shows the results of ELISA analysis of the ability of the anti-human TIM-3/CD39 bispecific antibody of the present invention to inhibit the binding between human TIM-3 and human Galectin-9.
[Figure 4-2] Figure 4-2 shows the results of ELISA analysis of the ability of the anti-human TIM-3/CD39 bispecific antibody of the present invention to inhibit the binding between human TIM-3 and human Galectin-9.
[Figure 4-3] Figure 4-3 shows the results of ELISA analysis of the ability of the anti-human TIM-3/CD39 bispecific antibody of the present invention to inhibit the binding between human TIM-3 and human Galectin-9.
[Figure 5-1] Figure 5-1 shows the results of flow cytometry analysis of the ability of the anti-human TIM-3/CD39 bispecific antibody of the present invention to inhibit the binding between human TIM-3 and Phosphatidylserine.
[Figure 5-2] Figure 5-2 shows the results of flow cytometry analysis of the ability of the anti-human TIM-3/CD39 bispecific antibody of the present invention to inhibit the binding between human TIM-3 and Phosphatidylserine.
[Figure 5-3] Figure 5-3 shows the results of flow cytometry analysis of the ability of the anti-human TIM-3/CD39 bispecific antibody of the present invention to inhibit the binding between human TIM-3 and Phosphatidylserine.
[Figure 6] Figure 6 shows the results of evaluating the ability of the anti-human TIM-3/CD39 bispecific antibody to inhibit human CD39 activity.
[Figure 7-1] Figure 7-1 shows the results of evaluating the effects of the anti-human TIM-3/CD39 bispecific antibody of the present invention on activation of human peripheral blood T cells by examining activation of PBMCs by SEB (staphylococcal enterotoxin B).
[Figure 7-2] Figure 7-2 shows the results of evaluating the effects of the anti-human TIM-3/CD39 bispecific antibody of the present invention on activation of human peripheral blood T cells by examining activation of PBMCs by SEB (staphylococcal enterotoxin B).
[Figure 8-1] Figure 8-1 shows, as the effects of the anti-human TIM-3/CD39 bispecific antibody of the present invention on maturation/activation of monocyte-derived dendritic cells (MDDC), the results of analyzing the expression distributions of HLA-DR, CD86 and CD83 molecules in MDDC with CD14 negative/CD1a positive characteristics after addition of various antibodies.
[Figure 8-2] Figure 8-2 shows, as the effects of the anti-human TIM-3/CD39 bispecific antibody of the present invention on maturation/activation of monocyte-derived dendritic cells (MDDC), the results of analyzing the expression distributions of HLA-DR, CD86 and CD83 molecules in MDDC with CD14 negative/CD1a positive characteristics after addition of various antibodies.
[Figure 9] Figure 9 shows, as the effects of the anti-human TIM-3/CD39 bispecific antibody of the present invention on maturation/activation of monocyte-derived dendritic cells (MDDC), the results of the concentrations of TNFalpha, IL-1beta, IL-6, CXCL9, CXCL10 and CXCL11 in the culture supernatant of MDDC treated for 24 hours after addition of various antibodies using Luminex.
[Figure 10] Figure 10 shows, as the effects of the anti-human TIM-3/CD39 bispecific antibody of the present invention on activation of CD8T cells through MDDC, the results of the concentrations of IFNgamma and TNFalpha in the culture supernatant of MDDC treated for 6 days after addition of various antibodies using Luminex.
[Figure 11] Figure 11 shows, as the effect of the anti-human TIM-3/CD39 bispecific antibody of the present invention on the antitumor activity in a co-culture system of PBMCs and tumor cells, the result of a change in the number of tumor cells with time in evaluation of anti-human TIM-3/CD39 bispecific antibodies using three types of PBMCs from different donors.
[Figure 12] Figure 12 shows, as the effect of the anti-human TIM-3/CD39 bispecific antibody of the present invention on the antitumor activity in a co-culture system of PBMCs and tumor cells, the result of a change in the number of tumor cells with time in evaluation of other anti-human TIM-3/CD39 bispecific antibodies.
[Figure 13] Figure 13 shows the results of evaluating the effects of the anti-human TIM-3/CD39 bispecific antibody of the present invention on proliferation of human peripheral blood T cells in tumor microenvironments by examining proliferation of T cells by CD3/CD28 antibody stimulation in the presence of ATP.
[Figure 14-1] Figure 14-1 shows the results of evaluating the effects of the anti-human TIM-3/CD39 bispecific antibody of the present invention on activation of inflammasome in M1-like macrophage.
[Figure 14-2] Figure 14-2 shows the results of evaluating the effects of the anti-human TIM-3/CD39 bispecific antibody of the present invention on activation of inflammasome in MDDC.

### Description of Embodiments

As described above, TIM-3 and CD39 are both considered to function as immune checkpoint molecules, and some cancer treatment antibodies targeting each of the antigens are under preclinical development or clinical development.

TIM-3 is a substance, the expression of which increases in T cells that are exhausted and become positive for expression of PD-1, and antibodies capable of inhibiting binding of a ligand to TIM-3 are expected to release the exhaustion of cytotoxic T cells. However, major preclinical tests reported so far on anti-TIM-3 antibodies indicate that administration of the TIM-3 antibody alone exhibits a limited inhibitory effect on tumors except for some bone-marrow hematopoietic cell tumors expressing TIM-3, and combined use of such antibodies with another immune checkpoint inhibitor such as a PD-1 antibody, and the like, is necessary for obtaining potent drug efficacy against common cancer typified by solid cancer.

On the other hand, it is suggested that CD39 has the enzymatic activity of hydrolyzing ATP, and has a suppressive effect on the antitumor activity of cytotoxic T cells by catalyzing a decomposing cascade from ATP into adenosine, which suppresses the function of T cells, in tumor microenvironments with an increased concentration of extracellular ATP. An antibody that binds to CD39 and suppresses ATPase activity is expected to avoid suppression of cytotoxic T cells by decreasing the concentration of adenosine in tumor microenvironments, but drug efficacy of a CD39 antibody agent alone is limited upon administration against a lot of tumors that does not express CD39, except for tumors highly expressing CD39, such as multiple myeloma.

Under such circumstances, the present inventors have demonstrated that the problem to be addressed by the present invention can be solved by providing a heterodimeric antibody or antibody derivative having a binding ability to TIM-3 antigen and a binding ability to CD39 antigen.

More specifically, the present invention, as a first embodiment, provides a heterodimeric antibody or antibody derivative that is a combination of two different half-molecules, one of which has a binding ability to TIM-3 antigen and the other of which has a binding ability to CD39 antigen (hereinafter, referred to simply as an "antibody etc."). The antibody etc. is a heterodimeric antibody having two types of antibody half-molecules.

The heterodimeric antibody etc. of the present invention, which
- eliminates signals suppressing immunity against cancer cells, to enhance immune activity against the cancer cells, resulting in inhibition of proliferation of cancer cells, and
- decreases the amount of adenosine generated by CD39 antigen of immune cells from ATP released from dying tumor cells under attacks, and cancels the suppression of immune responses by the adenosine, resulting in inhibition of proliferation of cancer cells,
can be used to treat cancer cells, to shrink/eliminate the tumor, and as well as to activate T cells toxic to cancer cells when the T cells are exhausted.

When simply referring to "antibody" in the present invention, the antibody may be derived from any species of mammals, and the species from which the antibody is derived are not limited to humans, but may be derived from mouse, rat, guinea pig, hamster, rabbit, VHH antibody (variable domain of heavy chain of heavy chain antibodies) derived from camelid animal (such as camel, and alpaca), ostrich antibodies, and so on. For example, in the case of the human antibody, this includes various isotypes such as IgG, IgM, IgA, IgD, IgE, etc.

In this embodiment of the present invention, an "antibody derivative" of the above antibody may also be used as long as it has the functional characteristics of binding to TIM-3 antigen and binding to CD39 antigen, and enhancing immune activity against cancer cells.

When referring to the antibody derivative in the present invention, the present invention may provide, for example, as an embodiment, an antibody derivative having the amino acid sequence of six CDRs (complementarity determining regions CDR1-CDR3 of the heavy chain and CDR1-CDR3 of the light chain) for each of the half-molecules of the above heterodimeric antibody, and the amino acid sequence of the human antibody-derived constant regions, with other amino acid sequences comprising the amino acid sequence derived from the original antibody combined with the amino acid sequence derived from the human antibody. Examples of the antibody derivative include, but are not limited to, a humanized antibody in which the amino acid sequences other than the complementarity determining regions (CDRs) of the above-described "antibody" are replaced with the amino acid sequences derived from a human antibody, a chimeric antibody such as those in which the variable region of the above antibody is linked to the constant regions of a human antibody, and a single chain antibody (e.g., scFv) in which the heavy chain and light chain are linked by a linker.

The heavy chain variable region and the light chain variable region in scFv may be linked so that either one is on the N-terminal side, and, in other words, the order of these regions may be, from N-terminal side to C-terminal side, heavy chain variable region-linker-light chain variable region or light chain variable region-linker-heavy chain variable region. The linker connecting between the heavy chain variable region and the light chain variable region may be any linker known in the art for use in the field. For example, in the examples described later in this application, GGGGSx4 (i.e., a linker composed of four consecutive GGGGS amino acid sequences) can be used.

The scFv antibodies exemplified here can be used as a form of a single chain, but a dimeric structure may be prepared by using two scFv-Fcs each of which is produced by binding the Fc region of the antibody commonly used in the field of the art, to the scFv via a linker. In this case, for example, the Fc region can be attached to the C-terminus of the scFv via a linker (e.g., a linker consisting of GGGG).

In the present invention, the antibody derivatives of the present invention also include functional fragments of the above-described antibody etc. and modified antibodies thereof. Functional fragments of the antibody etc. in the present invention include F(ab')2, Fab', Fab, single chain Fv (scFv), and so on. Modified antibodies include Fc effector function modified antibody, IgG1LALA, IgG1_N297A, IgG4_S228P, and so on.

When referring to "half-molecule" of an antibody etc. in the present invention, it means a single molecule or a fragment thereof when the bond between the heavy chains in the antibody etc. is dissociated. In one example, a half-molecule of an antibody etc., is a combination of the heavy chain (H chain) and light chain (L chain) of an antibody derived from various isotypes such as IgG, IgM, IgA, IgD, and IgE. For example, among these, in the case where an antibody etc. is an IgG antibody, a half-molecule is, for example, a complex consisting of one H chain of IgG and one L chain of IgG. The half-molecule of the antibody etc. may also include those derived from an antibody derivative, such as a chimeric antibody or a humanized antibody, as described above.

Further, the half-molecules of the antibody etc. may include any types of the half-molecules of an antibody etc. other than those mentioned above, such as a molecule consisting of a single H chain which is prepared by dissociating a bond between two H chains of an antibody consisting of two H chains found in camelids, etc., so-called a heavy-chain (H-chain) antibody (also called as a VHH (VH originating from heavy-chain antibody)) (these are collectively called a "single domain antibody"), a single chain Fv molecule (scFv molecule) that can be linked to another scFv molecule, a molecule in which an additional Fc region is added to a single-chain Fv molecule (scFv-Fc molecule), a molecule with a Knob structure for applying Knob-into-hole technology to the Fc domain of the scFv-Fc molecule, a molecule with a Hole structure for applying Knob-into-hole technology to the Fc domain of the scFv-Fc molecule and the like.

That is, the heterodimeric antibody etc. of the present invention is an antibody etc. constituted with a combination of a half-molecule of an antibody etc. having a binding ability to the TIM-3 antigen and a half-molecule of an antibody etc. having a binding ability to the CD39 antigen (i.e., a bispecific antibody). In this case, as the structure of the antibody etc., it is preferred that the half-molecule of the antibody etc. having a binding ability to the TIM-3 antigen is the half-molecule of the antibody etc. identified by the amino acid sequence of a total of six portions that are CDR1-3 of the heavy chain and CDR1-3 of the light chain, each of which have a specific sequence disclosed herein, and the half-molecule of the antibody etc. having a binding ability to the CD39 antigen is the half-molecule of the antibody etc. identified by the amino acid sequence of a total of six portions that are CDR1-3 of the heavy chain and CDR1-3 of the light chain, each of which have a specific sequence disclosed herein. It is possible to form an antibody etc. that can simultaneously achieve binding to the TIM-3 antigen by the half-molecule of the antibody etc. having a binding ability to the TIM-3 antigen and binding to the CD39 antigen by the half-molecule of the antibody etc. having a binding ability to the CD39 antigen.

Half-molecule having binding ability to TIM-3 antigen constituting heterodimeric antibody etc. of the present invention

The half-molecule having a binding ability to the TIM-3 antigen constituting the heterodimeric antibody etc. in this embodiment of the present invention is characterized as those, by way of example, by having the amino acid sequences of a total of six portions that are CDR1-3 of the heavy chain and CDR1-3 of the light chain, which are identical to those of the antibody having a binding ability to the TIM-3 antigen. Examples of the antibody etc. having a binding ability to the TIM-3 antigen of the present invention may include those constituting the half-molecule which contain either set of the complementarity-determining regions of the heavy chain and the light chain, selected from the group consisting of (1-1) to (1-5) below:
(1-1) Complementarity-determining regions of the heavy chain, CDR1 (SYYMS, SEQ ID No: 1), CDR2 (TISNSGGSTYYPDSVKD, SEQ ID No: 2), and CDR3 (DPYYTNYVPMDY, SEQ ID No: 3), and
complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);
(1-2) Complementarity-determining regions of heavy chains, CDR1 (DYYMD, SEQ ID No: 9), CDR2 (YIYPNKGGTSYNQKFKG, SEQ ID No: 10), and CDR3 (SGYGNYYTMDY, SEQ ID No: 11), and
complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);
(1-3) Complementarity-determining regions of the heavy chains, CDR1 (DYYMD, SEQ ID No: 17), CDR2 (YIYPNKGGTSYNQKFKG, SEQ ID No: 18), and CDR3 (GGYYSYYSYDY, SEQ ID No: 19), and
complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);
(1-4) Complementarity-determining regions of the heavy chains, CDR1 (DYYMD, SEQ ID No: 25), CDR2 (YIYPNKGGTSYNQKFKG, SEQ ID No: 26), and CDR3 (SGYKAYYAMDY, SEQ ID No: 27), and
complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);
(1-5) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and
complementarity-determining regions of the light chain, CDR1 (KASENVGTYVS, SEQ ID No: 36), CDR2 (GASNRYT, SEQ ID No: 37), and CDR3 (GQSYSYPLT, SEQ ID No: 38); but may also include an antibody etc. identified by a combination of CDRs other than a combination of the above CDRs, as long as the antibody etc. is characterized by having a binding ability to the TIM-3 antigen.

The half-molecule having a binding ability to the TIM-3 antigen constituting the heterodimeric antibody etc. of the present invention may comprise any of the combinations (1-1) to (1-5) of the sequences of CDR1-CDR3 of the heavy chain and the sequences of CDR1-CDR3 of the light chain, but the results of studies by the present inventors have demonstrated that it is possible to provide a more effective heterodimeric antibody etc. when the half-molecule having a binding ability to the TIM-3 antigen is the half-molecule of (1-1) or (1-5) among (1-1) to (1-5).

In this embodiment of the present invention, the amino acid sequences of the heavy chain variable region VH domain of the half-molecule having a binding ability to the TIM-3 antigen, among the heterodimeric antibody etc., can be specified, for example, as having an amino acid sequence selected from the group consisting of the amino acid sequences of the heavy chain variable region VH domain of (VH-1-1) to (VH-1-5) below:
(VH-1-1) the amino acid sequence of SEQ ID No: 7, or an amino acid sequence comprising one or a few amino acid substitutions (e.g., conservative substitutions), insertions, or deletions in regions of the amino acid sequence of SEQ ID No: 7 other than CDR1 (SEQ ID No: 1), CDR2 (SEQ ID No: 2), and CDR3 (SEQ ID No: 3);
(VH-1-2) the amino acid sequence of SEQ ID No: 15, or an amino acid sequence comprising one or a few amino acid substitutions (e.g., conservative substitutions), insertions, or deletions in regions of the amino acid sequence of SEQ ID No: 15 other than CDR1 (SEQ ID No: 9), CDR2 (SEQ ID No: 10), and CDR3 (SEQ ID No: 11);
(VH-1-3) the amino acid sequence of SEQ ID No: 23, or an amino acid sequence comprising one or a few amino acid substitutions (e.g., conservative substitutions), insertions, or deletions in regions of the amino acid sequence of SEQ ID No: 23 other than CDR1 (SEQ ID No: 17), CDR2 (SEQ ID No: 18), and CDR3 (SEQ ID No: 19);
(VH-1-4) the amino acid sequence of SEQ ID No: 31, or an amino acid sequence comprising one or a few amino acid substitutions (e.g., conservative substitutions), insertions, or deletions in regions of the amino acid sequence of SEQ ID No: 31 other than CDR1 (SEQ ID No: 25), CDR2 (SEQ ID No: 26), and CDR3 (SEQ ID No: 27);
(VH-1-5) the amino acid sequence of SEQ ID No: 39, or an amino acid sequence comprising one or a few amino acid substitutions (e.g., conservative substitutions), insertions, or deletions in regions of the amino acid sequence of SEQ ID No: 39 other than CDR1 (SEQ ID No: 33), CDR2 (SEQ ID No: 34), and CDR3 (SEQ ID No: 35);
wherein the numbers (VH-1-1) to (VH-1-5) correspond to the numbers (1-1) to (1-5) of the aforementioned combinations of the heavy chain CDR1 to CDR3 sequences. For example, the amino acid sequence of SEQ ID No: 7 of (VH-1-1) means the sequence containing the amino acid sequences of heavy chain CDR1 to CDR3 identified in (1-1) SEQ ID No: 1 to SEQ ID No: 3.

In this case, for the amino acid sequence of a region of the heavy chain variable region VH domain, other than CDRs, or the heavy chain constant region CH domain, the amino acid sequences of existing antibodies, for example, derived from a region of the VH domain other than CDRs, or the CH domain, of any human antibody, or any amino acid sequences modified from such an amino acid sequences. Specifically, for example, since it is known in the art that, among the amino acid sequences of the heavy chain variable region VH domain, each of CDR1 to CDR3 is essential for binding to the target antigen, while an antibody comprising one or several amino acid substitutions (e.g., conservative substitutions), insertions, or deletions in regions other than CDR1 to CDR3 of the amino acid sequences can be used, a half molecule having an amino acid sequence with one or several amino acid substitutions (e.g., conservative substitutions), insertions, or deletions in regions other than CDR1 to CDR3 can also be used in the present invention as long as it can constitute an antibody or an antibody derivative thereof that have a binding ability to the TIM-3 antigen and enhance the immune activity against cancer cells.

In this embodiment of the present invention, the amino acid sequences of the light chain variable region VL domain of the half-molecule having a binding ability to the TIM-3 antigen, among the heterodimeric antibody etc., can be specified, for example, as having an amino acid sequence selected from the group consisting of the amino acid sequences of the light chain variable region VL domain of (VL-1-1) to (VL-1-5) below:
(VL-1-1) the amino acid sequence of SEQ ID No: 8, or an amino acid sequence comprising one or a few amino acid substitutions (e.g., conservative substitutions), insertions, or deletions in regions the amino acid sequence of SEQ ID No: 8 other than CDR1 (SEQ ID No: 4), CDR2 (SEQ ID No: 5), and CDR3 (SEQ ID No: 6);
(VL-1-2) the amino acid sequence of SEQ ID No: 16, or an amino acid sequence comprising one or a few amino acid substitutions (e.g., conservative substitutions), insertions, or deletions in regions the amino acid sequence of SEQ ID No: 16 other than CDR1 (SEQ ID NO: 12), CDR2 (SEQ ID NO: 13), and CDR3 (SEQ ID NO: 14);
(VL-1-3) the amino acid sequence of SEQ ID No: 24, or an amino acid sequence comprising one or a few amino acid substitutions (e.g., conservative substitutions), insertions, or deletions in regions the amino acid sequence of SEQ ID No: 24 other than CDR1 (SEQ ID NO: 20), CDR2 (SEQ ID NO: 21), and CDR3 (SEQ ID NO: 22);
(VL-1-4) the amino acid sequence of SEQ ID No: 32, or an amino acid sequence comprising one or a few amino acid substitutions (e.g., conservative substitutions), insertions, or deletions in regions the amino acid sequence of SEQ ID No: 32 other than CDR1 (SEQ ID NO: 28), CDR2 (SEQ ID NO: 29), and CDR3 (SEQ ID NO: 30);
(VL-1-5) the amino acid sequence of SEQ ID No: 40, or an amino acid sequence comprising one or a few amino acid substitutions (e.g., conservative substitutions), insertions, or deletions in regions the amino acid sequence of SEQ ID No: 40 other than CDR1 (SEQ ID NO: 36), CDR2 (SEQ ID NO: 37), and CDR3 (SEQ ID NO: 38);
wherein the numbers (VL-1-1) to (VL-1-5) correspond to the numbers (1-1) to (1-5) of the aforementioned combinations of the light chain CDR1 to CDR3 sequences. For example, the amino acid sequence of SEQ ID No: 8 of (VL-1-1) means the sequence containing the amino acid sequences of light chain CDR1 to CDR3 identified in (1-1) SEQ ID No: 4 to SEQ ID No: 6.

In this case, for the amino acid sequence of a region of the light chain variable region VL domain, other than CDRs, or the light chain constant region CL domain, the amino acid sequences of existing antibodies, for example, derived from a region of the VL domain, other than CDRs, or the CL domain, of any human antibody, or any amino acid sequences modified from such an amino acid sequence. Specifically, for example, since it is known in the art that, among the amino acid sequences of the light chain variable region VL domain, each of CDR1 to CDR3 is essential for binding to the target antigen, while an antibody comprising one or several amino acid substitutions (e.g., conservative substitutions), insertions, or deletions in regions other than CDR1 to CDR3 of the amino acid sequences can be used, a half molecule having an amino acid sequence with one or several amino acid substitutions (e.g., conservative substitutions), insertions, or deletions in regions other than CDR1 to CDR3 can also be used in the present invention as long as it can constitute an antibody etc. that have a binding ability to the TIM-3 antigen and enhance the immune activity against cancer cells.

As described above, in the present invention, the antibody derivatives of the present invention also include functional fragments of the above-described antibody etc. and modified antibodies thereof. The functional fragments or modified antibodies that can be used in the present invention may be those which can bind to the TIM-3 antigen, inhibit binding between TIM-3-Galectin-9, and resultantly eliminate immunosuppressive signals based on the TIM-3-Galectin-9 pathway, resulting in capable of acting as so-called immune checkpoint inhibitors, or those which resultantly induce an ability to inhibit binding cytotoxicity against cancer cells. The functional fragments or modified antibodies that can be used in the present invention may be those having an ability to bind to TIM-3 antibody to inhibit binding between TIM-3-Galectin-9, and/or having an ability to bind to TIM-3 antibody to inhibit binding between TIM-3 and phosphatidylserine (PtdSer), separately from the activity of inhibiting binding between TIM-3-Galectin-9. The functional fragments or modified antibodies may be any fragments or modified antibodies as long as they have the above characteristics, and for example, single-chain Fv (scFv) can be used as such functional fragments.

For example, when a heterodimeric single-chain Fv (scFv) antibody is used as a heterodimeric antibody etc. of the present invention, the half-molecule having a binding ability to the TIM-3 antigen which constitute the heterodimeric antibody etc. of the present invention can be specified as a single chain by linking the amino acid sequence of the heavy chain variable region VH domain described below with the amino acid sequence of the light chain variable region VL domain described below:
(1-1) an amino acid sequence in which the heavy chain variable region of (VH-1-1) and the light chain variable region of (VL-1-1) are connected via a linker (variable region of half-molecule of Hu003_13_F20 single-chain antibody);
(1-2) an amino acid sequence in which the heavy chain variable region of (VH-1-2) and the light chain variable region of (VL-1-2) are connected via a linker (variable region of half-molecule of Hu149_83 single-chain antibody);
(1-3) an amino acid sequence in which the heavy chain variable region of (VH-1-3) and the light chain variable region of (VL-1-3) are connected via a linker (variable region of half-molecule of Hu428_83 single-chain antibody);
(1-4) an amino acid sequence in which the heavy chain variable region of (VH-1-4) and the light chain variable region of (VL-1-4) are connected via a linker (variable region of half-molecule of Hu621_83 single-chain antibody);
(1-5) an amino acid sequence in which the heavy chain variable region of (VH-1-5) and the light chain variable region of (VL-1-5) are connected via a linker (variable region of half-molecule of Hu072_11 single-chain antibody),
wherein the numbers (1-1) to (1-5) correspond to the numbers of the aforementioned combinations of the heavy chain CDR1 to CDR3 sequences and the numbers of the aforementioned combinations of the light chain CDR1 to CDR3 sequences. For example, the (1-1) amino acid sequence of the single-chain Fv antibody means the sequence including the (1-1) a sequence including amino acid sequences of heavy chain CDR1 to CDR3 identified in SEQ ID No: 1 to SEQ ID No: 3 and amino acid sequences of light chain CDR1 to CDR3 identified in SEQ ID No: 4 to SEQ ID No: 6.

Half-molecule having binding ability to CD39 antigen which constitute heterodimeric antibody etc. of the present invention

The half-molecule having a binding ability to the CD39 antigen which constitute the heterodimeric antibody etc. in this embodiment of the present invention is characterized as those, by way of example, by having the amino acid sequences of a total of six portions that are CDR1-3 of the heavy chain and CDR1-3 of the light chain, which are identical to those of the antibody having a binding ability to the CD39 antigen. Examples of the antibody etc. having a binding ability to the CD39 antigen of the present invention may include those constituted with the half-molecule which contain either set of the complementarity-determining regions of the heavy chain and the light chain selected from the group consisting of (2-1) to (2-7) below:
(2-1) Complementarity-determining regions of the heavy chain, CDR1 (GFSIKDTY, SEQ ID NO: 41), CDR2 (IDPANVNT, SEQ ID NO: 42), and CDR3 (ALYGYDDDAYYFDY, SEQ ID NO: 43), and
complementarity-determining regions of the light chain, CDR1 (ESVDNYGISF, SEQ ID NO: 44), CDR2 (AAS, SEQ ID NO: 45), and CDR3 (QQSKEVPYT, SEQ ID NO: 46);
(2-2) Complementarity-determining regions of the heavy chain, CDR1 (GYSFTDYN, SEQ ID NO: 49), CDR2 (IDPYSGGT, SEQ ID NO: 50), and CDR3 (GLYGYDDDANYFDD, SEQ ID NO: 51), and
complementarity-determining regions of the light chain, CDR1 (SSVSY, SEQ ID NO: 52), CDR2 (ATS, SEQ ID NO: 53), and CDR3 (QQRSSYPLT, SEQ ID NO: 54);
(2-3) Complementarity-determining regions of the heavy chain, CDR1 (NYGVH, SEQ ID NO: 57), CDR2 (VILRRGSTDYNAAFMS, SEQ ID NO: 58), and CDR3 (TAVVAGDYFDY, SEQ ID NO: 59), and
complementarity-determining regions of the light chain, CDR1 (KASQNVGTNVA, SEQ ID NO: 60), CDR2 (SASYRYS, SEQ ID NO: 61), and CDR3 (QQYNSYPLT, SEQ ID NO: 62);
(2-4) Complementarity-determining regions of the heavy chain, CDR1 (NYGMN, SEQ ID NO: 65), CDR2 (WINTYTGEPTYADDFKG, SEQ ID NO: 66), and CDR3 (KGYYGYPNYYAMDY, SEQ ID NO: 67), and
complementarity-determining regions of the light chain, CDR1 (KASQNVGTAVA, SEQ ID NO: 68), CDR2 (SASNRYT, SEQ ID NO: 69), and CDR3 (QQYSSYPIT, SEQ ID NO: 70);
(2-5) Complementarity-determining regions of the heavy chain, CDR1 (SYGVH, SEQ ID NO: 73), CDR2 (VIWRRGSTDYNAAFMS, SEQ ID NO: 74), and CDR3 (GISTATSWFAY, SEQ ID NO: 75), and
complementarity-determining regions of the light chain, CDR1 (KASQNVGTNVA, SEQ ID NO: 76), CDR2 (SASYRYS, SEQ ID NO: 77), and CDR3 (QQYNSYPLT, SEQ ID NO: 78);
(2-6) Complementarity-determining regions of the heavy chain, CDR1 (SYGVH, SEQ ID NO: 81), CDR2 (VIWRRGSTDYNAAFMS, SEQ ID NO: 82), and CDR3 (VRGDAMDY, SEQ ID NO: 83), and
complementarity-determining regions of the light chain, CDR1 (KASQNVGTNVA, SEQ ID NO: 84), CDR2 (SASYRYS, SEQ ID NO: 85), and CDR3 (QQYNSYPLT, SEQ ID NO: 86);
(2-7) Complementarity-determining regions of the heavy chain, CDR1 (DYGMH, SEQ ID NO: 89), CDR2 (YISSGSSIIYYADTVKG, SEQ ID NO: 90), and CDR3 (KDYPYAMDY, SEQ ID NO: 91), and
complementarity-determining regions of the light chain, CDR1 (KASQNVGTNVA, SEQ ID NO: 92), CDR2 (WASNRFT, SEQ ID NO: 93), and CDR3 (QQYSSSPYT, SEQ ID NO: 94), but may also include an antibody etc. identified by a combination of CDRs other than a combination of the above CDRs, as long as the antibody etc. is characterized by having a binding ability to the CD39 antigen.

The half-molecule having a binding ability to the CD39 antigen which constitute the heterodimeric antibody etc. of the present invention may comprise any of the combinations (2-1) to (2-7) of the sequences of CDR1-CDR3 of the heavy chain and the sequences of CDR1-CDR3 of the light chain, but the results of studies by the present inventors have demonstrated that it is possible to provide a more effective heterodimeric antibody etc. when the half-molecule having a binding ability to the CD39 antigen is the half-molecule of (2-1) or (2-3) to (2-7) among (2-1) to (2-7).

In this embodiment of the present invention, the amino acid sequences of the heavy chain variable region VH domain of the half-molecule having a binding ability to the CD39 antigen, in the heterodimeric antibody etc., can be specified, for example, as having an amino acid sequence selected from the group consisting of the amino acid sequences of the heavy chain variable region VH domain of (VH-2-1) to (VH-2-7) below:
(VH-2-1) the amino acid sequence of SEQ ID No: 47, or an amino acid sequence comprising one or a few amino acid substitutions (e.g., conservative substitutions), insertions, or deletions in regions of the amino acid sequence of SEQ ID No: 47 other than CDR1 (SEQ ID NO: 41), CDR2 (SEQ ID NO: 42), and CDR3 (SEQ ID NO: 43);
(VH-2-2) the amino acid sequence of SEQ ID No: 55, or an amino acid sequence comprising one or a few amino acid substitutions (e.g., conservative substitutions), insertions, or deletions in regions of the amino acid sequence of SEQ ID No: 55 other than CDR1 (SEQ ID NO: 49), CDR2 (SEQ ID NO: 50), and CDR3 (SEQ ID NO: 51);
(VH-2-3) the amino acid sequence of SEQ ID No: 123, or an amino acid sequence comprising one or a few amino acid substitutions (e.g., conservative substitutions), insertions, or deletions in regions of the amino acid sequence of SEQ ID No: 123 other than CDR1 (SEQ ID NO: 57), CDR2 (SEQ ID NO: 58), and CDR3 (SEQ ID NO: 59);
(VH-2-4) the amino acid sequence of SEQ ID No: 71, or an amino acid sequence comprising one or a few amino acid substitutions (e.g., conservative substitutions), insertions, or deletions in regions of the amino acid sequence of SEQ ID No: 71 other than CDR1 (SEQ ID NO: 65), CDR2 (SEQ ID NO: 66), and CDR3 (SEQ ID NO: 67);
(VH-2-5) the amino acid sequence of SEQ ID No: 79, or an amino acid sequence comprising one or a few amino acid substitutions (e.g., conservative substitutions), insertions, or deletions in regions of the amino acid sequence of SEQ ID No: 79 other than CDR1 (SEQ ID NO: 73), CDR2 (SEQ ID NO: 74), and CDR3 (SEQ ID NO: 75);
(VH-2-6) the amino acid sequence of SEQ ID No: 87, or an amino acid sequence comprising one or a few amino acid substitutions (e.g., conservative substitutions), insertions, or deletions in regions of the amino acid sequence of SEQ ID No: 87 other than CDR1 (SEQ ID NO: 81), CDR2 (SEQ ID NO: 82), and CDR3 (SEQ ID NO: 83);
(VH-2-7) the amino acid sequence of SEQ ID No: 95, or an amino acid sequence comprising one or a few amino acid substitutions (e.g., conservative substitutions), insertions, or deletions in regions of the amino acid sequence of SEQ ID No: 95 other than CDR1 (SEQ ID NO: 89), CDR2 (SEQ ID NO: 90), and CDR3 (SEQ ID NO: 91);
wherein the numbers (VH-2-1) to (VH-2-7) correspond to the numbers (2-1) to (2-7) of the aforementioned combinations of the heavy chain CDR1 to CDR3 sequences. For example, the amino acid sequence of SEQ ID No: 47 of (VH-2-1) means the sequence including the amino acid sequences of heavy chain CDR1 to CDR3 identified in (2-1) SEQ ID No: 41 to SEQ ID No: 43.

In this case, for the amino acid sequence of a region of the heavy chain variable region VH domain, other than CDRs, or the heavy chain constant region CH domain, the amino acid sequences of existing antibodies, for example, derived from a region of the VH domain, other than CDRs, or the CH domain, of any human antibody, or any amino acid sequences modified from such an amino acid sequences can be used. Specifically, for example, since it is known in the art that, among the amino acid sequences of the heavy chain variable region VH domain, each of CDR1 to CDR3 is essential for binding to the target antigen, while an antibody comprising one or several amino acid substitutions (e.g., conservative substitutions), insertions, or deletions in regions other than CDR1 to CDR3 of the amino acid sequences can be used, a half molecule having an amino acid sequence with one or several amino acid substitutions (e.g., conservative substitutions), insertions, or deletions in regions other than CDR1 to CDR3 can also be used in the present invention as long as it can constitute an antibody or an antibody derivative thereof that have a binding ability to the CD39 antigen and enhance the immune activity against cancer cells.

In this embodiment of the present invention, the amino acid sequences of the light chain variable region VL domain of the half-molecule having a binding ability to the CD39 antigen, among the heterodimeric antibody etc., can be specified, for example, as having an amino acid sequence selected from the group consisting of the amino acid sequences of the light chain variable region VL domain of (VL-2-1) to (VL-2-7) below:
(VL-2-1) the amino acid sequence of SEQ ID No: 48, or an amino acid sequence comprising one or a few amino acid substitutions (e.g., conservative substitutions), insertions, or deletions in regions of the amino acid sequence of SEQ ID No: 48 other than CDR1 (SEQ ID NO: 44), CDR2 (SEQ ID NO: 45), and CDR3 (SEQ ID NO: 46);
(VL-2-2) the amino acid sequence of SEQ ID No: 56, or an amino acid sequence comprising one or a few amino acid substitutions (e.g., conservative substitutions), insertions, or deletions in regions of the amino acid sequence of SEQ ID No: 56 other than CDR1 (SEQ ID NO: 52), CDR2 (SEQ ID NO: 53), and CDR3 (SEQ ID NO: 54);
(VL-2-3) the amino acid sequence of SEQ ID No: 124, or an amino acid sequence comprising one or a few amino acid substitutions (e.g., conservative substitutions), insertions, or deletions in regions of the amino acid sequence of SEQ ID No: 124 other than CDR1 (SEQ ID NO: 60), CDR2 (SEQ ID NO: 61), and CDR3 (SEQ ID NO: 62);
(VL-2-4) the amino acid sequence of SEQ ID No: 72, or an amino acid sequence comprising one or a few amino acid substitutions (e.g., conservative substitutions), insertions, or deletions in regions of the amino acid sequence of SEQ ID No: 72 other than CDR1 (SEQ ID NO: 68), CDR2 (SEQ ID NO: 69), and CDR3 (SEQ ID NO: 70);
(VL-2-5) the amino acid sequence of SEQ ID No: 80, or an amino acid sequence comprising one or a few amino acid substitutions (e.g., conservative substitutions), insertions, or deletions in regions of the amino acid sequence of SEQ ID No: 80 other than CDR1 (SEQ ID NO: 76), CDR2 (SEQ ID NO: 77), and CDR3 (SEQ ID NO: 78);
(VL-2-6) the amino acid sequence of SEQ ID No: 88, or an amino acid sequence comprising one or a few amino acid substitutions (e.g., conservative substitutions), insertions, or deletions in regions of the amino acid sequence of SEQ ID No: 88 other than CDR1 (SEQ ID NO: 84), CDR2 (SEQ ID NO: 85), and CDR3 (SEQ ID NO: 86);
(VL-2-7) the amino acid sequence of SEQ ID No: 96, or an amino acid sequence comprising one or a few amino acid substitutions (e.g., conservative substitutions), insertions, or deletions in regions of the amino acid sequence of SEQ ID No: 96 other than CDR1 (SEQ ID NO: 92), CDR2 (SEQ ID NO: 93), and CDR3 (SEQ ID NO: 94);
wherein the numbers (VL-2-1) to (VL-2-7) correspond to the numbers (2-1) to (2-7) of the aforementioned combinations of the light chain CDR1 to CDR3 sequences. For example, the amino acid sequence of SEQ ID No: 48 of (VL-2-1) means the sequence containing the amino acid sequences of light chain CDR1 to CDR3 identified in (2-1) SEQ ID No: 44 to SEQ ID No: 46.

In this case, for the amino acid sequence of a region of the light chain variable region VL domain, other than CDRs, or the light chain constant region CL domain, the amino acid sequences of existing antibodies, for example, derived from a region of the VL domain, other than CDRs, or the CL domain, of any human antibody, or any amino acid sequence modified from such an amino acid sequence can be used. Specifically, for example, since it is known in the art that, among the amino acid sequences of the light chain variable region VL domain, each of CDR1 to CDR3 is essential for binding to the target antigen, while an antibody comprising one or several amino acid substitutions (e.g., conservative substitutions), insertions, or deletions in regions other than CDR1 to CDR3 of the amino acid sequences can be used, a half molecule having an amino acid sequence with one or several amino acid substitutions (e.g., conservative substitutions), insertions, or deletions in regions other than CDR1 to CDR3 can also be used in the present invention as long as it can constitute an antibody etc. that have a binding ability to the CD39 antigen and enhance the immune activity against cancer cells.

As described above, in the present invention, the antibody derivatives of the present invention also include functional fragments of the above-described antibody etc. and modified antibodies thereof. The functional fragments or modified antibodies that can be used in the present invention may be any of relevant fragments and modified antibodies as long as they are characterized by being capable of acting as so-called immune checkpoint inhibitors which can bind to the CD39 antigen and inhibit the enzymatic activity of hydrolyzing ATP, decrease the amount of adenosine produced from ATP by an action of CD39 antigen of immune cells, and release the suppression of immune responses which is caused by adenosine, or being capable of resultantly inhibiting proliferation of cancer cells, and for example, single-chain Fv (scFv) can be used as such functional fragments.

For example, when a heterodimeric single-chain Fv (scFv) antibody is used as a heterodimeric antibody etc. of the present invention, the half-molecule having a binding ability to the CD39 antigen which constitute the heterodimeric antibody etc. of the present invention can be specified as a single chain by linking the amino acid sequence of the heavy chain variable region VH domain described below with the amino acid sequence of the light chain variable region VL domain described below:
(2-1) an amino acid sequence in which the heavy chain variable region of (VH-2-1) and the light chain variable region of (VL-2-1) are connected via a linker (variable region of half-molecule of 6-3hIG single-chain antibody);
(2-2) an amino acid sequence in which the heavy chain variable region of (VH-2-2) and the light chain variable region of (VL-2-2) are connected via a linker (variable region of half-molecule of B23-7h1IF single-chain antibody);
(2-3) an amino acid sequence in which the heavy chain variable region of (VH-2-3) and the light chain variable region of (VL-2-3) are connected via a linker (variable region of half-molecule of HuP4_IB single-chain antibody);
(2-4) an amino acid sequence in which the heavy chain variable region of (VH-2-4) and the light chain variable region of (VL-2-4) are connected via a linker (variable region of half-molecule of T86 single-chain antibody);
(2-5) an amino acid sequence in which the heavy chain variable region of (VH-2-5) and the light chain variable region of (VL-2-5) are connected via a linker (variable region of half-molecule of I67 single-chain antibody);
(2-6) an amino acid sequence in which the heavy chain variable region of (VH-2-6) and the light chain variable region of (VL-2-6) are connected via a linker (variable region of half-molecule of Tre291 single-chain antibody);
(2-7) an amino acid sequence in which the heavy chain variable region of (VH-2-7) and the light chain variable region of (VL-2-7) are connected via a linker (variable region of half-molecule of mAnE67 single-chain antibody),
wherein the numbers (2-1) to (2-7) correspond to the numbers of the aforementioned combinations of the heavy chain CDR1 to CDR3 sequences and the numbers of the aforementioned combinations of the light chain CDR1 to CDR3 sequences. For example, the (2-1) amino acid sequence of the single-chain Fv antibody means the sequence including the (2-1) a sequence including amino acid sequences of heavy chain CDR1 to CDR3 identified in SEQ ID No: 41 to SEQ ID No: 43 and amino acid sequences of light chain CDR1 to CDR3 identified in SEQ ID No: 44 to SEQ ID No: 46.

Production of an antibody etc. or half-molecules of the antibody etc.

Antibodies from which the half-molecule constituting the heterodimeric antibody etc. of the present invention is derived can be obtained by a method in which TIM-3 antigen is administered, as an immunogen, to animals of the aforementioned species, for the antibody as a source of the half-molecule having a binding ability to the TIM-3 antigen, and CD39 antigen is administered, as an immunogen, to animals of the aforementioned species, for the antibody as a source of the half-molecule having a binding ability to the CD39 antigen, and the antibody producing cells collected from the bodies of each animals are fused with myeloma cells, followed by culture (so-called hybridoma method), by a method in which a vector for protein expression containing a DNA sequence that can define the amino acid sequence of the antibody etc. is designed, which is introduced into cells for protein production to recombinantly obtain the antibodies (so-called recombinant DNA method), or by a method in which the variable regions of the antibody etc. as single-chain antibodies (scFv) are expressed on the surface of phage to obtain a phage that binds to antigens (so-called phage display method).

All of these methods are commonly used in the technical field, and the antibody etc. can be prepared by referring to the following documents:
- For the hybridoma method, see, for example, Kohler and Milstein et al., Nature(1975), Vol.256, p. 495-97, Hongo et al., Hybridoma (1995), Vol.14, No. 3, p. 253-260, Harlow et al., Antibodies: A Laboratory Manual, (Cold Spring Harbor Laboratory Press (1988), Vol.2) and Hammerling et al., Monoclonal Antibodies and T-Cell Hybridomas, p.563-681 (Elsevier, N.Y., 1981), etc.,
- For the recombinant DNA method, see, for example, US4816567,
- For the phage display method, see, for example, US5223409, US5403484, and US5571698 of Ladner et al., US5427908 and US5580717 of Dower et al., US5969108 and US6172197 of McCafferty et al. and US5885793, US6521404, US6544731, US6555313, US6582915 and US6593081 by Griffiths et al. and others.

### fcsij

When a heterodimeric antibody etc. of the present invention is produced by the recombinant DNA method or the phage display method, DNA sequences that can code for the amino acid sequences of the heterodimeric antibody etc. of the present invention can be produced by a method in which it is obtained from cells producing the desired antibody etc., a method in which it is designed based on codons optimized for the animal species used in the expression system based on the amino acid sequence of the antibody etc., or a combination of these methods.
when the heterodimeric antibody etc. of the present invention is produced by the recombinant DNA method, the prepared DNA sequences can be obtained by methods well known to those skilled in the art, for example, by a method in which the prepared DNA sequence is incorporated into an expression vector suitable for the cell type for protein expression (e.g., CHO cells) in which the antibody etc. is to be expressed, which is introduced into the cell type for protein expression.

When a heterodimeric antibody etc. of the present invention is produced by the recombinant DNA method, since the structure of the heterodimeric antibody is a combination of two types of the heavy chains and two types of the light chains, the heterodimeric antibody etc. of the present invention can be produced by, for example, a method in which the following vectors:
a vector containing a DNA sequence that codes for the heavy chain amino acid sequence of the half-molecule having a binding ability to TIM-3 antigen,
a vector containing a DNA sequence that codes for the heavy chain amino acid sequence of the half-molecule having a binding ability to CD39 antigen,
a vector containing a DNA sequence that codes for the light chain amino acid sequence of the half-molecule having a binding ability to TIM-3 antigen, and
a vector containing a DNA sequence that codes for the light chain amino acid sequence of the half-molecule having a binding ability to CD39 antigen
are introduced into the cell type for protein expression to intracellularly express four proteins (i.e., heavy chain and light chain of half molecule having a binding ability to TIM-3 antigen and heavy chain and light chain of half molecule having a binding ability to CD39 antigen), thereby intracellularly producing the desired antibody.

When a heterodimeric antibody etc. of the present invention is produced by the phage display method, a library of phages which displays the produced DNA sequences of H chains and L chains connected by short amino acid sequences on the phages is used to select antibodies with affinity for the target molecules.

In the case of a method for producing a heterodimeric antibody etc., a homodimeric antibody is formed with a probability of 50% when two proteins (heavy chain-light chain of half-molecule having a binding ability to TIM-3 antigen and heavy chain-light chain of half-molecule having a binding ability to CD39 antigen) are randomly combined together. Therefore, it is preferred to use the "knobs-into-holes" technique for the production of a divalent bispecific antibody (see, for example, the method described in US7183076B2). In this technique, the "Knob" structure is introduced into the CH3 domain of the constant region of the one of the two half-molecules of the antibody etc., and the "Hole" structure is introduced into the CH3 domain of the constant region of the other half-molecule of the antibody etc.. By combining the half-molecule of the antibody etc. having the "Knob" structure in the constant region and the half-molecule of the antibody etc. having the "Hole" structure in the constant region, it is possible to facilitate to form the heavy chain heterodimers when two asymmetric heavy chains are formed. The "Knob" and "Hole" structures can be used in combination with the aforementioned heavy chain variable region or single chain Fv (scFv) antibody variable region.

### Functions of the heterodimeric antibody etc. of the present invention

In an aspect, the heterodimeric antibody etc. of the present invention inhibits the function of the TIM-3 antigen expressed on cancer cells, or inhibits the function of the TIM-3 antigen expressed on immune cells, thereby eliminating immunosuppressive signals against cancer cells, and releasing the exhaustion of immune cells. As a result, by activating immune cells to proliferate immune cells, increasing the cytotoxicity of immune cells against cancer cells, and enhancing the secretion of cytokines by immune cells, the heterodimeric antibody etc. exhibits a tumor growth inhibiting effect and a cancer treatment effect.

TIM-3 antigen (also known as HAVCR2) is known as an immune checkpoint molecule expressed on Th1 cells (type 1 T helper cells), dendritic cells, CD8+ T cells and other lymphocyte subsets, and is a key regulator of T cell tolerance and has been shown to play a pivotal role in autoimmunity and T cell exhaustion during chronic viral infection. TIM-3 is known to interact with a lot of ligands such as Phosphatidylserine (PtdSer), Carcinoembryonic antigen-related cell adhesion molecule 1 (CEACAM1), Galectin-9, and high mobility group box 1 (HMGB1), and, among them, the TIM-3-Galectin-9 pathway is known to negatively regulate an immune function of Th1 cells (type 1 T helper cells).

The studies by the present inventors have revealed that the heterodimeric antibody etc. of the present invention inhibits the binding between TIM-3 and Galectin-9 by 50% or more.

Therefore, as a direct function, the heterodimeric antibody etc. of the present invention binds to the TIM-3 antigen, inhibits the function of the TIM-3 antigen and inhibits the binding between TIM-3-Galectin-9, thereby exhibiting an ability to eliminate immunosuppressive signals that negatively regulate Th1 cell (type 1 T helper cell) immunity via the TIM-3-Galectin-9 pathway, especially exhibiting an ability to eliminate immunosuppressive signals against cancer cells.

In other words, the heterodimeric antibody etc. of the present invention binds to TIM-3 antigen expressed on cancer cells or immune cells to eliminate immunosuppressive signals based on the TIM-3-Galectin-9 pathway involving TIM-3 antigen, and i.e., the heterodimeric antibody etc. can function as an immune checkpoint inhibitor.

The studies by the present inventors have also shown that the heterodimeric antibody etc. of the present invention completely inhibits the binding between TIM-3 and Phosphatidylserine. In other words, as a direct function, the heterodimeric antibody etc. of the present invention binds to the TIM-3 antigen to inhibit the function of the TIM-3 antigen, thereby exhibiting an ability to inhibit the binding between TIM-3 and phosphatidylserine (PtdSer).

As a result of the above-mentioned direct function of suppressing the function of TIM-3 antigen expressed on immune cells, the antibody etc. of the present invention can activate, as a secondary effect, the immune cells by relieving the exhaustion of the immune cells, resulting in exhibition of a tumor growth inhibiting effect and a cancer treatment effect.

The immune checkpoint involving TIM-3 functions through a mechanism different from other known immune checkpoint molecules, such as PD-1/PD-L1 and CTLA-4/B7 (CD80/CD86). Therefore, the heterodimeric antibody etc. of the present invention can be used as a backup medicament when the PD-1/PD-L1 inhibitors (e.g., Nivolumab, Pembrolizumab (anti-PD-1 antibodies), Atezolizumab, Durvalumab (anti-PD-L1 antibodies)) or CTLA-4/B7 (CD80/CD86) inhibitors (Ipilimumab (anti-CTLA-4 antibody)) are not effective, or the heterodimeric antibody etc. of the present invention can also be used in combination with these existing immune checkpoint inhibitors.

In this way, the heterodimeric antibody etc. of the present invention is preferably capable of suppressing the function of TIM-3 expressed on cancer cells or immune cells and relieving the exhaustion of the immune cells, thereby activating immune cells and exerting cancer growth suppression effects and cancer therapeutic effects. Such activation of T cells may occur in vitro or in vivo. For example, to determine in advance whether an antibody against TIM-3 antigen has the ability to activate T cells that are cytotoxic to such cancer cells in a live body (in vivo), the antibody having an ability to bind to the TIM-3 antigen can be screened in vitro by measuring whether T cells are actually activated to obtain the antibody actually having an ability to activate T cells.

In the context of the present invention, the exhaustion of immune cells refers to a state where immune cells have become dysfunctional. Specifically, it includes, but not limited to, a decrease in cytokine secretion and cytotoxicity of T cells, a decrease in cytokine secretion and cytotoxicity of NK cells, a decrease in antigen-presenting ability of dendritic cells, and activation of regulatory T cells. In the case where the immune cells are in a state of exhaustion, the individual's immune response against cancer cells does not have a function or has a decreased function, resulting in reduced activity to eliminate cancer cells. The exhaustion of immune cells is known to occur when checkpoint proteins (such as PD-1, CTLA-4, TIM-3, etc.) increase on the surface of T cells or when immune system including effector T cells are subjected to long-term stimulation by proliferating cancer cells. However, it is considered to be a reversible condition, and also considered to be possible to relieve the state of exhaustion by activating the same cells.

In the context of the present invention, activation of T cells can be specified by indicators such as T cell proliferation, increased cytotoxicity of T cells against cancer cells, and secretion of cytokines from T cells. When the activation of T cells is observed by the heterodimeric antibody etc. of the present invention, it indicates that the heterodimeric antibody etc. of the present invention has an ability to inhibit the activity of TIM-3 in T cells, resulting in exhibition of the tumor growth suppression effects and of the cancer therapeutic effects.

In another aspect, the heterodimeric antibody etc. of the present invention has an ability to inhibit the function of the CD39 antigen expressed on immune cells, to decrease the amount of adenosine produced by the CD39 antigen of immune cells from ATP released from dying tumor cells, and to release suppression of immune responses which is caused by the adenosine, thereby releasing suppressive effect on the antitumor activity of cytotoxic T cells, and, as a result, resulting in exhibition of the ability to enhance the immune activity against cancer cells.

CD39 (also known as Ectonucleoside Triphosphate Diphosphohydrolase 1 (ENTPD1)) is recognized as a substance that may function as an immune checkpoint molecule different from TIM-3 antigen. The CD39 has the enzymatic activity of binding to extracellular ATP (eATP) and hydrolyzing the ATP into AMP. The expression of CD39 has been shown to increase in intratumor immune cells, and adenosine, generated by extracellular hydrolysis into adenosine of eATP released from stressed cells or dying cells, suppresses immune responses.

The studies by the present inventors have demonstrated that the heterodimeric antibody etc. of the present invention inhibits the ATPase activity of CD39, and inhibits the ATPase activity more potently in cells co-expressing CD39 and TIM-3 than in cells expressing CD39 alone.

Therefore, since the heterodimeric antibody etc. of the present invention inhibits the binding between CD39 and extracellular ATP (eATP), it has an ability to decrease the amount of adenosine generated by the action of the CD39 antigen of immune cells from ATP, released from tumor cells dying under attacks from immune system cells, and to eliminate suppression of immune responses, which is caused by the adenosine, resulting in enhancement of immune activity against cancer cells.

Here, the enhancement of immune activity refers to activation of T cells which includes, but not limited to, removal of immunosuppressive signals, relieving T cell exhaustion, proliferation of T cells, increase in cytotoxicity of T cells against cancer cells, promotion of T cell cytokine secretion, enhancement of tumor infiltration of activated T cells, promotion of dendritic cell (DC) maturation, enhancement of dendritic cell-mediated activation of antigen-specific T cells, and enhancement of dendritic cell-mediated tumor cytotoxic activity of T cells. Thus, the heterodimeric antibody etc. of the present invention can be used to inhibit proliferation of cancer cells.

As described above, the heterodimeric antibody etc. of the present invention is characterized by
- exhibiting a tumor growth inhibiting effect or cancer treatment effect which is exhibited as a result of inhibition of the function of TIM-3 antigen expressed on cancer cells or TIM-3 antigen expressed on immune cells, and elimination of immunosuppressive signals against cancer cells to release the exhaustion of immune cells, thereby inducing activation of immune cells such as proliferation of immune cells, increase of the cytotoxicity of immune cells against cancer cells, and enhancement of the secretion of cytokines by immune cells, and
- exhibiting an ability to enhance the immune activity against cancer cells which is exhibited as a result of inhibition of the function of CD39 antigen expressed on immune cells, decrease of the amount of adenosine generated by the CD39 antigen of immune cells from ATP released from dying tumor cells, and elimination of suppression of immune responses which is caused by the adenosine, thereby induction of release suppressive effect to the antitumor activity of cytotoxic T cells. Therefore, since the heterodimeric antibody etc. of the present invention releases the suppression of immune responses of T cells which is induced by tumor cells or cancer cells, the heterodimeric antibody etc. is characterized by inducing cytotoxicity against cancer cells while inducing no cytotoxicity against normal cells.

Since highly exhausted cytotoxic T cells or dendritic cells simultaneously express both TIM-3 antigen and CD39 antigen, it is considered that more potent antitumor effect may be obtained by inhibiting the functions of the immune checkpoint molecules of both TIM-3 antigen and CD39 antigen simultaneously.

Here, cancer cells targeted by the heterodimeric antibody etc. of the present invention are not limited, and examples thereof may include blood cancer, melanoma, breast cancer, lung cancer, colon cancer, gastric cancer, pancreatic cancer, and liver cancer.

The studies by the present inventors have demonstrated that the heterodimeric antibody etc. of the present invention has, in addition to the above actions, characteristic actions as follows:
- an action of enhancing the proliferation of IFNgamma-positive CD8+ T cells by SEB (staphylococcal enterotoxin B) stimulation (this action is more potent as compared to an action which is exhibited by simultaneously applying the anti-TIM-3 antibody and the anti-CD39 antibody);
- an action of enhancing the proliferation of antigen-specific CD8+ T cells by peptide (this action is more potent as compared to an action which is exhibited by simultaneously applying the anti-TIM-3 antibody and the anti-CD39 antibody;
- an action of enhancing the maturation of dendritic cells (DC) and enhancing secretion of CXCL9 that promotes tumor infiltration of activated T cells and secretion of TNFa and IL-6 as proinflammatory cytokines (this action is not observed when the anti-TIM-3 antibody or the anti-CD39 antibody is applied alone, or the anti-TIM-3 antibody and the anti-CD39 antibody are simultaneously applied);
- an action of enhancing the activation of antigen-specific T cells via dendritic cells (this action is not observed when the anti-TIM-3 antibody or the anti-CD39 antibody is applied alone, or the anti-TIM-3 antibody and the anti-CD39 antibody are simultaneously applied);
- an action of enhancing the tumor cytotoxic activity of T cells via dendritic cells (this action is not observed when the anti-TIM-3 antibody or the anti-CD39 antibody is applied alone, or the anti-TIM-3 antibody and the anti-CD39 antibody are simultaneously applied);
and so on.

In other words, the heterodimeric antibody etc. of the present invention exhibits antitumor activity that is not observed when the anti-TIM-3 antibody and the anti-CD39 antibody are simultaneously applied, and, therefore, exhibits an action that cannot be exhibited by simply simultaneously inhibiting the functions of TIM-3 antigen and CD39 antigen. The heterodimeric antibody etc. of the present invention exhibits potent cytotoxic activity even against tumor cells that do not express TIM-3 antigen and CD39 antigen.

### Applications of antibody etc.

Based on the aforementioned action of the heterodimeric antibody etc. of the present invention to enhance immune activity against cancer cells and to activate immune cells having cytotoxicity against cancer cells, the heterodimeric antibody etc. of the present invention the present invention can provide a pharmaceutical composition for the treatment of cancer, comprising the heterodimeric antibody etc. of the present invention. The pharmaceutical composition can treat cancer in a subject in need of cancer treatment or prevention through the action of the heterodimeric antibody etc. of the present invention to activate immune cells with cytotoxicity against cancer cells.

In the present invention, cancer cells that can be therapeutically targeted by the antibody etc. of the present invention are cancer cells that express TIM-3 protein, cancer cells that can receive immunosuppressive signals from immune cells expressing TIM-3 protein, cancer cells that express CD39 protein, or cancer cells that can receive immunosuppressive signals from immune cells expressing CD39 protein, or any combination thereof. Examples of such cancer cells may include cells derived from cancer selected from the group consisting of: leukemia (including chronic lymphocytic leukemia, acute lymphocytic leukemia), lymphoma (including non-Hodgkin lymphoma, Hodgkin lymphoma, T-cell lymphoma, B-cell lymphoma, Burkitt lymphoma, malignant lymphoma, diffuse lymphoma, follicular lymphoma), myeloma (including multiple myeloma), melanoma, lung cancer, breast cancer, colon cancer, kidney cancer, gastric cancer, ovarian cancer, pancreatic cancer, cervical cancer, uterine cancer, endometrial cancer, esophageal cancer, liver cancer, head and neck cancer, head and neck squamous cell carcinoma, skin cancer, urinary tract cancer, prostate cancer, choriocarcinoma, pharyngeal cancer, laryngeal cancer, blood cancer, myoma, male germinoma, endometrial hyperplasia, endometriosis, embryonal carcinoma, fibrosarcoma, Kaposi's sarcoma, hemangioma, cavernous hemangioma, hemangioblastoma, retinoblastoma, astrocytoma, neurofibroma, oligodendromatosis, medulloblastoma, neuroblastoma, glioma, rhabdomyosarcoma, glioblastoma, osteogenic sarcoma, leiomyosarcoma, thyroid sarcoma, and Wilms tumor.

In embodiments of the present invention, the cancer cells are preferably cancer cells that express TIM-3 antigen, for example, cells derived from, among the above described cancers, blood cancer, melanoma, breast cancer, lung cancer, colon cancer, gastric cancer, pancreatic cancer and liver cancer.

In present invention, the heterodimeric antibody etc. of the present invention can also be combined with other drugs, such as other antibodies or anticancer agents, to provide a composition. For example, the heterodimeric antibody etc. of the present invention can also be linked with drugs, and administered as an antibody-drug complex (ADC).

In the present invention, the heterodimeric antibody etc. of the present invention can also be formulated with a physiologically/pharmacologically acceptable diluent or carrier. Suitable carriers include, but not limited to, buffer (such as phosphate buffer, citrate buffer, acetate buffer), salts (such as sodium chloride), sugars (such as glucose, trehalose, mannitol, sorbitol), additives (such as amino acids such as arginine, surfactants such as polysorbates).

The heterodimeric antibody etc. of the present invention can be stored after lyophilization (freeze-dried preparation) and can be used after reconstitution with an aqueous buffer solution, as mentioned above, when necessary. A formulation containing the antibody etc. of the present invention can be administered in various dosage forms, including parenteral drugs such as injections and intravenous injections drips.

The dosage of the heterodimeric antibody etc. of the present invention may vary depending on symptoms, age, weight, etc., but typically may range from 0.01 mg to 1000 mg per kg body weight per dose for parenteral administration, preferably, for example, from 0.05 mg to 500 mg, from 0.05 mg to 100 mg, from 0.05 mg to 50 mg, or from 0.05 mg to 20 mg per kg body weight per day, more preferably from 0.1 mg to 10 mg per kg body weight per day, which can be administered via a suitable administration route such as intraperitoneal injection, subcutaneous injection, intramuscular injection, intratumoral injection, or intravenous injection, depending on the type of cancer.

In another embodiment, based on the aforementioned ability of the antibody etc. of the present invention to activate immune cells having cytotoxicity against cancer cells, the present invention may also provide a method of treating or preventing cancer in a subject in need of cancer treatment or prevention, in which an effective amount of the antibody etc. of the present invention is administered to the subject. Treatment or prevention of cancer with the antibody etc. of the present invention occurs due to the activation of immune cells with cytotoxicity against cancer cells in the body.

In the case of administering the heterodimeric antibody etc. of the present invention to a living body for the treatment or prevention of cancer, the antibody etc. can be administered in combination with an adjuvant (e.g., as described in Clin. Microbiol. Rev., 7:277-289, 1994) to effectively generate cellular or humoral immunity in the body, or can be administered in the particle dosage form such as liposome formulations, particle-based formulations bound to beads with diameters in the range of a few micrometers, lipid-bound formulations.

Based on the aforementioned action of the heterodimeric antibody etc. of the present invention, the heterodimeric antibody etc. of the present invention can also be used in a process for measuring whether the heterodimeric antibody etc. of the present invention can exhibit cytotoxicity against cancer cells, comprising the following steps:
- a step of bringing cancer cells collected from a subject into contact in vitro with a heterodimeric antibody etc. of the present invention; and
- a step of measuring whether the cell viability of the cancer cells is decreased or a step of measuring whether the secretion of immune-activating substances is enhanced, under culture conditions.

In this case, whether or not the heterodimeric antibody etc. of the present invention can exhibit cytotoxicity against cancer cells can be determined by measuring, in the presence of peripheral blood lymphocytes from the same subject, whether cell viability of the cancer cells is decreased or whether immune cells derived from peripheral blood lymphocytes are activated when the heterodimeric antibody etc. of the present invention is present.

By this method, referring to the results of measuring whether or not the heterodimeric antibody etc. of the present invention can exhibit cytotoxicity against cancer cells, the enhancement of cytotoxicity against the cancer cells when the heterodimeric antibody etc. of the present invention is administered to a subject can be measured based on the in vitro cytotoxicity action against cancer cells collected from the subject.

By this method, the enhancement of cytotoxicity against the cancer cells when the heterodimeric antibody etc. of the present invention is administered to the subject can be measured based on the results of measuring whether the heterodimeric antibody etc. of the present invention enhances secretion of immune activation substances in vitro.

The present invention can also provide a measurement kit comprising a heterodimeric antibody etc. of the present invention, for measuring, in vitro, activation of cytotoxicity against cancer cells collected from a subject, activation of secretion of immune activation substances, or activation of immune cells, by the heterodimeric antibody etc. of the present invention, as aforementioned.

The following examples are provided to illustrate the invention in detail. The examples shown below are not intended to limit the invention in any way.

### Examples

Example 1: Production of Anti-Human TIM-3 Monoclonal Antibody

In this example, first, mRNA of a monoclonal antibody binding specifically to human TIM-3 was obtained from the spleen of mice immunized with human TIM-3, and a phage display method was used to produce monoclonal antibodies binding specifically to human TIM-3.

Mice (MRL/lpr) were immunized with recombinant human TIM-3 (rhTIM-3-His: SinoBological, 0390-H08H) and Expi293 cells transiently expressing human TIM-3 antigen, and the spleens were collected after confirmation of elevated serum antibody titers. The sequence of human TIM-3 protein and the nucleotide sequence encoding the protein were prepared in accordance with Accession No. JX049979.1.

Total RNA was extracted from the spleens of the immunized mice, which was converted to cDNA, and the nucleotide sequences encoding the antibody genes (VH gene and Vkappa gene) were amplified by PCR. The nucleotide sequence encoding the VH gene and the nucleotide sequence encoding the Vkappa gene were connected by a linker (GGGGS x 4) to form a nucleotide sequence encoding single chain Fv (hereafter referred to as scFv), which was inserted into a phagemid (ThermoFisher Scientific) derived from pTZ19R. E. coli (DH12S strain) (ThermoFisher Scientific) was transformed with the phagemid inserted with the scFV nucleotide sequence, and infected with helper phage (M13KO7, New England Biolabs) to construct scFv phage library (size: about 1 × 10⁸ clones).

The scFv antibody phage library was subjected to panning with recombinant human TIM-3, and the post-panning phage-infected E. coli was subject to IPTG-induction to express scFv, which was screened for scFv clones that specifically bind to human TIM-3 (Accession No. JX049979.1) stable-expressing Jurkat cells.

The nucleotide sequences of the scFvs that were positive in the screening were then sequenced and connected by a linker (GGGG) to a modified version of an antibody Fc region (Fc-LALA with L234A and L235A mutations in the Fc region of IgG1) to form scFv-Fc-LALA antibodies or to a modified version of an antibody Fc region introduced with LALA (L234A mutation and L235A mutation) in the Fc region of IgG1 to form antibodies in IgG1-LALA format, which were expressed in ExpiCHO cells (Thermo Fisher Scientific), and the antibodies were affinity purified using Protein A column.

From these antibodies, (1-1) 003 antibody, (1-2) 149 antibody, (1-3) 428 antibody, (1-4) 621 antibody and (1-5) 072 antibody were selected.

### Example 2: Preparation of Recombinant Human CD39

In this example, recombinant human CD39 for use in immunization of animals was produced.

The recombinant human CD39 (Accession No. NM_001776.6) was transiently expressed on FS293 cells (ThermoFisher Scientific), and purified. Specifically, human CD39-ECD (a nucleotide sequence corresponding to 38 to 478 aa of Accession No. NM_001776.6) was inserted into an expression vector, and FS293 cells were transformed by the expression vector using an electroporation method with MaxCyte-STX (MaxCyte), and the transformed cells were cultured for 4 to 5 days to express recombinant human CD39 (38 to 478 aa). The recombinant human CD39 in the culture supernatant was subjected to batch purification with Ni-NTA Agarose Resin (FujiFilm-Wako), then gel-filtered, and collected.

### Example 3: Production of Anti-Human CD39 Antibody (B Cell Fractionation Method)

In this example, the recombinant human CD39 produced in Example 2 was used to produce a monoclonal antibody that binds specifically to human CD39 by a B cell fractionation method from the spleen or lymph node of mice immunized with human CD39.

### (3-1) Production of anti-human CD39 antibody

From the spleens of mice immunized with human CD39, a monoclonal antibody that specifically binds to human CD39 was produced by the B cell fractionation method.

Mice (BALB/c or MRL/lpr) were immunized with recombinant human CD39 (in-house product, Accession No. NM_001776.6) and Expi293 cells transiently expressing human CD39 antigen (38 to 478 aa of Accession No. NM_001776.6), and the spleens were collected after confirmation of elevated serum antibody titers.

A single-cell suspension of spleen cells was prepared from the spleens, and IgG-positive B cells were purified using Memory B Cell Isolation Kit, mouse (Miltenyi Biotec, 130-095-838). The purified B cells were labeled with APC-anti-mouse IgG1 and APC-anti-mouse IgG2a included in the kit. Further, the cells were stained with PE-human CD39 (in-house product, 38 to 478 aa of Accession No. NM_001776.6 to which the Myc tag and the His tag were added to the C-terminus), PE/Cy7-anti-mouse/human CD45R/B220 (BioLegend, 103222) and BV421-anti-mouse IgM (BioLegend, 406518).

Using FACS Aria III (BD), a population of cells that are IgM-negative, IgG-positive and B220-positive and have a binding ability to human CD39 was selected from the above stained cells which was aliquoted into a 96-well plate at one cell per well, each well of which contains 150 microliters of a medium. The medium used was RPMI-1640 (Wako Pure Chemical Industries, Ltd., 189-02025) supplemented with 40% FBS (Equitech-Bio, SFBM30-0500) and 1% penicillin-streptomycin (nacalai tesque, 09367-34). The cells were left to stand under 5% CO₂ at 37°C for 1 hour, and feeder cells at the concentration of 3 to 5 × 10⁴ cells/ml suspended in RPMI-1640 with cytokines were then added to the 96-well plate at 100 microliters per well, and the cells were cultured under 5% CO₂ at 37°C for 12 to 14 days. As the feeder cells, 3T3 cells expressing mouse CD40L irradiated with a 50 Gy X ray, which had been preserved in a frozen state, were thawed and used.

Genes corresponding to the heavy chain variable region and the light chain variable region of the antibodies were taken from the B cells cultured in the 96-well plate, which were amplified by the RT-PCR method. As primers for use in the amplification, sequences containing regions of the 5'-terminus and the 3'-terminus of each of the heavy chain variable region and the light chain variable region were selected based on information from a published document (Lotta von Boehmer et al., Nature Protocols, vol 11, p. 1908-1923 (2016)), and mix primers with a plurality of sequences in view of the diversity of antibody gene sequences were used (heavy chain: H1 mix and H2 mix, light chain: k mix).

After the amplification of the antibody gene, the linker sequence required to be inserted into the antibody expression vector was added to the terminus of the gene through a PCR reaction, and the heavy chain variable region and the light chain variable region were inserted into a vector for mouse IgG1 expression and a vector for mouse kappa chain expression, respectively, through a ligation reaction.

The ligation product was transformed into E. coli, and plasmids for antibody expression were collected. The heavy chain expression plasmid (derived from a H1 or H2 mix amplification product) and the light chain expression plasmid (derived from a k mix amplification product) were transfected into CHO cells. The antibodies produced in the culture supernatant from CHO cells were collected, and put together as a bulk antibody, and its CD39 inhibitory activity was confirmed.

### (3-2) Evaluation of inhibitory activity of antibody on CD39

For evaluation of inhibitory activity on CD39, wild-type SK-MEL28 cells expressing human CD39 (Accession No. NM_001776.6) were used. The wild-type SK-MEL28 cells were adjusted to 5 × 10³ cells/well using E-MEM medium, and seeded on a 96-well V-bottom plate at 50 microliters per well. Thereto, a dilution series of each antibody were added at 50 microliters per well, and left to stand in an incubator at 37°C for 1 hour. Next, 5 microliters of ATP (SIGMA, A2383) adjusted at 200 nM using E-MEM medium was added to each well, and the cells were left to stand at room temperature for 30 minutes. Thereafter, the plate was centrifuged at 700 g for 3 minutes, and 50 microliters of the supernatant was collected in a 96-well polystyrene (white) plate.

To the culture supernatant was added 50 microliters of CellTiter-Glo (registered trademark) 2.0 (Promega, G9242) for ATP quantification, the mixture was left to stand at room temperature for 10 minutes, and the amount of ATP was measured by luminescence detection with a microplate reader. Considering that the level of residual ATP in wells where the cells were not seeded was 100% and the level of residual ATP in wells where the cells were seeded but the antibody was not added was 0%, the ratio of inhibition of ATP degradation by each antibody was calculated from the percentage level of residual ATP in the measurement sample, and the inhibitory activity of the antibody on ATP degradation by CD39 was evaluated.

E. coli was transformed with plasmids of bulk antibodies confirmed to have inhibitory activity on ATP degradation by CD39, and single colonies of each of the heavy chain and the light chain were collected from the inoculated plate to obtain plasmid DNA. The heavy chain expression plasmid and the light chain expression plasmid derived from each of the single colonies were co-transfected into CHO cells, the antibodies produced in the culture supernatant from the CHO cells were collected, and as a single antibody, the CD39 inhibitory activity of the antibody was confirmed as above.

### (3-3) Production of anti-CD39 chimeric antibody

For the antibodies confirmed to have CD39 inhibitory activity, the genetic sequence of the variable region of each of the heavy chain expression plasmid and the light chain expression plasmid derived from each of the single colonies was analyzed by Sanger Sequencing, and based on the genetic sequences, the amino acid sequences were determined.

Primers were designed from the determined sequences, and the nucleotide sequences of antibody genes (VH gene and Vkappa gene) were amplified by PCR. The PCR amplification products of the amplified VH gene and Vkappa gene were connected by a linker (GGGGS x 4), and connected by a linker (GGGG) to a modified version of an antibody Fc region (Fc-LALA with L234A and L235A mutations in the Fc region of human IgG1, US7183076B2) to form scFv-Fc-LALA antibodies or to a modified version of an antibody Fc region (LALA with L234A mutation and L235A mutation in the Fc region of human IgG1) to form antibodies in IgG1-LALA format (US7183076B2), which were expressed in ExpiCHO cells (Thermo Fisher Scientific), and the antibodies were affinity purified using Protein A column.

From these antibodies, (2-1) 6-3 antibody and (2-2) 27-3 antibody were selected.

### Examples 4: Production of Anti-Human CD39 Antibody (Phage Display Method)

In this example, mRNA of the monoclonal antibody that binds specifically to human CD39 was obtained from the spleen or lymph node of the immunized mice prepared in Example 3, and monoclonal antibodies that bind specifically to human CD39 was produced by a phage display method.

Total RNA was extracted from the spleen or lymph node of the immunized mice prepared in Example 3, and scFv phage library (size: about 1 × 10⁸ clones) was constructed by the same method as the phage display method described in Example 1.

The scFv antibody phage library was subjected to panning with recombinant human CD39, Phagemid after the panning was extracted, and scFv regions were amplified by PCR, and transferred onto scFv secretory expression vectors. E. coli transfected with scFv secretory expression vectors were screened for scFv clones that inhibit CD39 activity using IPTG-induced secretion of scFv into the culture supernatant.

The nucleotide sequences of the scFvs that were positive in the screening were then sequenced and connected by a linker to a modified version of an antibody Fc region (Fc-LALA with L234A and L235A mutations in the Fc region of human IgG1) to form scFv-Fc-LALA antibodies or to a modified version of an antibody Fc region (LALA with L234A mutation and L235A mutation in the Fc region of human IgG1) to form antibodies in IgG1-LALA format, which were expressed in ExpiCHO cells (Thermo Fisher Scientific), and the antibodies were affinity purified using Protein A column.

From these antibodies, (2-3) P4 antibody, (2-4) T86 antibody, (2-5) I67 antibody, (2-6) Tre291 antibody and (2-7) mAnE67 antibody were selected.

### Example 5: Humanization of Mouse Antibodies

In this example, the mouse-derived anti-TIM-3 antibody produced in Example 1 and the mouse-derived anti-CD39 antibody produced in Example 4 were humanized.

The mouse antibodies were humanized by a CDR grafting method. Sequences having high homology with the framework sequences of the VH genes and the Vkappa genes of the mouse antibodies were selected from known human antibody sequences, to which the CDRs of the mouse antibodies were grafted to produce humanized VH genes and Vkappa genes.

If necessary, structurally important sites in the framework (Canonical, Vernier, Interface) were appropriately replaced with sequences from the original mouse antibody.

From a plurality of combinations of the humanized VH gene and the Vkappa gene, those having equal or higher affinity and physiological activity compared to the original mouse antibodies were selected as final humanized sequences.

The anti-TTM-3 antibodies, (1-1) 003 Antibody, (1-2) 149 antibody, (1-3) 428 antibody, (1-4) 621 antibody and (1-5) 072 antibody, were humanized by the above method to obtain Hu003_13_F20 antibody from (1-1) 003 antibody, Hu149_83 antibody from (1-2) 149 antibody, Hu428_83 antibody from (1-3) 428 antibody, Hu621_83 antibody from (1-4) 621 antibody, and Hu072_11 antibody and Hu072_13 antibody from (1-5) 072 antibody.

The anti-CD39 antibodies, (2-1) 6-3 Antibody, (2-2) 23-7 antibody and (2-3) P4 antibody, were humanized by the above method to obtain 6-3hIB antibody, 6-3hIG antibody, 6-3hIF antibody, 6-3hCB antibody, 6-3hHB antibody and 6-3hKB antibody from (2-1) 6-3 antibody, B23-7hIB antibody, B23-7hIG antibody, B23-7hIF antibody, B23-7hCB antibody, B23-7hHB antibody and B23-7hKB antibody from (2-2) 23-7 antibody, and HuP4_11 antibody, HuP4_41 antibody, HuP4_51 antibody and HuP4_IB antibody from (2-3) P4 antibody.

### Example 6: Identification of Sequences of Anti-Human TIM-3 Antibodies and Anti-Human CD39 Antibodies

In this example, the amino acid sequences of the antibodies obtained in Examples 1, 3, 4 and 5 were identified.

All of (1-1) 003 antibody, (1-2) 149 antibody, (1-3) 428 antibody, (1-4) 621 antibody and (1-5) 072 antibody obtained in Example 1 were humanized in Example 5. In this example, the amino acid sequences of these humanized antibodies were identified.

In addition, (2-1) 6-3 antibody, (2-2) 27-3 antibody and (2-3) P4 antibody obtained in Example 3 were humanized in Example 5. In this example, the amino acid sequences of these humanized antibodies were identified.

For other antibodies (i.e., (2-4) T86 antibody, (2-5) I67 antibody, (2-6) Tre291 antibody and (2-7) mAnE67 antibody obtained in Example 4), the amino acid sequences of mouse antibodies were identified.

The amino acid sequences of CDRs of the anti-TIM-3 antibodies and anti-CD39 antibodies were identified as follows:

**[Table 1]**

| CDRs of anti-TIM-3 antibodies | | | |
|---|---|---|---|
| Antibody VH | VH-CDR1 | VH-CDR2 | VH-CDR3 |
| (1-1) Hu003_13 F20 | SYYMS | TISNSGGSTYYPDSVKD | DPYYTNYVPMDY |
| (1-2) Hu149 83 | DYYMD | YIYPNKGGTSYNQKFKG | SGYGNYYTMDY |
| (1-3) Hu428 83 | DYYMD | YIYPNKGGTSYNQKFKG | GGYYSYYSYDY |
| (1-4) Hu621 83 | DYYMD | YIYPNKGGTSYNQKFKG | SGYKAYYAMDY |
| (1-5) Hu072_11 | SYYMS | I TISNSGGSTYYPDSVKD | DPYYTNYVPMDY |

| Antibody VK | VK-CDR1 | VK-CDR2 | VK-CDR3 |
|---|---|---|---|
| (1-1) Hu003_13 F20 | KASQYVDTYVA | SASTRHT | AQYSSSPLT |
| (1-2) Hu149 83 | KASQSVGNNVA | YASNRYT | QQHYSSPST |
| (1-3) Hu428 83 | KASQSVGNNVA | YASNRYT | QQHYSSPYT |
| (1-4) Hu621 83 | KASQSVGNNVA | YASNRYT | QQHYSSPYT |
| (1-5) Hu072 11 | KASENVGTYVS | GASNRYT | GQSYSYPLT |

The amino acid sequences of the VH and VL regions of these anti-TIM-3 antibodies were as follows (underlines indicate CDR sequences).

**[Table 2-1]**

| Variable regions of anti-TIM-3 antibodies |
|---|
| <Hu003_13_F20> |
| VH region: QVQLQESGGG LVKPGGSLKL SCAASGFTFS SYYMSWVRQT PDKRLEWVAT ISNSGGSTYY PDSVKDRFTI SRDNAKNTLY LQMSSLKSED TAMYYCARDP YYTNYVPMDY WGQGTMVTVS S (SEQ ID NO: 7) |
| VK region: DIVMTQSPSF LSASVGDRVT ITCKASQYVD TYVAWYQQRP GKAPKPLIYS ASTRHTGVPS RFSGSGSGTE FTLTISSLQP EDFATYFCAQ YSSSPLTFGG GTKLELK (SEQ ID NO: 8) |
| <Hu149_83> |
| VH region: QVQLVQSGAE VKKPGASVKV SCKASGYTFT DYYMDWVRQA PGQGLEWLGY IYPNKGGTSY NQKFKGRVTM TTDKSTSTAY MELRSLRSDD TAVYYCARSG YGNYYTMDYW GQGTTVTVSS (SEQ ID NO: 15) |
| VK region: DIVMTQSQKF MSTSVGDRVS ITCKASQSVG NNVAWYQQKP GQSPKLLIYY ASNRYTGVPD RFTGSGSGTD FTLTISNMQS EDLADYFCQQ HYSSPSTFGG GTKLEIK (SEQ ID NO: 16) |
| <Hu428_83> |
| VH region: QVQLVQSGAE VKKPGASVKV SCKASGYTFT DYYMDWVRQA PGQGLEWLGY IYPNKGGTSY NQKFKGRVTM TTDKSTSTAY MELRSLRSDD TAVYYCARGG YYSYYSYDYW GQGTTVTVSS (SEQ ID NO: 23) |
| VK region: DIVMTQSQKF MSTSVGDRVS ITCKASQSVG NNVAWYQQKP GQSPKLLIYY ASNRYTGVPD RFTGSGSGTD FTLTISNMQS EDLADYFCQQ HYSSPYTFGG GTKLEIK (SEQ ID NO: 24) |
| <Hu621_83> |
| VH region: QVQLVQSGAE VKKPGASVKV SCKASGYTFT DYYMDWVRQA PGQGLEWLGY IYPNKGGTSY NQKFKGRVTM TTDKSTSTAY MELRSLRSDD TAVYYCARSG YKAYYAMDYW |
| GQGTTVTVSS (SEQ ID NO: 31) |
| VK region: DIVMTQSQKF MSTSVGDRVS ITCKASQSVG NNVAWYQQKP GQSPKLLIYY ASNRYTGVPD RFTGSGSGTD FTLTISNMQS EDLADYFCQQ HYSSPYTFGG GTKLEIK (SEQ ID NO: 32) |
| <Hu072_11> |
| VH region: QVQLQESGGG LVKPGGSLKL SCAASGFTFS SYYMSWVRQT PDKRLEWVAT ISNSGGSTYY PDSVKDRFTI SRDNAKNTLY LQMSSLKSED TAMYYCARDP YYTNYVPMDY WGQGTMVTVS S (SEQ ID NO: 39) |
| VK region: DIVMTQSPDS LAVSLGERVT LSCKASENVG TYVSWYQQKP RQPPKLLIYG ASNRYTGVPD RFSGSGSGTD FTLTISSLQA EDVALYYCGQ SYSYPLTFGQ GTRLEIK (SEQ ID NO: 40) |

In addition to these, a humanized anti-TIM-3 antibody having the following amino acid sequences of VH and VL regions was obtained (underlines indicate CDR sequences).

**[Table 2-2]**

| Variable region of humanized anti-TIM-3 antibody |
|---|
| <Hu072_13> |
| VH region: QVQLQESGGG LVKPGGSLKL SCAASGFTFS SYYMSWVRQT PDKRLEWVAT ISNSGGSTYY PDSVKDRFTI SRDNAKNTLY LQMSSLKSED TAMYYCARDP YYTNYVPMDY WGQGTMVTVS S (SEQ ID NO: 125) |
| VK region: NIVMTQSPDS LAVSLGERAT INCKASENVG TYVSWYQQKP GQPPKLLIYG ASNRYTGVPD RFSGSGSGTD FTLTISSLQA EDVAVYYCGQ SYSYPLTFGQ GTKLEIK (SEQ ID NO: 126) |

The amino acid sequences of CDRs of the anti-CD39 antibodies were identified as follows.

**[Table 3]**

| CDRs of anti-CD39 antibodies | | | |
|---|---|---|---|
| Antibody VH | VH-CDR1 | VH-CDR2 | VH-CDR3 |
| (2-1) 6-3hIG | GFSIKDTY | IDPANVNT | ALYGYDDDAYYFDY |
| (2-2) B23-7hIF | GYSFTDYN | IDPYSGGT | GLYGYDDDANYFDD |
| (2-3) HuP4_IB | NYGVH | VILRRGSTDYNAAFMS | TAWAGDYFDY |
| (2-4) T86 | NYGMN | WINTYTGEPTYADDFKG | KGYYGYPNYYAMDY |
| (2-5) 167 | SYGVH | VIWRRGSTDYNAAFMS | GISTATSWFAY |
| (2-6) Tre291 | SYGVH | VIWRRGSTDYNAAFMS | VRGDAMDY |
| (2-7) mAnE67 | DYGMH | YISSGSSIIYYADTVKG | KDYPYAMDY |

| Antibody VK | VL-CDR1 | VL-CDR2 | VL-CDR3 |
|---|---|---|---|
| (2-1) 6-3hIG | ESVDNYGISF | AAS | QQSKEVPYT |
| (2-2) B23-7hIF | SSVSY | ATS | QQRSSYPLT |
| (2-3) HuP4_IB | KASQNVGTNVA | SASYRYS | QQYNSYPLT |
| (2-4) T86 | KASQNVGTAVA | SASNRYT | QQYSSYPIT |
| (2-5) 167 | KASQNVGTNVA | SASYRYS | QQYNSYPLT |
| (2-6) Tre291 | KASQNVGTNVA | SASYRYS | QQYNSYPLT |
| (2-7) mAnE67 | KASQNVGTNVA | WASNRFT | QQYSSSPYT |

The amino acid sequences of the VH and VL regions of these anti-CD39 antibodies were as follows (underlines indicate CDR sequences).

**[Table 4-1]**

| Variable regions of anti-CD39 antibodies |
|---|
| <6-3hIG> |
| VH region: QVQLVQSGAE LVKPGASVKL SCKASGFSIK DTYVHWVKQA PGKGLEWIGK IDPANVNTKF DPKFQGRVTI TADTSTNTAY LQLSSLRSED TAVYYCALYG YDDDAYYFDY WGQGTLVTVS S (SEQ ID NO: 47) |
| VK region: EIVLTQSPAS LSVSPGERAT ISCRASESVD NYGISFMNWF QQKPGQAPKL LIYAASNQGS GVPARFSGSG SGTDFTLTIS SLEPEDFAVY YCQQSKEVPY TFGQGTKLEI K (SEQ ID NO: 48) |
| <B23-7hIF> |
| VH region: EVQLVQSGGE LVKPGASVKI SCKVSGYSFT DYNLNWVRQA PGKGLEWVGN IDPYSGGTRY NQKFKGRFTI TIDQSKNTAY MQLNSLRSED TAVYYCGLYG YDDDANYFDD WGQGTLVTVS S (SEQ ID NO: 55) |
| VK region: EIVLTQSPAS LSASPGERVT ISCSASSSVS YMHWFQQKPG QAPKLWIYAT SNLASGVPAR FSGSGSGTDY TLTISSMEPE DFATYYCQQR SSYPLTFGQG TKVEIK (SEQ ID NO: 56) |
| <HuP4_IB> |
| VH region: QVQLLESGPE LVKPSATLSI TCTVSGFSLT NYGVHWIRQA PGKGLEWLGV ILRRGSTDYN AAFMSRFTIS KDNSKNQVYF KLRSVTAEDT AVVAGDYFDY WGQGTMVTVS S S (SEQ ID NO: 123) |
| VK region: DIQMTQSHST LSLSVGDRVT ITCKASQNVG TNVAWYQQKP GQPPKALIYS ASYRYSGVPS RFSGSGSGTD FTLTISSVQA EDVAVYYCQQ YNSYPLTFGP GTKLEIK (SEQ ID NO: 124) |
| <T86> |
| VH region: QIQLVQSGPE LKKPGETVKI SCEASGYTFI NYGMNWVKQA PGKGLKWMGW INTYTGEPTY ADDFKGRFAF SLETSANTAY LQINNLKNED TATYFCARKG YYGYPNYYAM DYWGQGTSVT VSS (SEQ ID NO: 71) |
| VK region: DIVMTQSQKF MSTSVGDRVS ITCKASQNVG TAVAWYQQKP GQSPKLLIYS ASNRYTGVPD RFTGSGSGTD FTLTISNMQS EDLADYFCQQ YSSYPITFGT GTKLEIK (SEQ ID NO: 72) |

**[Table 4-2]**

| |
|---|
| <I67> |
| VH region: DVQLQESGPS LVQPSQSLSI NCTVSGFSLS SYGVHWVRQS PGKGLEWLGV IWRRGSTDYN AAFMSRLSIT KDNSKSQVFF KMNRLQADDT ATYYCAKGIS TATSWFAYWG QGTLVTVSA (SEQ ID NO: 79) |
| VK region: DIVMTQSHKF MSTSVGDRVS VTCKASQNVG TNVAWYQQKP GQSPKALIYS ASYRYSGVPD RFTGSGSGTD FTLTISNVQS EDLAEYFCQQ YNSYPLTFGA GTKLELK (SEQ ID NO: 80) |
| <Tre291> |
| VH region: QVQLKQSGPG LVQPSQSLSI TCTVSGFSLT SYGVHWVRQS PGKGLEWLGV IWRRGSTDYN AAFMSRLSIT KDTSKSQVFF KMNSLQTDDT AIYYCAGVRG DAMDYWGQGT SVTVSS (SEQ ID NO: 87) |
| VK region: DIVMTQSHKS MSTSVGDRVS VTCKASQNVG TNVAWYQQKP GQSPKALIYS ASYRYSGVPD RFTGSGSGTD FTLTISNVQS EDLAEYFCQQ YNSYPLTFGG GTKLEIK (SEQ ID NO: 88) |
| <mAnE67> |
| VH region: EVQLVESGGG LVKPGGSRKL SCAASGFTFS DYGMHWVRQA PEKGLEWVAY ISSGSSIIYY ADTVKGRFTI SRDNAKNTLF LQMTGLRSED TAMYYCARKD YPYAMDYWGQ GTSLAVSS (SEQ ID NO: 95) |
| VK region: SIVMTQTPKF MSTSVGDRVS VTCKASQNVG TNVAWYQQKP GQSPKALIYW ASNRFTGVPD RFTGSGSGTD FTLTISNVQS EDLADYFCQQ YSSSPYTFGS GTKLEIK (SEQ ID NO: 96) |

In addition to these, humanized anti-CD39 antibodies having the following amino acid sequences of VH and VL regions were obtained (underlines indicate CDR sequences).

**[Table 5-1]**

| Variable regions of humanized anti-CD39 antibodies |
|---|
| <6-3hIB> |
| VH region: QVQLLQSGAE VKKPGATVKI SCKVSGFSIK DTYVHWVRQP PEQGLEWIGK IDPANVNTKF DPKFQGRLTI TADTSKNTAY LELSSLRSED TAVYYCALYG YDDDAYYFDY WGQGTLLTVS S (SEQ ID NO: 97) |
| VK region: DIVLTQSPAT LALSVGQRAT LSCRASESVD NYGISFMNWF QQKPGQPPRL LIYAASNQGS GIPARFSGSG SGTDFTLTIN PLESEDFAVY YCQQSKEVPY TFGPGTKLEI K (SEQ ID NO: 98) |
| <6-3hIF> |
| VH region: QVQLVQSGAE LVKPGASVKL SCAASGFSIK DTYVHWVRQA PGKGLEWVGK IDPANVNTKF DPKFQGRFTI TADTSKNTAY LQLSSLRSED TAVYYCALYG YDDDAYYFDY WGQGTLVTVS S (SEQ ID NO: 99) |
| VK region: DIVLTQSPAS LSVSPGDRAT ISCRASESVD NYGISFMNWF QQKPGQAPKL LIYAASNQGS GVPARFSGSG SGTDFTLTIS SLEPEDFAVY YCQQSKEVPY TFGQGTKLEI K (SEQ ID NO: 100) |
| <6-3hCB> |
| VH region: QVQLLQSGAE VVKPGATVKI SCKVSGFSIK DTYVHWVRQP PGQGLEWIGK IDPANVNTKF DPKFQGRFTI TADTSINTAY LELSSLTSED TAVYYCALYG YDDDAYYFDY WGQGTLVTVS S (SEQ ID NO: 101) |
| VK region: NIVLTQSPAT LSLSLGQRAT ISCRASESVD NYGISFMNWF QQKPGQPPQL LIYAASNQGS GIPARFSGSG SGTDFTLNIN PLESEDFAVY YCQQSKEVPY TFGPGTKLEI K (SEQ ID NO: 102) |
| <6-3hHB> |
| VH region: QVQLQQSGAE LKKPGASVKI SCKVSGFSIK DTYVHWVRQP PGKGLEWIGK IDPANVNTKF DPKFQGRFTI TADTSTDTAY LELSSLRSED TAVYYCALYG YDDDAYYFDY WGQGTMLTVS S (SEQ ID NO: 103) |
| VK region: DIVLTQSPAT LSLSLGQRAT LSCRASESVD NYGISFMNWF QQKPGQPPRL LIYAASNQGS GIPARFSGSG SGTDFTLNIT PLESEDFAVY YCQQSKEVPY TFGPGTKLEL K (SEQ ID NO: 104) |

**[Table 5-2]**

| |
|---|
| <6-3nkB> |
| VH region: QVQLLQSGAE VVKPGATVKI SCKVSGFSIK DTYVHWVRQR PERGLEWIGK IDPANVNTKF DPKFQGRLTI TADTSTDTAY LELSSLRSED TAVYYCALYG YDDDAYYFDY WGQGTLLTVS S (SEQ ID NO: 105) |
| VK region: DIVLTQSPAT LSLSPGQRAT ISCRASESVD NYGISFMNWF QQKPGQPPRL LIYAASNQGS GIPARFSGSG SGTDFTLNIH PLESEDFAVY YCQQSKEVPY TFGPGTKLEI |
| K (SEQ ID NO: 106) |
| <B23-7hIB> |
| VH region: EVQLLQSGPE VVKPGATVKI SCKVSGYSFT DYNLNWVKQP PGKNLEWIGN IDPYSGGTRY NQKFKGRFTI TIDQSINTAY MELRSLTSED TAVYYCGLYG YDDDANYFDD WGQGTMLTVS S (SEQ ID NO: 107) |
| VK region: EIVLTQSPAI LSPSPGERVT LNCSASSSVS YMHWFQQKPG QAPRLWIYAT SNLASGIPAR FSGSGSGTEY TLTISRMESE DFAVYYCQQR SSYPLTFGQG TKLEIK (SEQ ID NO: 108) |
| <B23-7hIG> |
| VH region: EVQLVQSGAE LKKPGASVKI SCKVSGYSFT DYNLNWVKQA PGKGLEWIGN IDPYSGGTRY NQKFKGRVTI TIDQSTSTAY MELSSLRSED TAVYYCGLYG YDDDANYFDD WGQGTLVTVS S (SEQ ID NO: 109) |
| VK region: EIVLTQSPAT LSLSPGERAT ISCSASSSVS YMHWFQQKPG QAPKLWIYAT SNLASGVPAR FSGSGSGTDY TLTISSMEPE DFAVYYCQQR SSYPLTFGGG TKVEIK (SEQ ID NO: 110) |
| <B23-7hCB> |
| VH region: EVQLVQSGPE VKKPGATVKI SCKVSGYSFT DYNLNWVKQP PGKNLEWIGN IDPYSGGTRY NQKFKGRFTI TIDQSINTAY MHLRSLRSED TAVYYCGLYG YDDDANYFDD WGQGTMLTVS S (SEQ ID NO: 111) |
| VK region: EIVLTQSPAT LSPSPGERVT INCSASSSVS YMHWFQQKPG TPPRLWIYAT SNLASGIPAR FSGSGSGTEY TLTISSMESE DFAVYYCQQR SSYPLTFGPG TKVEIK (SEQ ID NO: 112) |

**[Table 5-3]**

| |
|---|
| <B23-7hHB> |
| VH region: EVQLLQSGPE VVKPGATVKI SCKVSGYSFT DYNLNWVRQY PGKNLEWVGN IDPYSGGTRY NQKFKGRFTI TIDQSKNTAY MELRSLTSED TAVYYCGLYG YDDDANYFDD WGQGTMLTVS S (SEQ ID NO: 113) |
| VK region: EIVLTQSPAI LSLSPGERVT ITCSASSSVS YMHWFQQKPG QAPRLWIYAT SNLASGIPAR FSGSGSGTEY TLTISSMESE DFAVYYCQQR SSYPLTFGQG TKLEIK (SEQ ID NO: 114) |
| <B23-7hKB> |
| VH region: EVQLLQSGAE VVKPGATVKI SCKVSGYSFT DYNLNWVKQP NGKNLEWIGN IDPYSGGTRY NQKFKGRFTI TIDQSINTAY MQLNSLRSED TAVYYCGLYG YDDDANYFDD WGQGTLLTVS S (SEQ ID NO: 115) |
| VK region: EIVLTQSPAI LSPSPGERVT LTCSASSSVS YMHWFQQKPG KPPRLWIYAT SNLASGIPAR FSGSGSGTEY TLTISRMESE DFAVYYCQQR SSYPLTFGQG TKLEIK (SEQ ID NO: 116) |
| <HuP4_11> |
| VH region: EVQLVESGGA LVQPGRSLRL SCAVSGFTLS NYGVHWVRQA PGKGLEWVAV ILRRGSTDYN AAFMSRFTIS KDNSKNTVYL QMNSLRADDT AVVAGDYFDY WGQGTTVTVS S (SEQ ID NO: 119) |
| VK region: DIVMTQSQKF MSTSVGDRVS ITCKASQNVG TNVAWYQQKP GQSPKLLIYS ASYRYSGVPD RFTGSGSGTD FTLTISNMQS EDLADYFCQQ YNSYPLTFGG GTKLEIK (SEQ ID NO: 120) |
| <HuP4_41> |
| VH region: QVQLVESGGG VVQPGGSLRL SCVVSGFTLT NYGVHWVRQA PGKGLEWLAV ILRRGSTDYN AAFMSRLAIS KDNSKNTVYL ELNSLRLDDT AVVAGDYFDY WGQGTTVTVS S (SEQ ID NO: 63) |
| VK region: DIVMTQSQKFMSTSVGDRVSITCKASQNVGTNVAWYQQKPGQSPKLLIYSASYRYSGVPDRFTGSG SGTDFTLTISNMQSEDLADYFCQQYNSYPLTFGGGTKLEIK (SEQ ID NO: 64) |
| <HuP4_51> |
| VH region: QVQLVESGGG VVQPGGSLRL SCVVSGFSLT NYGVHWVRQA PGKGLEWLGV ILRRGSTDYN AAFMSRLAIS KDNSKNTVYL ELNSLRLDDT AVVAGDYFDY WGQGTTVTVS S (SEQ ID NO: 121) |
| VK region: DIVMTQSQKF MSTSVGDRVS ITCKASQNVG TNVAWYQQKP GQSPKLLIYS ASYRYSGVPD RFTGSGSGTD FTLTISNMQS EDLADYFCQQ YNSYPLTFGG GTKLEIK (SEQ ID NO: 122) |

### Example 7: Production of Anti-Human TIM-3/Anti-Human CD39 Bispecific Antibody

(7-1) In this example, bispecific antibodies that bind to both human TIM-3 and human CD39 were produced in scFv-Fc format.

Two types of antibody half-molecules, one of which is anti-human TIM-3 scFv and the other of which is anti-human CD39 scFv, were expressed on ExpiCHO cells (ThermoFisher Scientific) as a scFv-Fc heterodimer in Knob-into-Hole format, based on the human IgG1LALA H chain Fc region Knob sequence (US7183076B2) or human IgG1LALA H chain Fc region Knob sequence_Ctag (US7183076B2) and the human IgG1LALA H chain Fc region Hole sequence (US7183076B2).

When the human IgG1LALA H chain Fc region Knob sequence was used, the bispecific antibody was affinity purified with a Protein A column, the heterodimer was purified by ion-exchange chromatography, and the dimer fraction was then separated by size-exclusion chromatography. When the human IgG1LALA H chain Fc region Knob sequence_Ctag was used, the antibody was affinity purified using an anti-C-tag antibody column.

The produced bispecific antibodies of scFv-Fc structure are such that, in the written form, the former antibody half molecule is that of Hole type, and the latter antibody half-molecule is that of Knob type, where the antibody using the human IgG1LALA H chain Fc region Knob sequence_Ctag is indicated by the additional notation "_ct". The scFv-Fc homodimer antibody produced as a control antibody is indicated by "_sc".

(7-2) In this example, bispecific antibodies of another structure which bind to both human TIM-3 and human CD39 were also produced in Fab-scFv-Fc format.

Two types of half-molecules, one of which is anti-human TIM-3 Fab and the other of which is anti-human CD39 scFv, or two types of half-molecules, one of which is anti-human TIM-3 scFv and the other of which is anti-human CD39 Fab, were expressed in ExpiCHO cells (ThermoFisher Scientific) as a Fab-scFv-Fc heterodimer in Knob-into-Hole format, based on a structure in which the human IgG1LALA H chain Fc region Knob sequence (US7183076B2) is on the Fab side and the human IgG1LALA H chain Fc region Hole sequence (US7183076B2) is on the scFv side.

For purification of the bispecific antibody, the heterodimer was affinity purified with a CH1 antibody column (ThermoFisher Scientific), or affinity purified with a Protein A column, the heterodimer was purified by ion-exchange chromatography, and the dimer fraction was then separated by size-exclusion chromatography.

The produced bispecific antibodies of Fab-scFv-Fc structure, which are indicated by the additional notation "_Fsc", are different from those of (7-1) in that, in the written form, the former antibody half-molecule is that of Knob type and the latter antibody half-molecule is that of Hole type.

### Example 8: Evaluation of Affinity for Recombinant Antigen

(8-1) In this example, the binding affinity of the bispecific antibodies produced in Example 7 (7-1) to human TIM-3 recombinant antigen or human CD39 recombinant antigen was evaluated.

The bispecific antibodies that were used in this example are as follows:
6-3hIG_Hu003_Ct: scFv-Fc antibody consisting of the half-molecule of anti-CD39_scFv antibody (humanized) 6-3hIG (Hole type) and the half-molecule of anti-TIM-3_scFv antibody (humanized) Hu003 (Knob type with Ctag);
HuP4_41_Hu003_Ct: scFv-Fc antibody consisting of the half-molecule of anti-CD39_scFv antibody (humanized) HuP4_41 (Hole type) and the half-molecule of anti-TIM-3_scFv antibody (humanized) Hu003 (Knob type with Ctag);
HuP4_IB_Hu003: scFv-Fc antibody consisting of the half-molecule of anti-CD39_scFv antibody HuP4_IB (Hole type) and the half-molecule of anti-TIM-3_scFv antibody (humanized) Hu003 (Knob type);
T86_Hu003_Ct: scFv-Fc antibody consisting of the half-molecule of anti-CD39_scFv antibody T86 (Hole type) and the half-molecule of anti-TIM-3_scFv antibody (humanized) Hu003 (Knob type with Ctag);
I67_Hu003_Ct: scFv-Fc antibody consisting of the half-molecule of anti-CD39_scFv antibody I67 (Hole type) and the half-molecule of anti-TIM-3_scFv antibody (humanized) Hu003 (Knob type with Ctag);
Tre291_Hu003_Ct: scFv-Fc antibody consisting of the half-molecule of anti-CD39_scFv antibody Tre291 (Hole type) and the half-molecule of anti-TIM-3_scFv antibody (humanized) Hu003 (Knob type with Ctag);
mAnE67_Hu003_Ct: scFv-Fc antibody consisting of the half-molecule of anti-CD39_scFv antibody mAnE67 (Hole type) and the half-molecule of anti-TIM-3_scFv antibody (humanized) Hu003 (Knob type with Ctag); and
Hu072_11_HuP4_41_Ct: scFv-Fc antibody consisting of the half-molecule of anti-CD39_scFv antibody Hu072_11_(Knob type with Ctag) and the half-molecule of anti-TIM-3_scFv antibody (humanized) HuP4_41 (Hole type).

The monospecific antibodies used as controls are as follows:
Hu003_13_F20_sc: scFv-Fc homodimeric antibody of anti-TIM-3_scFv antibody (humanized) Hu003_13_F20;
Hu072_11_sc: scFv-Fc homodimeric antibody of anti-TIM-3_scFv antibody Hu072_11;
Hu003_13_F20_LA: IgG1LALA homodimeric antibody of anti-TIM-3_scFv antibody (humanized) Hu003_13_F20;
6-3hIG_sc: scFv-Fc homodimeric antibody of anti-CD39_scFv antibody (humanized) 6-3hIG;
6-3hIG_LA: IgG1LALA homodimeric antibody of anti-CD39_scFv antibody (humanized) 6-3hIG;
HuP4_41_sc: scFv-Fc homodimeric antibody of anti-CD39_scFv antibody HuP4_41;
HuP4_IB_sc: scFv-Fc homodimeric antibody of anti-CD39_scFv antibody HuP4_IB;
HuP4_IB_LA: IgG1LALA homodimeric antibody of anti-CD39_scFv antibody HuP4_IB;
T86_sc: scFv-Fc homodimeric antibody of anti-CD39_scFv antibody T86;
I67_sc: scFv-Fc homodimeric antibody of anti-CD39_scFv antibody I67;
Tre_291_sc: scFv-Fc homodimeric antibody of anti-CD39_scFv antibody Tre_291; and
mAnE67_sc: scFv-Fc homodimeric antibody of anti-CD39_scFv antibody mAnE67.

The affinity of the monoclonal antibodies and the anti-human TIM-3/CD39 bispecific antibodies of the present invention for the recombinant antigen was measured by surface plasmon resonance (SPR) interaction analysis using Biacore8K.

The antibodies were captured on a sensor chip CM5 immobilized with the anti-human IgG antibody (Cytiva), and recombinant human TIM-3 (rhTim-3-His: SinoBological, 10390-H08H) or recombinant human CD39 (in-house product, 38 to 478 aa of Accession No. NM_001776.6) was used as the analyte.

The dissociation constant (KD = kd/ka) was calculated based on the binding rate constant (ka) and dissociation rate constant (kd) of each antibody to human TIM-3 obtained by surface plasmon resonance.

The results are shown in Tables 6-1 and 6-2. The anti-human TIM-3/CD39 bispecific antibodies and the monoclonal antibodies of the present invention were exhibited to bind to the recombinant human TIM-3 and the recombinant human CD39 with the following dissociation constants (KD values).

**[Table 6-1]**

| Affinity for rhTIM-3 Bispecific antibodies | | | | |
|---|---|---|---|---|
| Clone | Format | ka (1/Ms) | kd (1/s) | KD (M) |
| 6-3hIG_Hu003_Ct | scFv-Fc | 2.44E+05 | 1.39E-03 | 5.71E-09 |
| HuP4_41_Hu003_Ct | scF-vFc | 2.41E+05 | 1.37E-03 | 5.66E-09 |
| HuP4_IB_Hu003 | scFv-Fc | 3.04E+05 | 1.68E-03 | 5.53E-09 |
| T86_Hu003_Ct | scFv-Fc | 2.14E+05 | 1.26E-03 | 5.90E-09 |
| I67_Hu003_Ct | scFv-Fc | 2.03E+05 | 1.40E-03 | 6.89E-09 |
| Tre291_Hu003_Ct | scFv-Fc | 2.27E+05 | 1.20E-03 | 5.28E-09 |
| mAnE67_Hu003_Ct | scFv-Fc | 2.44E+05 | 1.17E-03 | 4.80E-09 |
| Hu072_11_HuP4_41_Ct | scFv-Fc | 3.74E+05 | 2.36E-01 | 6.29E-07 |

| Monospecific antibodies | | | | |
|---|---|---|---|---|
| Clone | Format | ka (1/Ms) | kd (1/s) | KD (M) |
| Hu003_13_F20_sc | scFv-Fc | 2.25E+05 | 1.30E-03 | 5.79E-09 |
| Hu072_11_sc | scFv-Fc | 2.65E+05 | 2.33E-01 | 8.80E-07 |
| Hu003_13_F20_LA | IgG1LALA | 3.05E+05 | 1.44E-03 | 4.71E-09 |

**[Table 6-2]**

| Affinity for rhCD39 | | | | |
|---|---|---|---|---|
| Bispecific antibodies | | | | |
| Clone | Format | ka (1/Ms) | kd (1/s) | KD (M) |
| 6-3hIG_Hu003_Ct | scFv-Fc | 5.57E+05 | 1.98E-04 | 3.55E-10 |
| HuP4_41_Hu003_Ct | scFv-Fc | 8.87E+05 | 9.80E-05 | 1.10E-10 |
| HuP4_IB_Hu003 | scFv-Fc | 1.50E+06 | 5.00E-04 | 3.34E-10 |
| T86_Hu003_Ct | scFv-Fc | 3.63E+05 | 6.32E-04 | 1.74E-09 |
| I67_Hu003_Ct | scFv-Fc | 1.70E+06 | 2.78E-03 | 1.63E-09 |
| Tre291_Hu003_Ct | scFv-Fc | 1.04E+06 | 2.90E-03 | 2.79E-09 |
| mAnE67_Hu003_Ct | scFv-Fc | 5.57E+05 | 1.26E-03 | 2.26E-09 |
| Hu072_11_HuP4_41_Ct | scFv-Fc | 7.53E+05 | 1.11E-04 | 1.47E-10 |

| Monospecific antibodies | | | | |
|---|---|---|---|---|
| Clone | Format | ka (1/Ms) | kd (1/s) | KD (M) |
| 6-3hIG_sc | scFv-Fc | 4.02E+05 | 1.87E-04 | 4.65E-10 |
| 6-3hIG_LA | IgG1LALA | 3.07E+05 | 1.93E-04 | 6.29E-10 |
| HuP4_41_sc | scFv-Fc | 1.66E+06 | 1.76E-04 | 1.06E-10 |
| HuP4_IB_sc | scFv-Fc | 9.51E+05 | 4.17E-04 | 4.39E-10 |
| HuP4_IB_LA | IgG1LALA | 1.17E+06 | 4.66E-04 | 3.98E-10 |
| T86_sc | scFv-Fc | 1.92E+05 | 8.23E-04 | 4.28E-09 |
| I67_sc | scFv-Fc | 1.78E+06 | 2.94E-03 | 1.65E-09 |
| Tre_291_sc | scFv-Fc | 1.40E+06 | 3.10E-03 | 2.22E-09 |
| mAnE67_sc | scFv-Fc | 3.93E+05 | 1.87E-03 | 4.75E-09 |

(8-2) In this example, the binding affinity of the bispecific antibodies produced in Example 7 (7-2) to human TIM-3 recombinant antigen or human CD39 recombinant antigen was evaluated.

The bispecific antibodies that were used in this example are as follows:
6-3hIG_Hu003_Fsc: Fab-scFv-Fc antibody consisting of the half-molecule of anti-CD39 Fab antibody (humanized) 6-3hIG (Knob type) and the half-molecule of anti-TIM-3 scFv antibody (humanized) Hu003 (Hole type);
HuP4_IB_Hu003_Fsc: Fab-scFv-Fc antibody consisting of the half-molecule of anti-CD39 Fab antibody HuP4_IB (humanized) (Knob type) and the half-molecule of anti-TIM-3 scFv antibody (humanized) Hu003 (Hole type);
Hu003_HuP4_IB_Fsc: Fab-scFv-Fc antibody consisting of the half-molecule of anti-TIM-3 Fab antibody (humanized) Hu003 (Knob type) and the half-molecule of anti-CD39 scFv antibody HuP4_IB (humanized) (Hole type);
T86_Hu003_Fsc: Fab-scFv-Fc antibody consisting of the half-molecule of anti-CD39 Fab antibody T86 (Knob type) and the half-molecule of anti-TIM-3 scFv antibody (humanized) Hu003 (Hole type);
I67_Hu003_Fsc: Fab-scFv-Fc antibody consisting of the half-molecule of anti-CD39 Fab antibody I67 (Knob type) and the half-molecule of anti-TIM-3 scFv antibody (humanized) Hu003 (Hole type);
Tre291_Hu003_Fsc: Fab-scFv-Fc antibody consisting of the half-molecule of anti-CD39 Fab antibody Tre291 (Knob type) and the half-molecule of anti-TIM-3 scFv antibody (humanized) Hu003 (Hole type); and
mAnE67_Hu003_Fsc: Fab-scFv-Fc antibody consisting of the half-molecule of anti-CD39 antibody mAnE67 (Knob type) and the half-molecule of anti-TIM-3 scFv antibody (humanized) Hu003 (Hole type). The monospecific antibodies described in (8-1) were used as controls.

The affinity of the monoclonal antibodies and the anti-human TIM-3/CD39 bispecific antibodies of the present invention for the recombinant antigen was measured by the method described in (8-1).

The results are shown in Tables 6-3 and 6-4. The anti-human TIM-3/CD39 bispecific antibodies of the present invention were exhibited to bind to the recombinant human TIM-3 and the recombinant human CD39 with the following dissociation constants (KD values).

**[Table 6-3]**

| Affinity for rhTIM-3 | | | | |
|---|---|---|---|---|
| Bispecific antibodies | | | | |
| Clone | Format | ka (1/Ms) | kd (1/s) | KD (M) |
| 6-3hIG_Hu003_Fsc | Fab-scFv-Fc | 3.20E+05 | 1.72E-03 | 5.37E-09 |
| HuP4_IB_Hu003_Fsc | Fab-scFv-Fc | 2.97E+05 | 1.34E-03 | 4.49E-09 |
| Hu003_HuP4_IB_Fsc | Fab-scFv-Fc | 3.10E+05 | 1.25E-03 | 4.03E-09 |
| T86_Hu003_Fsc | Fab-scFv-Fc | 3.04E+05 | 1.37E-03 | 4.52E-09 |
| I67_Hu003_Fsc | Fab-scFv-Fc | 2.78E+05 | 1.78E-03 | 6.40E-09 |
| Tre291_Hu003_Fsc | Fab-scFv-Fc | 3.29E+05 | 1.29E-03 | 3.93E-09 |
| mAnE67_Hu003_Fsc | Fab-scFv-Fc | 2.72E+05 | 1.68E-03 | 6.18E-09 |

**[Table 6-4]**

| Affinity for rhCD39 | | | | |
|---|---|---|---|---|
| Bispecific antibodies | | | | |
| Clone | Format | ka (1/Ms) | kd (1/s) | KD (M) |
| 6-3hIG_Hu003_Fsc | Fab-scFv-Fc | 6.22E+05 | 1.31E-04 | 2.10E-10 |
| HuP4_IB_Hu003_Fsc | Fab-scFv-Fc | 7.76E+05 | 3.99E-04 | 5.15E-10 |
| Hu003_HuP4_IB_Fsc | Fab-scFv-Fc | 6.99E+05 | 4.39E-04 | 6.28E-10 |
| T86_Hu003_Fsc | Fab-scFv-Fc | 2.73E+05 | 6.55E-04 | 2.40E-09 |
| I67_Hu003_Fsc | Fab-scFv-Fc | 2.43E+06 | 1.93E-03 | 7.94E-10 |
| Tre291_Hu003_Fsc | Fab-scFv-Fc | 2.19E+06 | 2.98E-03 | 1.36E-09 |
| mAnE67_Hu003_Fsc | Fab-scFv-Fc | 4.79E+05 | 1.29E-03 | 2.70E-09 |

### Example 9: Evaluation of Simultaneous Binding of Bispecific Antibody to Antigens

In this example, simultaneous binding of anti-human TIM-3/CD39 bispecific antibodies to both targets (human TIM-3 and human CD39) was evaluated by interaction analysis of surface plasmon resonance (SPR) using Biacore8K.

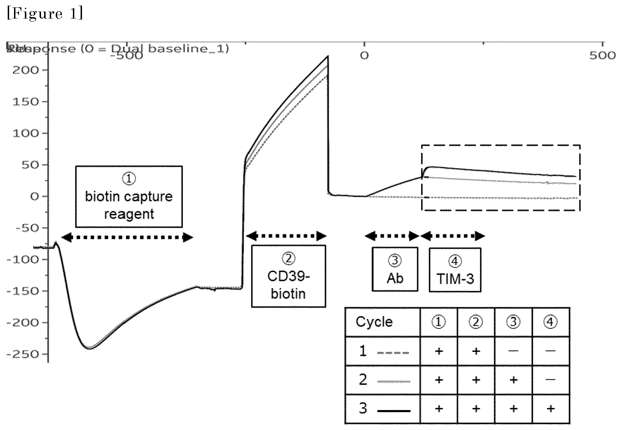

The bispecific antibodies tested in this example are identical to the bispecific antibodies used in Example 8. The procedure of the evaluation is shown in Figure 1.

The Biotin capture reagent was immobilized to Sensor Chip CAP (Cytiva), and biotinylated recombinant human CD39 (in-house product, 38 to 478 aa of Accession No. NM_001776.6) was bound as a primary antigen.

Next, the bispecific antibody was bound, and finally, recombinant human TIM-3 (rhTim-3-His: SinoBological, 10390-H08H) was bound as a secondary antigen. The binding of the secondary antigen was evaluated by subtracting a sensor gram of a reference to which recombinant human CD39 was not added.

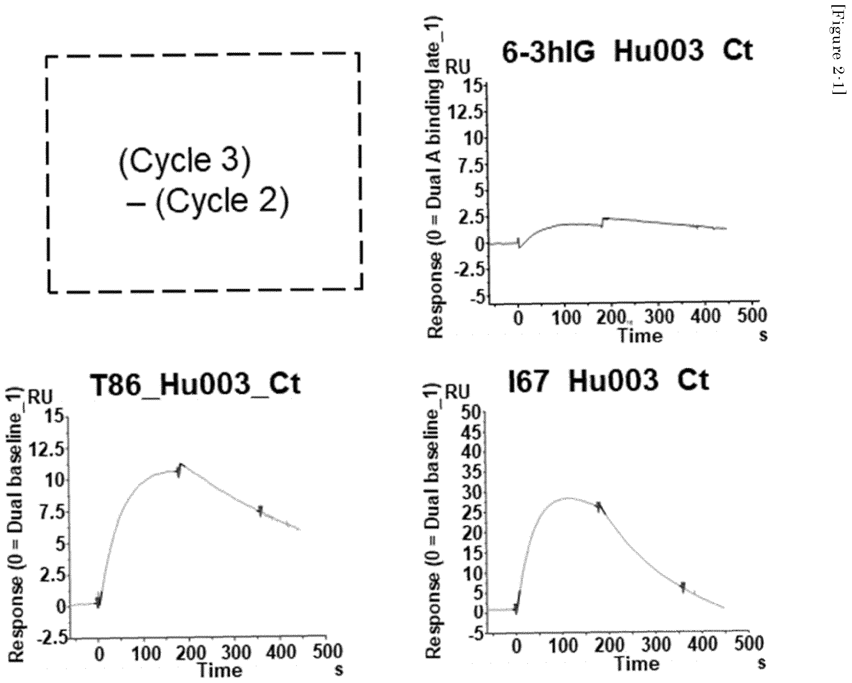

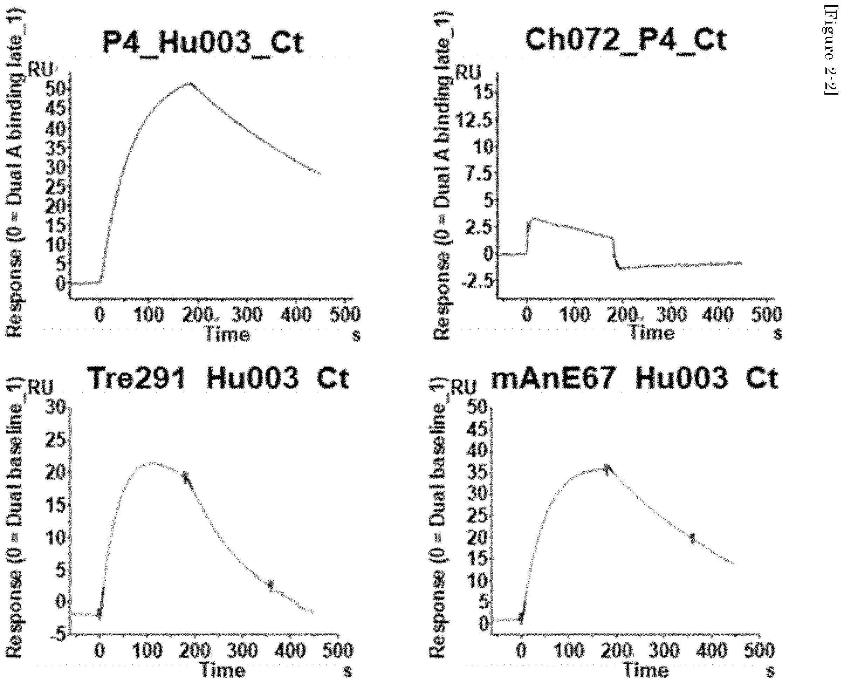

The results of analysis using Biacore8K are shown in Figure 2. As shown in the sensor grams of a difference obtained by subtracting the response (RU) in Cycle 2 from the response (RU) in Cycle 3 shown on the lower panel, a positive response was observed when recombinant human TIM-3 as an analyte passed along the surface of the sensor chip against the bispecific antibody bound to the biotinylated recombinant human CD39 captured on the sensor chip, and therefore, the anti-human TIM-3/CD39 bispecific antibodies of the present invention were exhibited to bind simultaneously to human TIM-3 and human CD39.

### Example 10: Binding Analysis to Antigen Expressed on Cell Surfaces

(10-1) In this example, the binding of the obtained anti-human TIM-3/CD39 bispecific antibodies of the present invention of (7-1) to an antigen expressed on Jurkat cell surfaces was analyzed by flow cytometry.

In this analysis, Jurkat cells stably expressing human TIM-3, Jurkat cells stably expressing human CD39, or Jurkat cells stably co-expressing human TIM-3 and human CD39 were used (Accession No. JX049979.1 for human TIM-3, Accession No. NM_001776.6 for human CD39).

The binding analysis was performed as follows. The above Jurkat cells stably expressing the respective antigens were aliquoted into 96-well V-bottom plates at 2 × 10⁵ cells/well, and washed once with 200 microliters of staining buffer [PBS(-), 1% BSA, 0.09% NaN₃]. Next, the dilution series of each antibody and the isotype control antibody were prepared with staining buffer, and added to each cell at 100 microliters/well, and the cells were left to stand at 4°C for 30 minutes.

Thereafter, the cells were washed once with staining buffer, a secondary antibody (PE-Goat Anti-human IgG/Jackson ImmunoResearch Laboratories, 109-116-170) diluted 100-fold with staining buffer was added to the cells at 100 microliters/well, the cells were left to stand at 4°C for 20 minutes, and then washed twice with staining buffer, to which 50 microliterss of a fixation buffer (Biolegend, 420801) was added, and were left to stand at room temperature for 20 minutes in order to fix the cells. The stained cells were suspended in staining buffer and analyzed by flow cytometer. From the results, MFI corresponding to the antibody concentration was plotted and the KD value was calculated.

The calculated dissociation constant KD (nM) valued of the antibodies are shown in Table 7-1. The anti-human TIM-3/CD39 bispecific antibodies of the present invention were shown to bind to human TIM-3 or human CD39 expressed on cell membranes.

**[Table 7-1]**

| Bispecific antibodies | | | | | | |
|---|---|---|---|---|---|---|
| Antibody | format | Jurkat/CD39 | | Jurkat/TIM-3 | | Jurkat/ TIM-3+CD39 |
| | | KD (nM) | | KD (nM) | | KD (nM) |
| HuP4_41_Hu003_Ct | scFv-Fc | 15.41 | | 2.523 | | 11.05 |
| T86-Hu003_Ct | scFv-Fc | 18.83 | | 2.768 | | 10.38 |
| 167-Hu003_Ct | scFv-Fc | 14.05 | | 1.857 | | 1.848 |
| Tre291-Hu003_Ct | scFv-Fc | 22.62 | | 2.133 | | 4.737 |
| mAnE67-Hu003_Ct | scFv-Fc | 2.695 | | 1.489 | | 1.693 |
| 6-3hIG-Hu003_Ct | scFv-Fc | 237 | | 1.919 | | 12.12 |
| Hu072_11_HuP4_41_Ct | scFv-Fc | 9.455 | | 44.51 | | 6.287 |

| Monospecific antibodies | | | | | | |
|---|---|---|---|---|---|---|
| Antibody | format | Jurkat/CD39 | Jurkat/TIM-3 | | Jurkat/ TIM-3+CD39 | |
| | | KD (nM) | KD (nM) | | KD (nM) | |
| Hu003_13_F20_sc | scFv-Fc | | 0.2738 | | 0.3394 | |
| Hu072_11_sc | scFv-Fc | | 26.82 | | 31.24 | |
| HuP4_41_sc | scFv-Fc | 1.883 | | | 2.079 | |
| T86_sc | scFv-Fc | 2.183 | | | 2.252 | |
| I67_sc | scFv-Fc | 0.3293 | | | 0.2078 | |
| Tre291_sc | scFv-Fc | 0.7999 | | | 0.9221 | |
| mAnE67_sc | scFv-Fc | 0.3343 | | | 0.3425 | |
| 6-3hIG_sc | scFv-Fc | 42.15 | | | 33.65 | |

(10-2) In this example, the binding of the anti-human TIM-3/CD39 bispecific antibodies of the present invention to the antigens expressed on 293T cell surfaces was analyzed by flow cytometry.

In this analysis, 293T cells stably expressing human TIM-3, 293T cells stably expressing human CD39, or 293T cells stably co-expressing human TIM-3 and human CD39 were used (Accession No. JX049979.1 for human TIM-3, Accession No. NM_001776.6 for human CD39).

The binding analysis was performed in the manner described in (10-1). In other words, in accordance with the procedure shown in (10-1), the 293T cells stably expressing the respective antigens were seeded in 96-well V-bottom plates, and stained with the dilution series of each antibody and the isotype control antibody, and the stained cells were analyzed by a flow cytometer. From the results, MFI corresponding to the antibody concentration was plotted and the KD value was calculated.

The calculated dissociation constant KD (nM) valued of the antibodies are shown in Tables 7-2 and 7-3. The anti-human TIM-3/CD39 bispecific antibodies of the present invention were shown to bind to human TIM-3 or human CD39 expressed on cell membranes.

**[Table 7-2]**

| Bispecific antibodies | | | | |
|---|---|---|---|---|
| Antibody | format | 293T/CD39 | 293T/TIM-3 | 293T/ TIM-3+CD39 |
| | | KD (nM) | KD (nM) | KD (nM) |
| 6-3hIG_Hu003_Fsc | Fab-scFv-Fc | 17.56 | 5.670 | 8.310 |
| HuP4_IB_Hu003_Fsc | Fab-scFv-Fc | 20.37 | 10.82 | 5.560 |
| Hu003_HuP4_IB_Fsc | Fab-scFv-Fc | 10.47 | 5.814 | 8.432 |
| T86_Hu003_Fsc | Fab-scFv-Fc | 9.191 | 4.790 | 21.51 |
| I67_Hu003_Fsc | Fab-scFv-Fc | 16.63 | 4.784 | 18.35 |
| Tre291_Hu003_Fsc | Fab-scFv-Fc | 25.38 | 4.156 | 16.86 |
| mAnE67_Hu003_Fsc | Fab-scFv-Fc | 3.945 | 6.003 | 10.65 |
| HuP4_IB_Hu003 | scFv-Fc | 11.42 | 8.66 | 8.388 |

**[Table 7-3]**

| Monospecific antibodies | | | | |
|---|---|---|---|---|
| Antibody | format | 293T/CD39 | 293T/TIM-3 | 293T/ TIM-3+CD39 |
| | | KD (nM) | KD (nM) | KD (nM) |
| Hu003_13_F20_sc | scFv-Fc | | 2.720 | 3.857 |
| Hu003_13_F20_LA | IgG1LALA | | 2.164 | 3.282 |
| 6-3hIG_sc | scFv-Fc | 18.55 | | 26.61 |
| 6-3hIG_LA | IgG1LALA | 7040 | | 16.84 |
| HuP4_IB_sc | scFv-Fc | 2.762 | | 4.747 |
| HuP4_IB_LA | IgG1LALA | 2.795 | | 3.475 |
| T86_sc | scFv-Fc | 3.563 | | 16.92 |
| T86_LA | IgG1LALA | 2.665 | | 5.000 |
| I67_sc | scFv-Fc | 2.368 | | 6.804 |
| I67_LA | IgG1LALA | 1.732 | | 4.191 |
| Tre291_sc | scFv-Fc | 2.431 | | 7.153 |
| Tre291_LA | IgG1LALA | 2.442 | | 4.791 |
| mAnE67_sc | scFv-Fc | 2.194 | | 5.503 |
| mAnE67_LA | IgG1LALA | 1.804 | | 3.658 |

### Example 11: Analysis of Binding Selectivity to Cells Co-Expressing Human TIM-3 and Human CD39

In this example, the ability of the anti-human TIM-3/CD39 bispecific antibodies of the present invention to bind selectively to cells expressing each of two antigens: human TIM-3 and human CD39 singly or expressing two antigens was analyzed by a flow cytometry method.

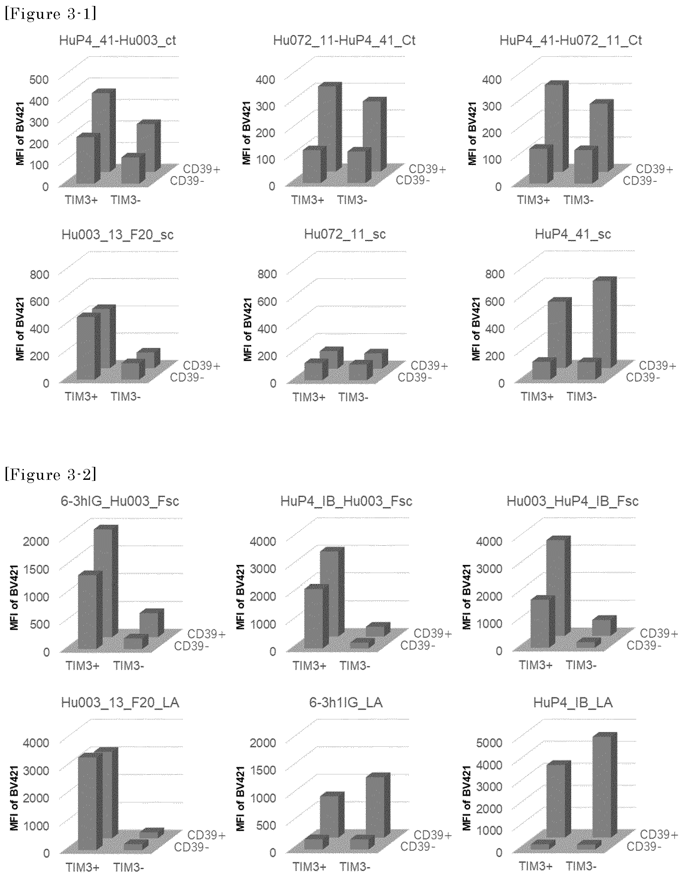

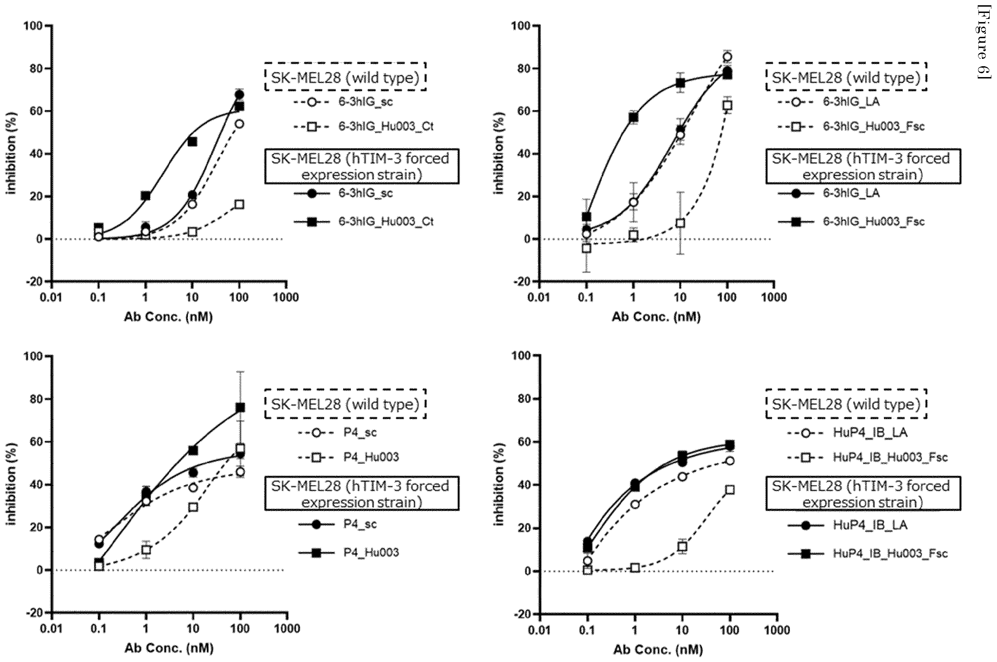

The antibodies that were used in this analysis are HuP4_41_Hu003_Ct, Hu072_11_HuP4_41_Ct and HuP4_41_Hu072_11_Ct as bispecific antibodies and Hu003_13_F20_sc, Hu072_11_sc and HuP4_41_sc as monospecific antibodies in the case of Figure 3-1, and 6-3hIG_Hu003_Fsc, HuP4_IB_Hu003_Fsc and Hu003_HuP4_IB_Fsc as bispecific antibodies and Hu003_13_F20_LA, 6-3hIG_LA and HuP4_IB_LA as monospecific antibodies in the case of Figure 3-2.

Jurkat cells, Jurkat cells stably expressing human TIM-3, Jurkat cells stably expressing human CD39, and Jurkat cells stably co-expressing human TIM-3 and human CD39 were used (Accession No. JX049979.1 for human TIM-3, Accession No. NM_001776.6 for human CD39).

The binding selectivity analysis was performed as follows. The four types of cells described above were aliquoted into the identical wells of 96-well V-bottom plates at 0.5 × 10⁵ cells/well, and washed once with 200 microliters of staining buffer [PBS(-), 1% BSA, 0.09% NaN₃]. Next, the antibodies and the isotype control antibody were prepared with staining buffer, and added to the cells at 0.1 nM per well (Figure 3-1) or at 1 nM (Figure 3-2), and the cells were left to stand at 4°C for 30 minutes.

Thereafter, after washing the cells once with staining buffer, secondary antibodies (Biolegend, BV421-anti-hIgG: M1310G05), and surface marker antibodies (Biolegend, FITC-anti-CD39: A1, or Alexa647-anti-TIM-3 of an in-house product) which do not compete with subject antibodies of this example (anti-human TIM-3/CD39 bispecific antibodies of the present invention) were then appropriately diluted with staining buffer, and added to the cells at 100 microliters/well, which were left to stand at 4°C for 20 minutes. Then the cells were washed twice with staining buffer, to which 50 microliters of a fixation buffer (Biolegend, 420801) was added, and the cells were left to stand at room temperature for 20 minutes to fix.

The stained cells were suspended in staining buffer and analyzed by a flow cytometer. The results were developed based on a level of FITC staining and a level of Alexa647 staining, and then divided into four quadrants according to the level of staining, which include "FITC-negative" and "Alexa647-negative" quadrants for Jurkat cells, "FITC-negative" and "Alexa647-positive" quadrants for Jurkat cells stably expressing human TIM-3, "FITC-positive" and "Alexa647-negative" quadrants for cells stably expressing human CD39, and "FITC-positive" and "Alexa647-positive" quadrants for Jurkat cells stably co-expressing human TIM-3 and human CD39, and the average amount of binding of the anti-human TIM-3/CD39 bispecific antibody to each cell group was calculated as MFI of BV421.

The results of plotting the calculated MFI of BV421 for the cells stained with the anti-human TIM-3/CD39 bispecific antibodies, and the control antibodies, i.e., the anti-human TIM-3 monoclonal antibodies or anti-human CD39 monoclonal antibodies, are shown in Figures 3-1 and 3-2 (the results for the bispecific antibodies are shown on the upper side, and the results for the monospecific antibodies are shown on the lower side).

These results showed that the anti-human TIM-3/CD39 bispecific antibodies of the present invention bound more selectively to Jurkat cells co-expressing human TIM-3 and human CD39 antigens than to cells expressing only human TIM-3 or cells expressing only human CD39.

### Example 12: Inhibition of Binding to Galectin-9

In this example, the ability of the anti-human TIM-3/CD39 bispecific antibodies of the present invention to inhibit binding between human TIM-3 and human Galectin-9 was analyzed by ELISA.

Human Galectin-9 (R&D Systems, 2045-GA) was adjusted to 2.5 micrograms/mL in a 50 mM carbonate buffer, pH 9.4, added at 50 microliters/well to 96 well ELISA plates (Corning, 9018), and left to stand at 4°C overnight. Then, the plates were washed with washing buffer [PBS (-), 0.1% Tween 20] three times and blocked for 2 hours at room temperature (Nacalai Tesque, Blocking One:0395395).

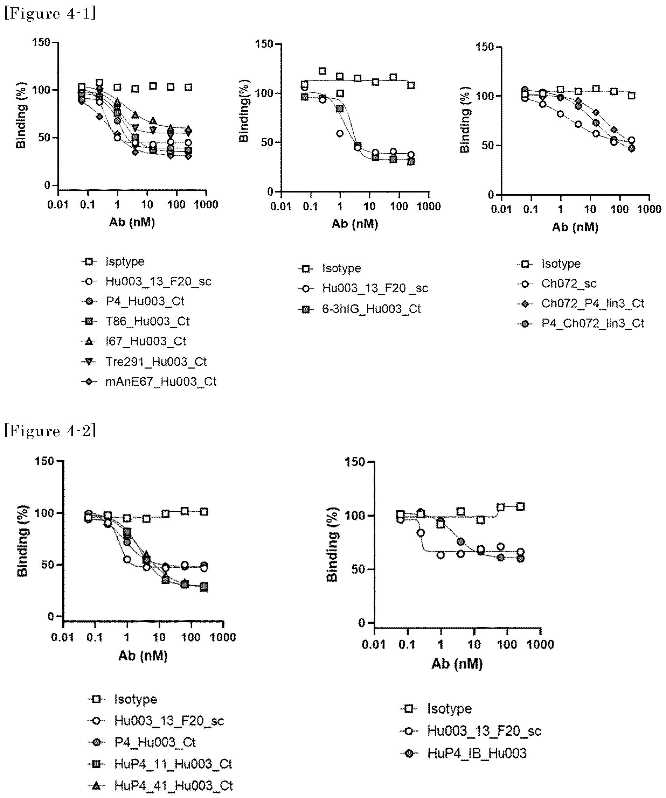

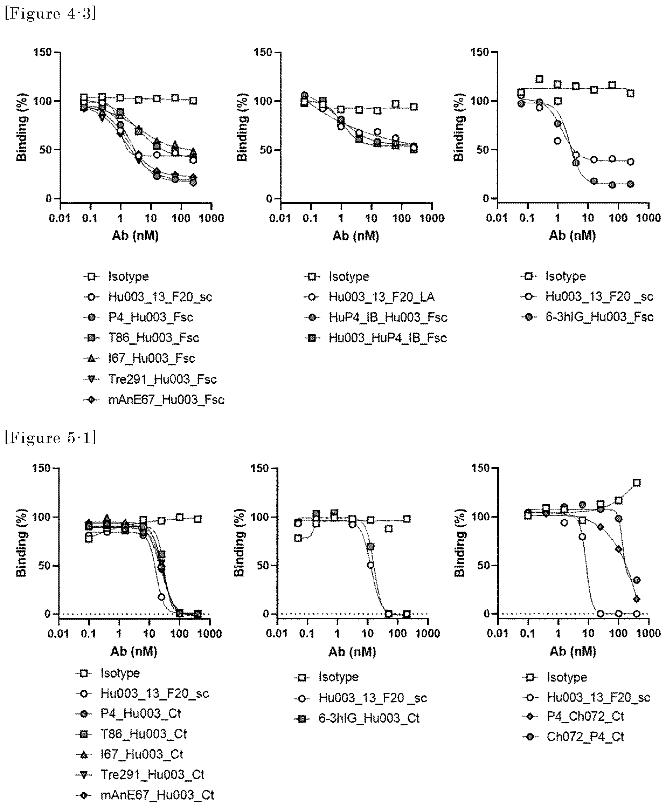

A 7-step of 4-fold dilution series of each of the antibodies were prepared in binding buffer [PBS (-), 0.05% Tween 20, 1/5 vol. Blocking One] and were mixed 1:1 with 200 ng/mL of biotinylated human TIM-3-Fc antigen (in-house product, SEQ ID No: 118), which were left to stand at room temperature for 1 hour. The antibodies used are indicated in Figures 4-1, 4-2 and 4-3.

The antigen/antibody mixture was added to the plate after blocking at 50 microliters/well and left to stand for 1 hour at room temperature. Then, the plates were washed three times with washing buffer, to which Streptavidin-HRP (Abcam, ab7403) diluted 15,000-fold in binding buffer was added at 50 microliters/well and left to stand for 30 minutes at room temperature. The plates were then washed three times with washing buffer, and TMB+ (Dako, S1599) was added to the plates at 50 microliters/well and left to stand for 10 minutes at room temperature. Finally, 1 N sulfuric acid was added to the plates at 50 microliters/well to stop color development reaction, and absorbance at 450 nm was measured.

The results of binding (%) of biotinylated human TIM-3-Fc antigen reacted with the antibodies to human Galectin-9 are shown in Figures 4-1, 4-2 and 4-3.

Figure 4-1 shows, in the left chart, the results for the bispecific antibodies of P4_Hu003_Ct, T86_Hu003_Ct, I67_Hu003_Ct, Tre291_Hu003_Ct and mAnE67_Hu003_Ct compared with Hu003_13_F20_sc (anti-TIM-3 antibody) as a control; in the middle chart, the results for the bispecific antibody of 6-3hIG_Hu003_Ct compared with Hu003_13_F20_sc (anti-TIM-3 antibody) as a control; and, in the right chart, the results for the bispecific antibodies of Ch072_P4_Ct and P4_Ch072_Ct compared with Ch072_sc (anti-TIM-3 antibody) as a control.

Figure 4-2 shows, in the left chart, the results for the bispecific antibodies of P4_Hu003_Ct, HuP4_11_Hu003_Ct and HuP4_41_Hu003_Ct compared with Hu003_13_F20_sc (anti-TIM-3 antibody) as a control; and, in the right chart, the results for the bispecific antibody of HuP4_IB_Hu003 compared with Hu003_13_F20_sc (anti-TIM-3 antibody) as a control.

Figure 4-3 shows, in the left chart, the results for the bispecific antibodies of P4_Hu003_Fsc, T86_Hu003_Fsc, I67_Hu003_Fsc, Tre291_Hu003_Fsc and mAnE67_Hu003_Fsc compared with Hu003_13_F20_sc (anti-TIM-3 antibody) as a control; in the middle chart, the results for the bispecific antibodies of HuP4_IB_Hu003_Fsc and Hu003_HuP4_IB_Fsc compared with Hu003_13_F20_LA (anti-TIM-3 antibody) as a control; and, in the right chart, the results for the bispecific antibody of 6-3hIG_Hu003_Fsc compared with Hu003_13_F20_sc (anti-TIM-3 antibody) as a control.

The anti-human TIM-3/CD39 bispecific antibodies produced according to the present invention were exhibited to inhibit binding between human TIM-3 and human Galectin-9, in the similar level to co-analyzed controls which are anti-human TIM-3 monoclonal antibodies.

### Example 13: Inhibition of Binding to Phosphatidylserine

In this example, the ability of the anti-human TIM-3/CD39 bispecific antibodies of the present invention to inhibit binding between human TIM-3 and Phosphatidylserine was analyzed by flow cytometry.

Jurkat cells were adjusted to 5 × 10⁵ cells/mL in RPMI-1640 medium supplemented with 10% FCS, 100 units/mL penicillin, and 100 micrograms/mL streptomycin, and were reacted with 100 ng/ mL of Anti-Fas (CD95) mAb (MBL, SY-001) in a 37°C incubator for 3 hours to induce apoptosis.

During apoptosis induction, a 7-step dilution series of each of the antibodies were prepared in FACS buffer [PBS (-), 0.5% BSA] and were mixed 1:1 with 8 micrograms/mL of biotinylated human TIM-3-Fc antigen (in-house product, SEQ ID NO: 118), which were left to stand for 1 hour at room temperature.

The cells after induction of apoptosis were aliquoted into 96 well V-bottom plates, washed with PBS (-), to which 50 microliters of Zombie Violet^{™} Dye diluted 1000-fold in PBS (-) was added, which were left to stand for 15 minutes at room temperature. After washing once the plates with PBS (-),50 microliters/well of Human TruStain FcX diluted 20-fold with Annexin V binding buffer was added to the plates, which was left to stand at room temperature for 10 minutes.

Next, 50 microliters/well of the antigen/antibody mixture was added to the plates and left to stand at room temperature for 30 minutes. Then, the plates were washed once with Annexin V binding buffer, to which 50 microliters of PE-Streptavidin (Biolegend, 405204) which were adjusted to 0.3 microliters/1 × 10⁶ cells using Annexin V binding buffer (Biolegend) was added and left to stand for 15 minutes at room temperature.

Then, the plates were washed once with Annexin V binding buffer, to which 100 microliters of APC-Annexin V (Biolegend, 640920) which was adjusted with Annexin V binding buffer (Biolegend) to 2 microliters/1 × 10⁶ cells was added and left to stand for 15 minutes at 4°C. Finally, 200 microliters of Annexin V binding buffer was added to the plates and analyzed by flow cytometer.

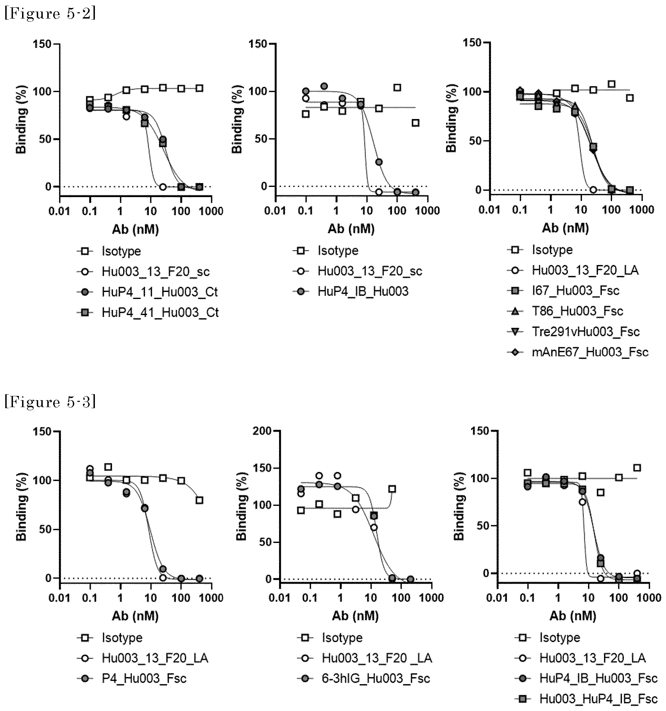

The results of flow cytometry analysis are shown in Figures 5-1, 5-2 and 5-3, and the IC50 values (nM) for each antibody, calculated based on the results, are shown in Table 8.

Figure 5-1 shows, in the left chart, the results for the bispecific antibodies of P4_Hu003_Ct, T86_Hu003_Ct, I67_Hu003_Ct, Tre291_Hu003_Ct and mAnE67_Hu003_Ct compared with Hu003_13_F20_sc (anti-TIM-3 antibody) as a control; in the middle chart, the results for the bispecific antibody of 6-3hIG_Hu003_Ct compared with Hu003_13_F20_sc (anti-TIM-3 antibody) as a control; and, in the right chart, the results for the bispecific antibodies of P4_Ch072_Ct and Ch072_P4_Ct compared with Hu003_13_F20_sc (anti-TIM-3 antibody) as a control.

Figure 5-2 shows, in the left chart, the results for the bispecific antibodies of HuP4_11_Hu003_Ct and HuP4_41_Hu003_Ct compared with Hu003_13_F20_sc (anti-TIM-3 antibody) as a control; in the middle chart, the results for the bispecific antibodies of HuP4_IB_Hu003 compared with Hu003_13_F20_sc (anti-TIM-3 antibody) as a control; and, in the right chart, the results for the bispecific antibodies of T86_Hu003_Fsc, I67_Hu003_Fsc, Tre291_Hu003_Fsc and mAnE67_Hu003_Fsc compared with Hu003_13_F20_LA (anti-TIM-3 antibody) as a control.

Figure 5-3 shows, in the left chart, the results for the bispecific antibody of P4_Hu003_Fsc compared with Hu003_13_F20_LA (anti-TIM-3 antibody) as a control; in the middle chart, the results for the bispecific antibody of 6-3hIG_Hu003_Fsc compared with Hu003_13_F20_LA (anti-TIM-3 antibody) as a control; and, in the right chart, the results for the bispecific antibodies of HuP4_IB_Hu003_Fsc and Hu003_HuP4_IB_Fsc compared with Hu003_13_F20_LA (anti-TIM-3 antibody) as a control.

The anti-human TIM-3/CD39 bispecific antibodies produced according to the present invention were exhibited to completely inhibit binding between human TIM-3 and Phosphatidylserine in the similar level to the co-analyzed controls which are anti-human TIM-3 monoclonal antibodies. The IC50 values (nM) for the binding inhibition of the antibodies to human TIM-3 are shown in Tables 8-1 to 8-3 (the antibodies shown in Tables 8-1 to 8-3 correspond to the antibodies shown in Figures 5-1 to 5-3).

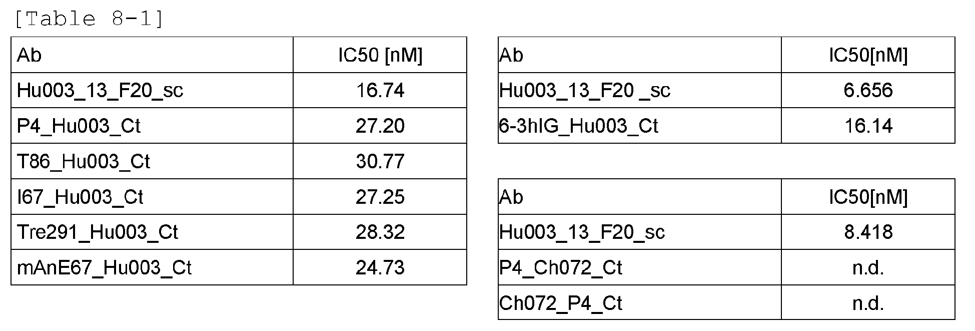

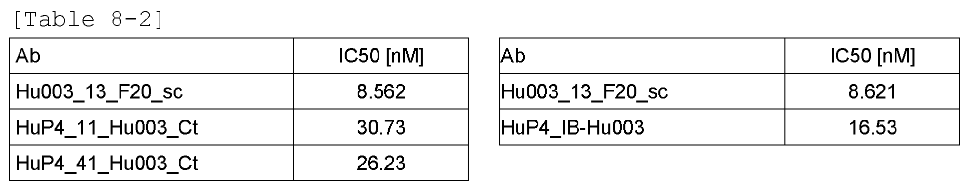

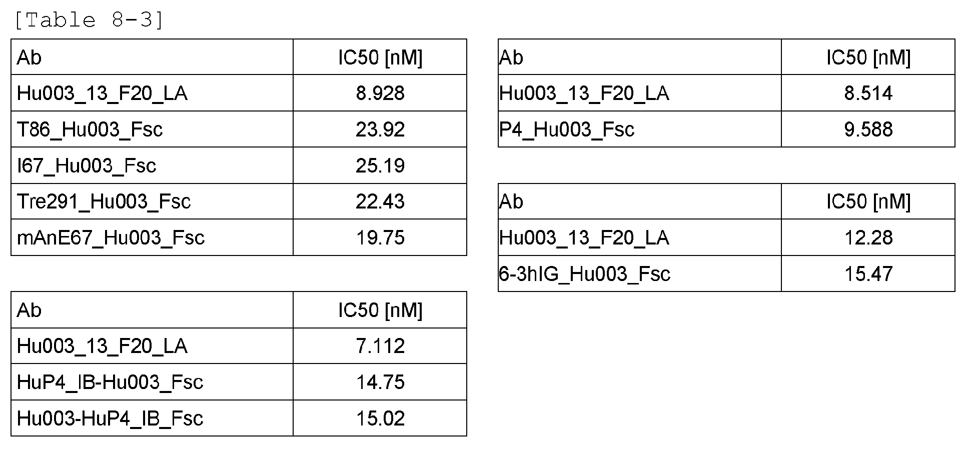

### Example 14: Evaluation of Ability to Inhibit ATP Degrading Activity of CD39

In this example, the ability of the anti-human TIM-3/CD39 bispecific antibodies of the present invention to inhibit human CD39 activity was evaluated.

The antibodies that were used are the bispecific antibodies of 6-3hIG_Hu003_Ct and 6-3hIG_Hu003_Fsc compared with the monospecific antibodies of 6-3hIG_sc and 6-3hIG_LA as their respective comparative controls (upper side in Figure 6), and the bispecific antibodies of P4_Hu003_Ct and HuP4_IB_Hu003_Fsc compared with the monospecific antibodies of P4_sc and HuP4_IB_LA as their respective controls (lower side in Figure 6). For evaluation, wild-type SK-MEL28 cells expressing human CD39 (Accession No. NM_001776.6), or SK-MEL28 cells stably expressing human TIM-3 (Accession No. JX049979.1) (TIM-3/SK-MEL28) were used.

Wild-type SK-MEL28 cells (for human CD39) and TIM-3/SK-MEL28 cells (for TIM-3) were adjusted to 5 × 10³ cells/well using E-MEM medium, and seeded on 96-well V-bottom plates at 50 microliters per well. A 4 step of 10-fold dilution series from 100 nM of each antibody were added at 50 microliters per well to 3 wells for each antibody sample, and left to stand in an incubator at 37°C for 1 hour. Next, 5 microliters of ATP (SIGMA, A2383) adjusted at 200 nM in E-MEM medium was added, and the cells were left to stand at room temperature for 30 minutes. Thereafter, the plate was centrifuged at 700 g for 3 minutes, and 50 microliters of the supernatant was collected in a 96-well polystyrene (white) plate.

To the culture supernatant was added 50 microliters of CellTiter-Glo (registered trademark) 2.0 (Promega, G9242) for ATP quantification, the mixture was left to stand at room temperature for 10 minutes, and the amount of ATP was measured by luminescence detection with a microplate reader.

Considering that the level of residual ATP in wells where the cells were not seeded was 100% and the level of residual ATP in wells where the cells were seeded but the antibody was not added was 0%, the ratio of inhibition of ATP degradation by each antibody was calculated from the percentage level of residual ATP in the measurement sample.

The results of inhibition of CD39 activity depending on the antibody concentration of each antibody are shown in Figure 6. The anti-human TIM-3/CD39 bispecific antibodies were exhibited to strongly inhibit the enzymatic activity of CD39 against TIM-3/SK-MEL28. This inhibitory action of enzymatic activity is shown to more highly inhibit enzymatic activity against cells co-expressing human CD39 and human TIM-3 simultaneously because stronger inhibition was exhibited against TIM-3/SK-MEL28 than wild-type SK-MEL28 that does not express human TIM-3.

On the other hand, the anti-human CD39 monoclonal antibodies used as comparative controls exhibited a similar inhibition property against TIM-3/SK-MEL28.

### Example 15: Assay for PBMCs Stimulation with SEB

In this example, effects of the anti-human TIM-3/CD39 bispecific antibodies of the present invention on activation of human peripheral blood T cell were evaluated by examining the activation of PBMCs with SEB (staphylococcal enterotoxin B).

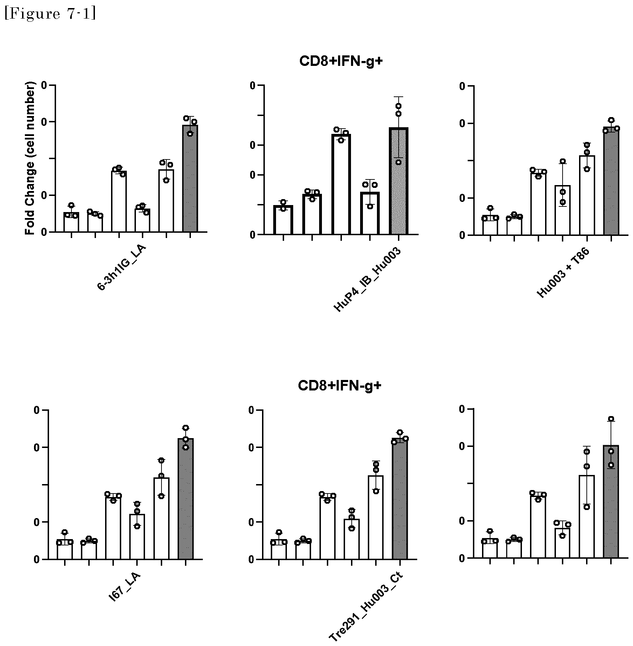

The antibodies that were used in this example are 6-3hIG_Hu003_Ct, HuP4_IB_Hu003, T86_Hu003_Ct, I67_Hu003_Ct, Tre291_Hu003_Ct and mAnE67_Hu003_Ct as bispecific antibodies derived from (7-1), compared to one or two antibodies from the following 7 types of monospecific antibodies shown in the charts of Figure 7-1 as comparative controls for the respective bispecific antibodies.
Hu003_13_F20_LA: IgG1-LALA antibody of anti-TIM-3 antibody (humanized) Hu003_13_F20
6-3hIG_LA: IgG1-LALA antibody of anti-CD39 antibody (humanized) 6-3hIG
HuP4_IB_sc: scFv_Fc-LALA antibody of anti-CD39 antibody (humanized) HuP4_IB
T86_LA: IgG1-LALA antibody of anti-CD39 antibody T86
I67_LA: IgG1-LALA antibody of anti-CD39 antibody I67
Tre_291_LA: IgG1-LALA antibody of anti-CD39 antibody Tre_291 mAnE67_LA: IgG1-LALA antibody of anti-CD39 antibody mAnE67.

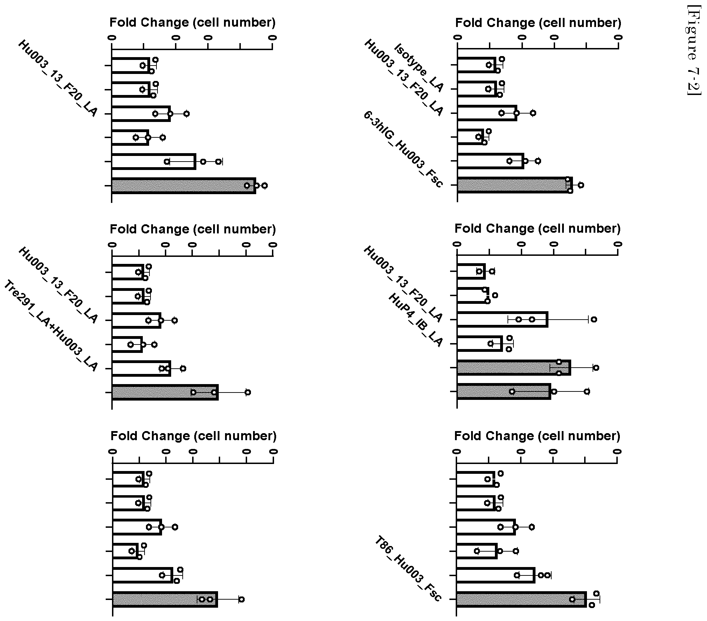

The antibodies that were used in this example are 6-3hIG_Hu003_Fsc, HuP4_IB_Hu003_Fsc or Hu003_HuP4_IB_Fsc, T86_Hu003_Fsc, I67_Hu003_Fsc, Tre291_Hu003_Fsc and mAnE67_Hu003_Fsc as bispecific antibodies derived from (7-2), and one or two antibodies from the following 7 types of monospecific antibodies shown in the charts of Figure 7-2 were used as comparative controls for the respective bispecific antibodies.
Hu003_13_F20_LA: IgG1-LALA antibody of anti-TIM-3 antibody (humanized) Hu003_13_F20
6-3hIG_LA: IgG1-LALA antibody of anti-CD39 antibody (humanized) 6-3hIG
HuP4_IB_LA: IgG1-LALA antibody of anti-CD39 antibody (humanized) HuP4_IB
T86_LA: IgG1-LALA antibody of anti-CD39 antibody T86
I67_LA: IgG1-LALA antibody of anti-CD39 antibody I67
Tre_291_LA: IgG1-LALA antibody of anti-CD39 antibody Tre_291 mAnE67_LA: IgG1-LALA antibody of anti-CD39 antibody mAnE67.

Peripheral blood monocytes (PBMCs) collected from blood of healthy human volunteer were suspended in RPMI-1640 medium supplemented with 10% FCS, 100 units/mL penicillin, 100 micrograms/mL streptomycin, and 1xGlutaMax (Gibco, 35050-061), which were seeded in 96-well U-bottom plates at a density of 0.7-0.8×10⁵ cells/well.

The antibodies were added to be a final concentration of 10 micrograms/mL and were reacted with PBMCs for 30 minutes at 37°C under 5% CO₂. Thereafter, SEB (staphylococcal enterotoxin B, Toxin Technology Inc., BT202RED) was added to be a final concentration of 1 ng/mL, and the cells were cultured for 10 days at 37°C under 5% CO₂.

On the day of staining, Brefeldin A (Biolegend) was added at a final concentration of 1x and SEB was added at a final concentration of 10 ng/mL, to the cultured PBMCs, which were cultured for 8 hours at 37°C under 5% CO₂.

The cells were collected in a 96-well V-bottom plate and washed once with 200 microliters of PBS (-). To stain dead cells, 50 microliters of Zombie-NIR (Biolegend) diluted 500-1000 times in PBS (-) was added to the cell culture, which were stained for 15 minutes at room temperature. Thereafter, 50 microliters of Human TruStain FcX (Biolegend), diluted 20 times in PBS (-), was added to the plates which incubated for 20 minutes at room temperature.

Then the biotinylated anti-human TIM-3 antibody (in-house product) which are not compete with the bispecific antibodies or monospecific antibodies to be used as subject antibodies were diluted to 1 microgram/mL in staining buffer [PBS (-), 1% BSA, 0.09% NaN₃] and 50 microliters of the aliquot was added to the cells, which were left to stand at 4°C for 1 hour. After washing once the cells with staining buffer, 100 microliters of each of T cell marker staining antibodies (Biolegend, anti-CD3: UCHT1, anti-CD4: RPA-T4, anti-CD8a: HIT8a) and T cell exhaustion marker staining antibodies (Biolegend, anti-CD39: A1, anti-PD-1: EH12) that were appropriately diluted in staining buffer was added to the plates. The cells were left to stand in the dark at 4°C for 30 minutes, were washed once with the staining buffer, to which 50 microliters of the fixation buffer was added, which was left to stand at room temperature for 20 minutes to fix the cells. After washing twice with Intracellular Staining Permeabilization Wash Buffer (Biolegend, 421002), 100 microliters of IFN-gamma staining antibody (Biolegend, anti-IFN-gamma: B27), diluted appropriately in Intracellular Staining Permeabilization Wash Buffer, was added to the cells, then left to stand in the dark at 4°C for 40 minutes. After washing twice with Intracellular Staining Permeabilization Wash Buffer, the cells were suspended in washing buffer to which counting beads (ThermoFisher Scientific, C36950) were added for cell counting, which were analyzed by flow cytometry.

The results of flow cytometry analysis, which reveal changes in the number of CD8-positive cells and that of IFNgamma-positive cells, are shown in Figures 7-1 and 7-2. The anti-human TIM-3/CD39 bispecific antibodies produced according to the present invention more significantly increased the number of CD8-positive IFNgamma-positive cells as compared to the result from the co-evaluated control condition when cultured with the addition of the anti-human TIM-3 monoclonal antibody, with the addition of the anti-human CD39 monoclonal antibody, or with the addition of both the anti-human TIM-3 monoclonal antibody and the anti-human CD39 monoclonal antibody. This example showed that the anti-human TIM-3/CD39 bispecific antibodies exhibited an effect higher than that of inhibition of human TIM-3 and human CD39 by separate antibodies.

### Example 16: Evaluation of Effects on Maturation/Activation of Monocyte-Derived Dendritic Cells (MDDCs)

In this example, the effects of the anti-human TIM-3/CD39 bispecific antibodies of the present invention on maturation/activation of monocyte-derived dendritic cells (MDDCs) were evaluated.

The subject antibodies that were used in this example are 6-3hIG_Hu003_Ct, P4_Hu003_Ct and Ch072_P4_Ct as bispecific antibodies, compared to the monospecific antibody of Hu003_LA or 6-3hIG_LA, or a combination of these two monospecific antibodies as a comparative control.

Eight days before the start of evaluation, cell fractions of CD14-positive cells separated by a magnetic bead method from PBMCs derived from healthy human volunteers were suspended in a culture medium of RPMI-1640 medium supplemented with 10% FCS, 100 units/ml penicillin, 100 micrograms/ml streptomycin, 1 × GlutaMax (Gibco, 35050-061) and 10 mM HEPES to which IL-4 (10 ng/ml) and GM-CSF (100 ng/ml) were further added. The cells were cultured for 8 days to prepare MDDCs.

On the starting day of evaluation, MDDCs differentiated from the CD14-positive cells were suspended in RPMI-1640 medium supplemented with 10% FCS, 100 units/ml penicillin, 100 micrograms/ml streptomycin, 1 × GlutaMax (Gibco, 35050-061) and 10 mM HEPES, and the cells were seeded in 96-well U-bottom plates (2.5 × 10⁴ cells/well), to which polyICLC (Oncovir Inc.) at a final concentration of 1 microgram/ml and a subject antibody at a final concentration of 10 micrograms/ml were added. The cells were cultured at 37°C under 5% CO₂ for 24 hours, and then were stained with staining antibodies (Biolegend, anti-CD1a: HI149, anti-CD14: HCD14, anti-HLADR: L243, anti-CD86: 2331, anti-CD83: HB15e).

Flow cytometry analysis was performed on the stained cells, and the expression levels of HLA-DR, CD86 and CD83 molecules of CD14-negative and CD1a-positive fractions were analyzed. The concentrations of TNFalpha, IL-1beta, IL-6, CXCL9, CXCL10 and CXCL11 in the collected culture supernatants were quantified by Luminex.

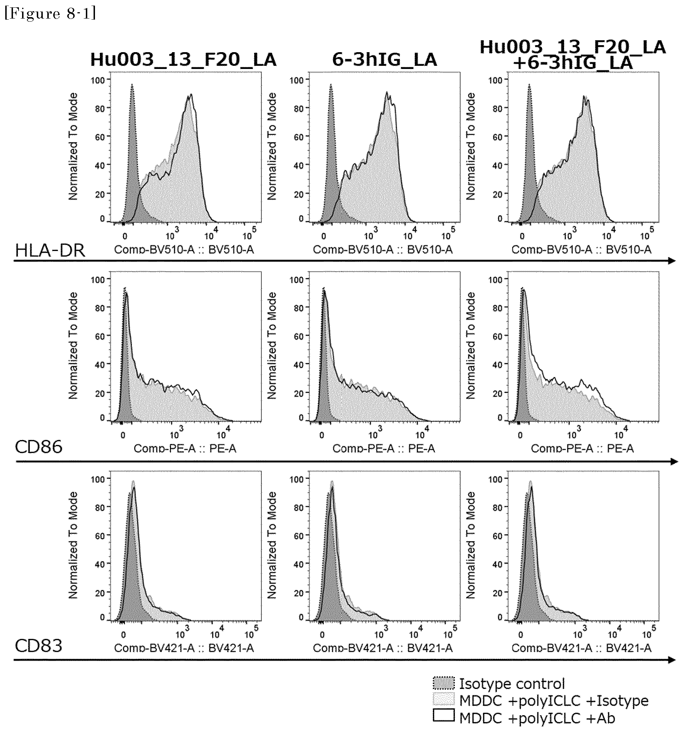

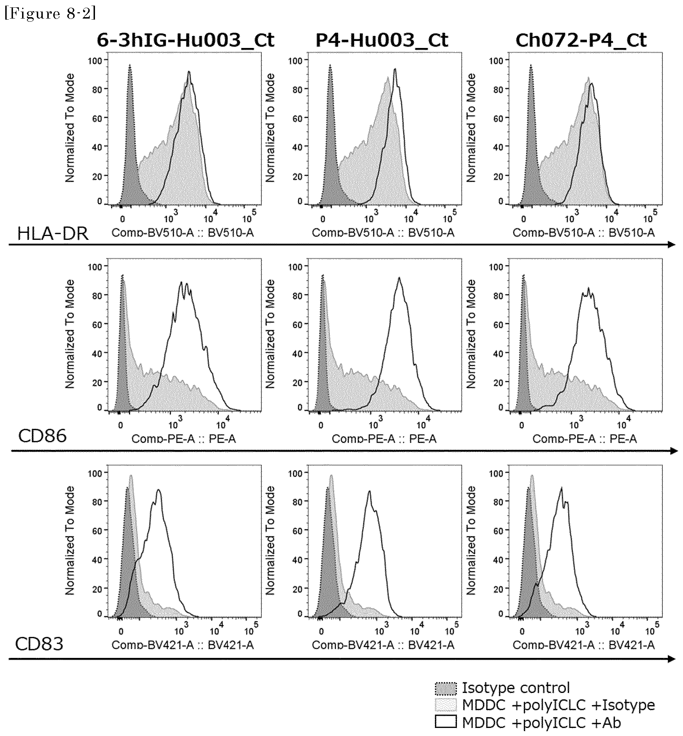

After addition of the antibodies including the subject antibodies and the control antibodies followed by incubation for 24 hours, the results of analyzing the expression distributions of HLA-DR, CD86 and CD83 molecules of CD14-negative/CD1a-positive MDDCs are shown in Figures 8-1 and 8-2. The evaluated control condition when cultured with the addition of the anti-human TIM-3 monoclonal antibody (Hu003_LA), with the addition of the anti-human CD39 monoclonal antibody (6-3hIG_LA), or with the addition of both the anti-human TIM-3 monoclonal antibody and the anti-human CD39 monoclonal antibody (Hu003_LA and 6-3hIG_LA) exhibited no change in expression distributions of HLA-DR, CD86 and CD83 molecules as compared to the isotype antibodies, whereas all the anti-human TIM-3/CD39 bispecific antibodies enhanced expression of HLA-DR, CD86 and CD83 molecules, and exhibited a substantially monomodal expression distribution.

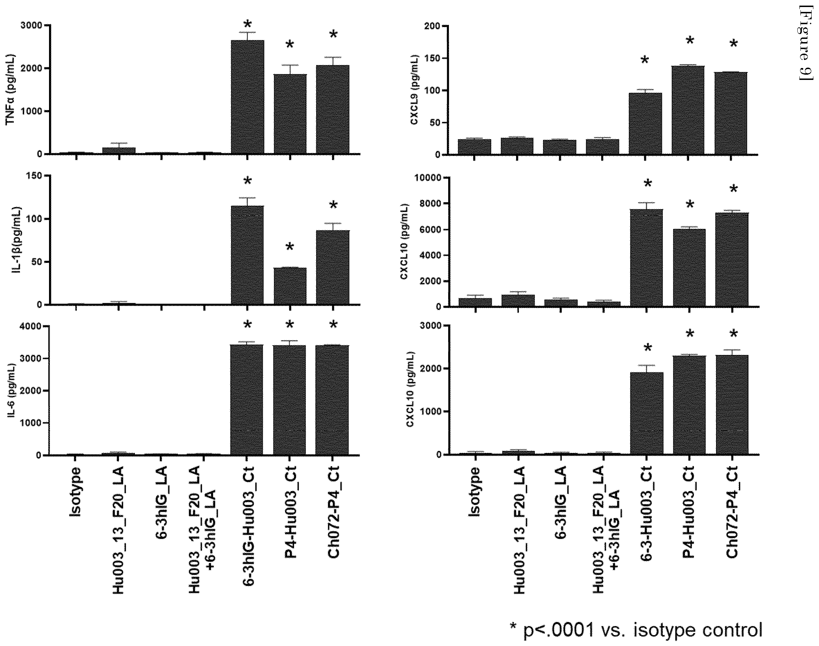

After addition of the antibodies followed by incubation for 24 hours, the results of the concentrations of TNFalpha, IL-1beta, IL-6, CXCL9, CXCL10 and CXCL11 in the culture supernatants of MDDCs measured using Luminex are shown in Figure 9. The evaluated control condition when cultured with the addition of the anti-human TIM-3 monoclonal antibody, with the addition of the anti-human CD39 monoclonal antibody, or with the addition of both the anti-human TIM-3 monoclonal antibody and the anti-human CD39 monoclonal antibody exhibited no significant effect on the concentration of any of the 7 types of measured cytokines/chemokines (TNFalpha, IL-1beta, IL-6, CXCL9, CXCL10 and CXCL11) in the culture supernatant as compared to the isotype antibodies, whereas in the condition when cultured with the addition of the anti-human TIM-3/CD39 bispecific antibody, the concentrations of these 7 types of cytokines/chemokines remarkably and significantly increased.

These analysis results showed that the anti-human TIM-3/CD39 bispecific antibodies remarkably enhanced the maturation and activation of MDDCs. These actions were shown to be unique to the anti-human TIM-3/CD39 bispecific antibodies because there were no such effects under the condition in which the anti-human TIM-3 monoclonal antibody and the anti-human CD39 monoclonal antibody were simultaneously applied to the cells.

### Example 17: Evaluation of Effects on Activation of CD8T Cells with MDDCs

In this example, the effects of the anti-human TIM-3/CD39 bispecific antibodies of the present invention on the activation of CD8T cells via MDDCs were evaluated.

The subject antibodies that were used in this example are 6-3hIG_Hu003_Ct, P4_Hu003_Ct and Ch072_P4_Ct as bispecific antibodies, compared to the monospecific antibody of Hu003_LA or 6-3hIG_LA, or a combination of these two monospecific antibodies as a comparative control as in Example 16.

Eight days before the start of evaluation, CD14-positive cells separated by a magnetic bead method from PBMCs derived from healthy human volunteers having HLA-A*02:01 as a HLA type were suspended in a culture medium of RPMI-1640 medium supplemented with 10% FCS, 100 unit/ml penicillin, 100 micrograms/ml streptomycin, 1 x GlutaMax (Gibco, 35050-061) and 10 mM HEPES to which IL-4 (10 ng/ml) and GM-CSF (100 ng/ml) were further added. The cells were cultured for 8 days to prepare MDDCs.

On the starting day of evaluation, MDDCs differentiated in the presence of 2 micrograms/mL of a 27 mer peptide containing a HLA-selective cytomegalovirus (CMV) peptide sequence (PWQAGILARNLVPMVATVQGQNLKYQE) were cultured for 4 hours. After 4 hours, the MDDCs washed with a medium and CD8-positive T cells separated by the magnetic bead method from PBMCs derived from the same donor were mixed 1:25 on a cell number basis, and were suspended in RPMI-1640 medium supplemented with 10% FCS, 100 units/ml penicillin, 100 micrograms/ml streptomycin, 1 x GlutaMax (Gibco, 35050-061) and 10 mM HEPES. Thereafter, IL-2 at a final concentration of 20 IU/mL, polyICLC (Oncovir Company) at 1 microgram/ml and the subject antibody at a final concentration of 10 micrograms/ml were added, and the cells were cultured at 37°C under 5% CO₂ for 6 days. After completion of the culture, the cells were stained with HLA-A*02:01 CMV pp65 Tetramer-NLVPMVATV-PE (MBL, TS-0010-1C), and T cell marker staining antibodies (Biolegend, anti-CD3: UCHT1, anti-CD4: RPA-T4, anti-CD8a: HIT8a).

Flow cytometry analysis was performed on the stained cells, and the proportion of CMV antigen-specific T cells in the CD8-positive cells was calculated. The concentrations of IFNgamma, TNFalpha and IL-6 in the collected culture supernatants were quantified by Luminex.

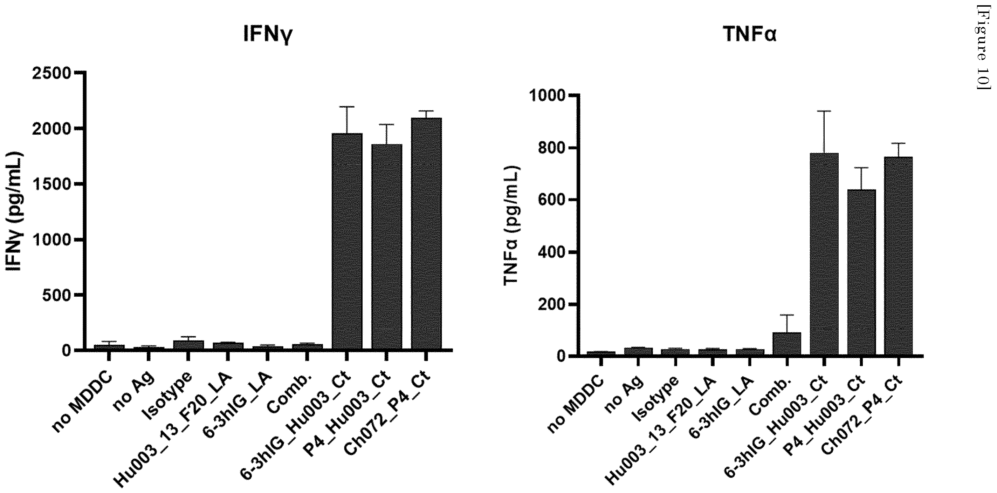

After addition of the antibodies followed by incubation for 6 days, the results of the concentrations of IFNgamma and TNFalpha in the culture supernatants of MDDCs measured using Luminex are shown in Figure 10. The evaluated control condition when cultured with the addition of the anti-human TIM-3 monoclonal antibody (Hu003_LA), with the addition of the anti-human CD39 monoclonal antibody (6-3hIG_LA), or with the addition of both the anti-human TIM-3 monoclonal antibody and the anti-human CD39 monoclonal antibody (Hu003_LA and 6-3hIG_LA) exhibited no significant effect on the concentrations of IFNgamma and TNFalpha in the culture supernatants as compared to the isotype antibodies, whereas in the condition when cultured with the addition of the anti-human TIM-3/CD39 bispecific antibody, the concentrations of IFNgamma and TNFalpha in the culture supernatants remarkably and significantly increased.

These analysis results showed that the anti-human TIM-3/CD39 bispecific antibodies remarkably enhanced the activation of CD8-positive T cells via MDDCs. The action was shown to be unique to the anti-human TIM-3/CD39 bispecific antibodies because there was no such effect under the condition in which the anti-human TIM-3 monospecific monoclonal antibody and the anti-human CD39 monospecific monoclonal antibody were simultaneously applied to the cells.

### Example 18: Evaluation of Antitumor Activity against Tumor Cell Lines by PBMCs Co-Culture

In this example, the effects of the anti-human TIM-3/CD39 bispecific antibodies of the present invention on antitumor activity in co-culture of PBMCs and tumor cells were evaluated.

The subject antibodies that were used in this example are the bispecific antibodies of Hu003_P4_Ct and 6-3hIG_Hu003_Ct, or P4_Hu003_Ct, I67_Hu003_Ct, T86_Hu003_Ct, Tre291_Hu003_Ct, mAnE67_Hu003_Ct and 6-3hIG_Hu003_Ct.

Eight days before the start of evaluation, CD14-positive cells separated by a magnetic bead method from PBMCs derived from healthy human volunteers were suspended in a culture medium RPMI-1640 medium supplemented with 10% FCS, 100 units/ml penicillin, 100 micrograms/ml streptomycin, 1 x GlutaMax (Gibco, 35050-061) and 10 mM HEPES to which IL-4 (10 ng/ml) and GM-CSF (100 ng/ml) were further added. The cells were cultured for 8 days to prepare MDDCs.

On the day before the starting day of evaluation, MDA-MB-231 strain (ATCC: HTB-26) constitutively expressing Human cytomegalovirus (strain AD169)-derived pp65 protein (accession number: P06725) and mRFP (Origene: PS100041) was suspended in RPMI-1640 medium supplemented with 10% FCS, 100 units/ml penicillin, 100 micrograms/ml streptomycin, 1 x GlutaMax (Gibco, 35050-061) and 10 mM HEPES, and was seeded in 96-well plates, and cultured at 37°C under 5% CO₂ for 1 day.

On the starting day of evaluation, PBMCs and MDDCs prepared from PBMCs of the same lot were mixed 10:1 on a cell number basis, suspended in RPMI-1640 medium supplemented with 10% FCS, 100 units/ml penicillin, 100 micrograms/ml streptomycin, 1 x GlutaMax (Gibco, 35050-061) and 10 mM HEPES, and were added to 96-well plates such that the number of PBMCs was twice the number of tumor cells seeded on the day before. To the wells containing the tumor cells and PBMCs/MDDCs, polyICLC (Oncovir Company) at a final concentration of 1 microgram/ml and the subject antibody at a final concentration of 10 micrograms/ml were added. The number of tumor cells recognized by mRFP in an Incucyte live cell analysis system (Sartorius AG) installed in CO₂ incubator set at 37°C under 5% CO₂ was recorded over time, and the antitumor activity of the subject antibodies was evaluated.

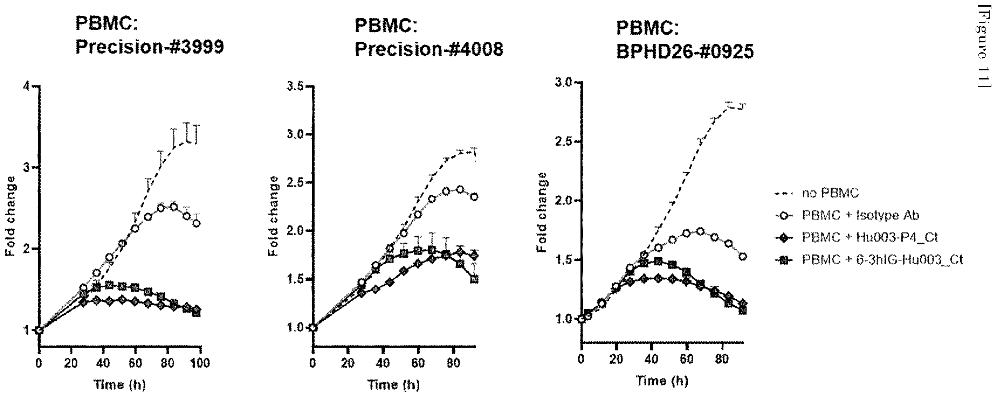

Changes in the number of tumor cells over time during the evaluation performed using the anti-human TIM-3/CD39 bispecific antibodies of P4-Hu003_Ct and 6-3_Hu003_Ct for three types of PBMCs from different donors are shown in Figure 11. In the condition when cultured with the addition of P4-Hu003_Ct and 6-3_Hu003_Ct, proliferation of tumor cells was remarkably suppressed as compared to the condition when cultured with the isotype control antibodies. Suppression of proliferation of tumor cells was observed in all the PBMCs used.

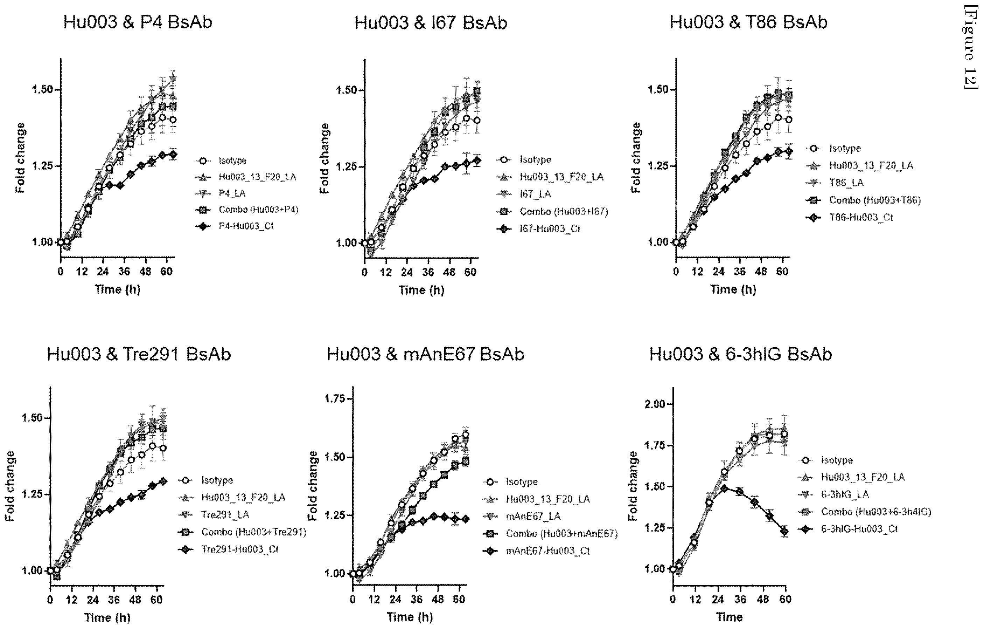

The results of performing the same analysis with six anti-human TIM-3/CD39 bispecific antibodies (P4_Hu003_Ct antibody, I67_Hu003_Ct antibody, T86_Hu003_Ct antibody, Tre291_Hu003_Ct antibody, mAnE67_Hu003_Ct antibody and 6-3hIG_Hu003_Ct antibody) are shown in Figure 12. As controls, the effects of the anti-human TIM-3 monoclonal antibody (Hu003_LA), the effects of the anti-human CD39 monoclonal antibody (P4_LA, I67_LA, T86_LA, Tre_291_LA, mAnE67_LA or 6-3hIG_LA), or the effects of both the anti-human TIM-3 monoclonal antibody and the anti-human CD39 monoclonal antibody was also evaluated.

All of the six anti-human TIM-3/CD39 bispecific antibodies evaluated were confirmed to remarkably suppress the proliferation of tumors. Any of the evaluated controls when cultured with the addition of the anti-human TIM-3 monoclonal antibody, with the addition of the anti-human CD39 monoclonal antibody or with the addition of both the anti-human TIM-3 monoclonal antibody and the anti-human CD39 monoclonal antibody did not suppress the proliferation of tumors.

These analysis results suggested that the anti-human TIM-3/CD39 bispecific antibodies remarkably enhanced the tumor proliferation suppressing action of PBMCs regardless of the donor of the cells. The action was shown to be unique to the anti-human TIM-3/CD39 bispecific antibodies because there was no such effect under the condition in which the anti-human TIM-3 monoclonal antibody and the anti-human CD39 monoclonal antibody were simultaneously applied to the cells.

### Example 19: Stimulation Assay with Anti-CD3/CD28 Antibody in the Presence of ATP

In this example, for evaluating the effects of the anti-human TIM-3/CD39 bispecific antibodies of the present invention on the proliferation of human T cells in tumor microenvironments, the effects of the anti-human TIM-3/CD39 bispecific antibodies of the present invention on the activation of human peripheral blood T cells in the presence of ATP at a concentration adjusted by simulating tumor microenvironments, were evaluated by examining the proliferation of T cells in PBMCs in response to stimulation with CD3/CD28 antibodies.

The subject antibodies in this example are 6-3hIG_Hu003_Fsc and HuP4_IB_Hu003_Fsc as bispecific antibodies, compared to one or two antibodies from the monospecific antibodies of Hu003_13_F20_LA, 6-3hIG_LA and HuP4_IB_LA which form the bispecific antibody as comparative controls.

On the starting day of evaluation, PBMCs derived from two healthy human volunteers were suspended in X-VIVO 15 medium, and seeded in 96-well U-bottom plates (4 x 10⁴ cells/well), to which the subject antibodies were added at a final concentration of 10 micrograms/ml. After culturing the cells at 37°C under 5% CO₂ for 30 minutes, ATP was added at a final concentration of 500 micro M, Dynabeads Human T-Activator CD3/CD28 (VERITAS Corporation) was further added at 1 microliter per well. The cells were cultured at 37°C under 5% CO₂ for 96 hours, followed by collection of cells and culture supernatants.

The cells were collected in a 96-well V-bottom plate and washed once with 200 microliters of PBS (-). To stain dead cells, 50 microliters of Zombie-NIR (Biolegend) diluted 500-1000 times in PBS (-) was added to the cell culture to incubate for 15 minutes at room temperature to stain dead cells. Thereafter, 50 microliters of Human TruStain FcX (Biolegend), diluted 20 times in PBS (-), was added to the plates which incubated for 20 minutes at room temperature. The cells were washed once with staining buffer, and T cell marker staining antibodies (Biolegend, anti-CD3: UCHT1, anti-CD4: RPA-T4, anti-CD8a: HIT8a) which were diluted as appropriate with staining buffer, were added at 100 microliters per well. The cells were left to stand in the dark at 4°C for 30 minutes, and then washed twice with staining buffer, and were again suspended in washing buffer, to which counting beads (ThermoFisher Scientific, C36950) were added for cell counting, which were analyzed with a flow cytometer.

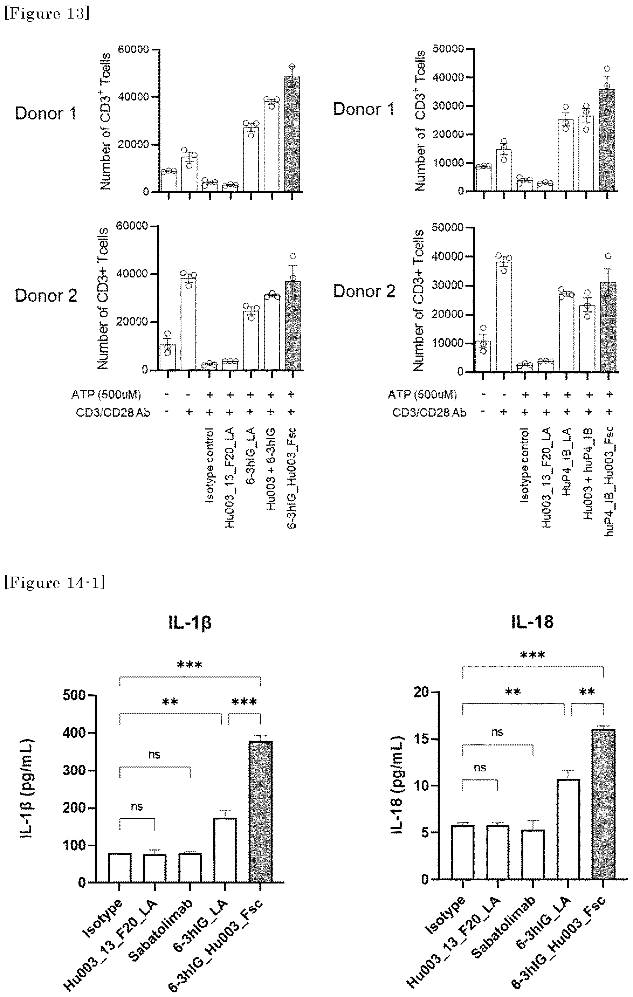

The results of flow cytometry analysis, which reveal the number of CD3-positive cells per well, are shown in Figure 13. Under the condition in which ATP was not added, the number of CD3-positive T cells in the PBMCs was increased by addition of Dynabeads Human T-Activator CD3/CD28, whereas in the presence of 500 micro M ATP, the number of CD3 cells noticeably decreased in the conditions when cultured with the addition of the isotype control antibody (CN2_LA) and the anti-TIM-3 antibody (Hu003-LA). On the other hand, in the conditions when cultured with the addition of the bispecific antibody of 6-3hIG_Hu003_Fsc or HuP4_IB_Hu003_Fsc, the number of CD3-positive T cells in PBMCs remarkably increased. The bispecific antibodies exhibited a higher proliferation enhancing effect on CD3-positive T cells compared to the combined application of two single antibodies from which each half molecule of the bispecific antibodies are derived.

These analysis results suggested that, even in tumor microenvironments, the anti-human TIM-3/CD39 bispecific antibodies exhibit a higher effect as compared to a case where human TIM-3 and human CD39 are inhibited by separate antibodies.

### Example 20: Evaluation of Ability to Activate Inflammasome

In this example, the ability of the bispecific antibodies of the present invention to enhance activation of inflammasome required to stimulate antitumor T cell immunity was evaluated.

The test antibodies in this example are 6-3hIG_Hu003_Fsc as a bispecific antibody, compared to the monospecific antibodies of Hu003_13_F20_LA and 6-3hIG_LA, from which the bispecific antibody was derived, as comparative controls.

First, for evaluating the ability to activate inflammasome in macrophage, 6 days before the start of evaluation, CD14-positive cells separated by a magnetic bead method from PBMCs derived from healthy human volunteers were suspended in a culture medium of RPMI-1640 medium supplemented with 10% FCS, 100 units/ml penicillin, 100 micrograms/ml streptomycin, 1 x GlutaMax (Gibco, 35050-061) and 10 mM HEPES to which GM-CSF (400 ng/ml) was further added. The cells were cultured for 6 days to prepare M1-like macrophage.

On the starting day of evaluation, the M1-like macrophage differentiated from the CD14-positive cells were suspended in X-VIVO 15 medium, and seeded in 96-well U bottom plates (1 x 10⁵ cells/well), to which the subject antibodies were added at a final concentration of 10 micrograms/ml. After culturing the cells at 37°C under 5% CO₂ for 1 hour, LPS was added at a final concentration of 10 ng/mL. After culturing the cells at 37°C under 5% CO₂ for 3 hours, ATP was added at a final concentration of 1 mM, and after further culturing the cells at 37°C under 5% CO₂ for 3 hours, the culture supernatants were collected, and the concentrations of IL-1beta and IL-18 were measured by the Luminex method.

The results of measuring the concentrations of IL-1beta and IL-18 in the culture supernatants are shown in Figure 14-1. In the condition when cultured with the addition of the anti-human TIM-3 monoclonal antibody (Hu003_LA, sabatolimab), there was no significant effect on the concentrations of IL-1beta and IL-18 in the culture supernatants as compared to the condition when cultured with the addition of the isotype antibody, whereas the concentrations significantly increased in the condition when cultured with the addition of the anti-human CD39 monoclonal antibody (6-3hIG_LA). Surprisingly, in the condition when cultured with the addition of the anti-human TIM-3/CD39 bispecific antibodies, the concentrations of IL-1beta and IL-18 in the culture supernatants remarkably increased, and were significantly enhanced even with respect to the condition when cultured with the addition of the anti-human CD39 monoclonal antibody.

Next, for evaluating the ability to activate inflammasome in dendritic cells, 7 days before the start of evaluation, CD14-positive cells separated by a magnetic bead method from PBMCs derived from healthy human volunteers were suspended in a culture medium of RPMI-1640 medium supplemented with 10% FCS, 100 units/ml penicillin, 100 micrograms/ml streptomycin, 1 x GlutaMax (Gibco, 35050-061) and 10 mM HEPES to which IL-4 (10 ng/ml) and GM-CSF (100 ng/ml) were further added. The cells were cultured for 7 days to prepare MDDCs.

On the day before the starting day of evaluation, human colon cancer strain SW620 cells expressing CMV antigen were suspended in RPMI-1640 medium supplemented with 10% FCS, 100 units/ml penicillin, 100 micrograms/ml streptomycin, 1 x GlutaMax (Gibco, 35050-061) and 10 mM HEPES, and were seeded in 96-well flat-bottom plates (30,000 cells/well), and cultured overnight. On the starting day of evaluation, the culture supernatants were removed, MDDCs suspended in X-VIVO 15 medium were added at 30,000 cells per well, and the subject antibodies were added at a final concentration of 10 micrograms/ml. After culturing the cells at 37°C under 5% CO₂ for 1 hour, healthy human-derived PBMCs stimulated with CMV antigen peptide (QYDPVAALF) in advance were added at 30,000 cells per well, and then after culturing the cells overnight, the culture supernatants were collected, and the concentration of IL-1beta was measured by the Luminex method.

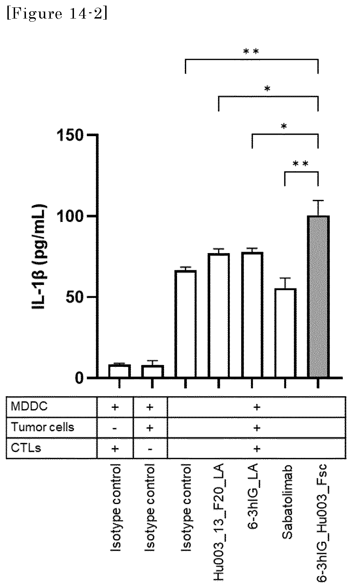

The results of measuring the concentrations of IL-1beta in the culture supernatants are shown in Figure 14-2. The conditions where the tumor cells and healthy human-derived PBMCs stimulated with CMV antigen peptide exhibited a higher concentration of IL-1beta in the culture supernatant as compared to the condition where the tumor cells were not included or where the PBMCs were not included. The condition where the tumor cells and healthy human-derived PBMCs stimulated with CMV antigen peptide, as well as the anti-human TIM-3 monospecific antibody of Hu003_13_F20_LA or the anti-human CD39 monospecific antibody of 6-3hIG_LA, exhibited a slightly higher IL-1beta concentration value as compared to the the condition where the isotype control antibody is included. On the other hand, the condition when cultured with the addition of the anti-human TIM-3/CD39 bispecific antibody of 6-3hIG_Hu003_Fsc exhibited a further higher IL-1beta concentration value, and exhibited a significantly increased concentration of IL-1beta in the culture supernatant as compared to the the conditions when cultured with the addition of the isotype control, with the addition of the anti-human TIM-3 monoclonal antibody or with the addition of the anti-human CD39 monoclonal antibody.

The series of analysis results show that, for enhancing the activation of inflammasome in M1-like macrophage or MDDC, the anti-human TIM-3/CD39 bispecific antibodies exhibited a higher effect compared to the anti-human TIM-3 monospecific antibody or the anti-human CD39 monospecific antibody.

### Industrial Applicability

The antibody or antibody derivative (antibody etc.) obtained according to the present invention has a binding ability to both TIM-3 antigen, expressed on cancer cells or immune cells, and CD39 antigen, expressed on various immune cells and non-immune cells (endothelial cells, fibroblast cells and the like), suppresses the function of TIM-3 antigen as well as the function of CD39 antigen, and thus can exhibit an effect to enhance immune activity against cancer cells, and a cancer treatment effect.

## Claims

1. A heterodimeric antibody or antibody derivative that is a combination of two different half-molecules, one of which has a binding ability to Tim-3 antigen and the other of which has a binding ability to CD39 antigen.

2. The antibody or antibody derivative according to claim 1, wherein the antibody or antibody derivative has an ability to enhance immune activity against cancer cells.

3. The antibody or antibody derivative according to claim 1 or 2, wherein the half-molecule having the binding ability to TIM-3 antigen is a half-molecule of the antibody or a half-molecule of the antibody derivative comprising either set of the complementarity-determining regions of the heavy chain and the light chain selected from the group consisting of:
(1-1) Complementarity-determining regions of the heavy chain, CDR1 (SYYMS, SEQ ID NO: 1), CDR2 (TISNSGGSTYYPDSVKD, SEQ ID NO: 2), and CDR3 (DPYYTNYVPMDY, SEQ ID NO: 3, and
complementarity-determining regions of the light chain, CDR1 (KASQYVDTYVA, SEQ ID NO: 4), CDR2 (SASTRHT, SEQ ID NO: 5), and CDR3 (AQYSSSPLT, SEQ ID NO: 6);
(1-2) Complementarity-determining regions of the heavy chain, CDR1 (DYYMD, SEQ ID NO: 9), CDR2 (YIYPNKGGTSYNQKFKG, SEQ ID NO: 10), and CDR3 (SGYGNYYTMDY, SEQ ID NO: 11), and
complementarity-determining regions of the light chain, CDR1 (KASQSVGNNVA, SEQ ID NO: 12), CDR2 (YASNRYT, SEQ ID NO: 13), and CDR3 (QQHYSSPST, SEQ ID NO: 14);
(1-3) Complementarity-determining regions of the heavy chain, CDR1 (DYYMD, SEQ ID NO: 17), CDR2 (YIYPNKGGTSYNQKFKG, SEQ ID NO: 18), and CDR3 (GGYYSYYSYDY, SEQ ID NO: 19), and
complementarity-determining regions of the light chain, CDR1 (KASQSVGNNVA, SEQ ID NO: 20), CDR2 (YASNRYT, SEQ ID NO: 21), and CDR3 (QQHYSSPYT, SEQ ID NO: 22);
(1-4) Complementarity-determining regions of the heavy chain, CDR1 (DYYMD, SEQ ID NO: 25), CDR2 (YIYPNKGGTSYNQKFKG, SEQ ID NO: 26), and CDR3 (SGYKAYYAMDY, SEQ ID NO: 27), and
complementarity-determining regions of the light chain, CDR1 (KASQSVGNNVA, SEQ ID NO: 28), CDR2 (YASNRYT, SEQ ID NO: 29), and CDR3 (QQHYSSPYT, SEQ ID NO: 30);
(1-5) Complementarity-determining regions of the heavy chain, CDR1 (SYYMS, SEQ ID NO: 33), CDR2 (TISNSGGSTYYPDSVKD, SEQ ID NO: 34), and CDR3 (DPYYTNYVPMDY, SEQ ID NO: 35), and
complementarity-determining regions of the light chain, CDR1 (KASENVGTYVS, SEQ ID NO: 36), CDR2 (GASNRYT, SEQ ID NO: 37), and CDR3 (GQSYSYPLT, SEQ ID NO: 38).

4. The antibody or antibody derivative according to claim 1 or 2, wherein the half-molecule having the binding ability to CD39 antigen is a half-molecule of the antibody or a half-molecule of the antibody derivative comprising either set of the complementarity-determining regions of the heavy chain and the light chain selected from the group consisting of:
(2-1) Complementarity-determining regions of the heavy chain, CDR1 (GFSIKDTY, SEQ ID NO: 41), CDR2 (IDPANVNT, SEQ ID NO: 42), and CDR3 (ALYGYDDDAYYFDY, SEQ ID NO: 43), and
complementarity-determining regions of the light chain, CDR1 (ESVDNYGISF, SEQ ID NO: 44), CDR2 (AAS, SEQ ID NO: 45), and CDR3 (QQSKEVPYT, SEQ ID NO: 46);
(2-2) Complementarity-determining regions of the heavy chain, CDR1 (GYSFTDYN, SEQ ID NO: 49), CDR2 (IDPYSGGT, SEQ ID NO: 50), and CDR3 (GLYGYDDDANYFDD, SEQ ID NO: 51), and
complementarity-determining regions of the light chain, CDR1 (SSVSY, SEQ ID NO: 52), CDR2 (ATS, SEQ ID NO: 53), and CDR3 (QQRSSYPLT, SEQ ID NO: 54);
(2-3) Complementarity-determining regions of the heavy chain, CDR1 (NYGVH, SEQ ID NO: 57), CDR2 (VILRRGSTDYNAAFMS, SEQ ID NO: 58), and CDR3 (TAVVAGDYFDY, SEQ ID NO: 59), and
complementarity-determining regions of the light chain, CDR1 (KASQNVGTNVA, SEQ ID NO: 60), CDR2 (SASYRYS, SEQ ID NO: 61), and CDR3 (QQYNSYPLT, SEQ ID NO: 62);
(2-4) Complementarity-determining regions of the heavy chain, CDR1 (NYGMN, SEQ ID NO: 65), CDR2 (WINTYTGEPTYADDFKG, SEQ ID NO: 66), and CDR3 (KGYYGYPNYYAMDY, SEQ ID NO: 67), and
complementarity-determining regions of the light chain, CDR1 (KASQNVGTAVA, SEQ ID NO: 68), CDR2 (SASNRYT, SEQ ID NO: 69), and CDR3 (QQYSSYPIT, SEQ ID NO: 70);
(2-5) Complementarity-determining regions of the heavy chain, CDR1 (SYGVH, SEQ ID NO: 73), CDR2 (VIWRRGSTDYNAAFMS, SEQ ID NO: 74), and CDR3 (GISTATSWFAY, SEQ ID NO: 75), and
complementarity-determining regions of the light chain, CDR1 (KASQNVGTNVA, SEQ ID NO: 76), CDR2 (SASYRYS, SEQ ID NO: 77), and CDR3 (QQYNSYPLT, SEQ ID NO: 78);
(2-6) Complementarity-determining regions of the heavy chain, CDR1 (SYGVH, SEQ ID NO: 81), CDR2 (VIWRRGSTDYNAAFMS, SEQ ID NO: 82), and CDR3 (VRGDAMDY, SEQ ID NO: 83), and
complementarity-determining regions of the light chain, CDR1 (KASQNVGTNVA, SEQ ID NO: 84), CDR2 (SASYRYS, SEQ ID NO: 85), and CDR3 (QQYNSYPLT, SEQ ID NO: 86);
(2-7) Complementarity-determining regions of the heavy chain, CDR1 (DYGMH, SEQ ID NO: 89), CDR2 (YISSGSSIIYYADTVKG, SEQ ID NO: 90), and CDR3 (KDYPYAMDY, SEQ ID NO: 91), and
complementarity-determining regions of the light chain, CDR1 (KASQNVGTNVA, SEQ ID NO: 92), CDR2 (WASNRFT, SEQ ID NO: 93), and CDR3 (QQYSSSPYT, SEQ ID NO: 94).

5. The antibody or antibody derivative according to claim 3, wherein the half-molecule having the binding ability to TIM-3 antigen is the half-molecule of (1-1) or (1-5).

6. The antibody or antibody derivative according to claim 4, wherein the half-molecule having the binding ability to CD39 antigen is any one of the half-molecules of (2-1), and (2-3)-(2-7).

7. The antibody or antibody derivative according to claim 1 or 2, wherein the amino acid sequence of the heavy chain variable region VH domain of the half-molecule having the binding ability to TIM-3 antigen is selected from the group consisting of:
(VH-1-1) the amino acid sequence of SEQ ID No: 7, or an amino acid sequence comprising one or a few amino acid substitutions (e.g., conservative substitutions), insertions, or deletions in regions of the amino acid sequence of SEQ ID No: 7 other than CDR1 (SEQ ID No: 1), CDR2 (SEQ ID No: 2), and CDR3 (SEQ ID No: 3);
(VH-1-2) the amino acid sequence of SEQ ID No: 15, or an amino acid sequence comprising one or a few amino acid substitutions (e.g., conservative substitutions), insertions, or deletions in regions of the amino acid sequence of SEQ ID No: 15 other than CDR1 (SEQ ID NO: 9), CDR2 (SEQ ID NO: 10), and CDR3 (SEQ ID NO: 11);
(VH-1-3) the amino acid sequence of SEQ ID No: 23, or an amino acid sequence comprising one or a few amino acid substitutions (e.g., conservative substitutions), insertions, or deletions in regions of the amino acid sequence of SEQ ID No: 23 other than CDR1 (SEQ ID NO: 17), CDR2 (SEQ ID NO: 18), and CDR3 (SEQ ID NO: 19);
(VH-1-4) the amino acid sequence of SEQ ID No: 31, or an amino acid sequence comprising one or a few amino acid substitutions (e.g., conservative substitutions), insertions, or deletions in regions of the amino acid sequence of SEQ ID No: 31 other than CDR1 (SEQ ID NO: 25), CDR2 (SEQ ID NO: 26), and CDR3 (SEQ ID NO: 27);
(VH-1-5) the amino acid sequence of SEQ ID No: 39, or an amino acid sequence comprising one or a few amino acid substitutions (e.g., conservative substitutions), insertions, or deletions in regions of the amino acid sequence of SEQ ID No: 39 other than CDR1 (SEQ ID NO: 33), CDR2 (SEQ ID NO: 34), and CDR3 (SEQ ID NO: 35).

8. The antibody or antibody derivative according to claim 1 or 2, wherein the amino acid sequence of the light chain variable region VL domain of the half-molecule having the binding ability to TIM-3 is selected from the group consisting of;
(VL-1-1) the amino acid sequence of SEQ ID No: 8, or an amino acid sequence comprising one or a few amino acid substitutions (e.g., conservative substitutions), insertions, or deletions in regions the amino acid sequence of SEQ ID No: 8 other than CDR1 (SEQ ID No: 4), CDR2 (SEQ ID No: 5), and CDR3 (SEQ ID No: 6);
(VL-1-2) the amino acid sequence of SEQ ID No: 16, or an amino acid sequence comprising one or a few amino acid substitutions (e.g., conservative substitutions), insertions, or deletions in regions the amino acid sequence of SEQ ID No: 16 other than CDR1 (SEQ ID NO: 12), CDR2 (SEQ ID NO: 13), and CDR3 (SEQ ID NO: 14);
(VL-1-3) the amino acid sequence of SEQ ID No: 24, or an amino acid sequence comprising one or a few amino acid substitutions (e.g., conservative substitutions), insertions, or deletions in regions the amino acid sequence of SEQ ID No: 24 other than CDR1 (SEQ ID NO: 20), CDR2 (SEQ ID NO: 21), and CDR3 (SEQ ID NO: 22);
(VL-1-4) the amino acid sequence of SEQ ID No: 32, or an amino acid sequence comprising one or a few amino acid substitutions (e.g., conservative substitutions), insertions, or deletions in regions the amino acid sequence of SEQ ID No: 32 other than CDR1 (SEQ ID NO: 28), CDR2 (SEQ ID NO: 29), and CDR3 (SEQ ID NO: 30);
(VL-1-5) the amino acid sequence of SEQ ID No: 40, or an amino acid sequence comprising one or a few amino acid substitutions (e.g., conservative substitutions), insertions, or deletions in regions the amino acid sequence of SEQ ID No: 40 other than CDR1 (SEQ ID NO: 36), CDR2 (SEQ ID NO: 37), and CDR3 (SEQ ID NO: 38);

9. The antibody or antibody derivative according to claim 1 or 2, wherein the half-molecule having the binding ability to TIM-3 antigen of the antibody derivative is selected from the following variable regions of the half-molecules which constitute the single chain Fv antibodies (scFv):
(1-1) an amino acid sequence in which the heavy chain variable region of (VH-1-1) and the light chain variable region of (VL-1-1) are connected via a linker;
(1-2) an amino acid sequence in which the heavy chain variable region of (VH-1-2) and the light chain variable region of (VL-1-2) are connected via a linker;
(1-3) an amino acid sequence in which the heavy chain variable region of (VH-1-3) and the light chain variable region of (VL-1-3) are connected via a linker;
(1-4) an amino acid sequence in which the heavy chain variable region of (VH-1-4) and the light chain variable region of (VL-1-4) are connected via a linker;
(1-5) an amino acid sequence in which the heavy chain variable region of (VH-1-5) and the light chain variable region of (VL-1-5) are connected via a linker.

10. The antibody or antibody derivative according to claim 1 or 2, wherein the amino acid sequence of the heavy chain variable region VH domain of the half-molecule having the binding ability to CD39 antigen is selected from the group consisting of:
(VH-2-1) the amino acid sequence of SEQ ID No: 47, or an amino acid sequence comprising one or a few amino acid substitutions (e.g., conservative substitutions), insertions, or deletions in regions of the amino acid sequence of SEQ ID No: 47 other than CDR1 (SEQ ID NO: 41), CDR2 (SEQ ID NO: 42), and CDR3 (SEQ ID NO: 43);
(VH-2-2) the amino acid sequence of SEQ ID No: 55, or an amino acid sequence comprising one or a few amino acid substitutions (e.g., conservative substitutions), insertions, or deletions in regions of the amino acid sequence of SEQ ID No: 55 other than CDR1 (SEQ ID NO: 49), CDR2 (SEQ ID NO: 50), and CDR3 (SEQ ID NO: 51);
(VH-2-3) the amino acid sequence of SEQ ID No: 123, or an amino acid sequence comprising one or a few amino acid substitutions (e.g., conservative substitutions), insertions, or deletions in regions of the amino acid sequence of SEQ ID No: 123 other than CDR1 (SEQ ID NO: 57), CDR2 (SEQ ID NO: 58), and CDR3 (SEQ ID NO: 59);
(VH-2-4) the amino acid sequence of SEQ ID No: 71, or an amino acid sequence comprising one or a few amino acid substitutions (e.g., conservative substitutions), insertions, or deletions in regions of the amino acid sequence of SEQ ID No: 71 other than CDR1 (SEQ ID NO: 65), CDR2 (SEQ ID NO: 66), and CDR3 (SEQ ID NO: 67);
(VH-2-5) the amino acid sequence of SEQ ID No: 79, or an amino acid sequence comprising one or a few amino acid substitutions (e.g., conservative substitutions), insertions, or deletions in regions of the amino acid sequence of SEQ ID No: 79 other than CDR1 (SEQ ID NO: 73), CDR2 (SEQ ID NO: 74), and CDR3 (SEQ ID NO: 75);
(VH-2-6) the amino acid sequence of SEQ ID No: 87, or an amino acid sequence comprising one or a few amino acid substitutions (e.g., conservative substitutions), insertions, or deletions in regions of the amino acid sequence of SEQ ID No: 87 other than CDR1 (SEQ ID NO: 81), CDR2 (SEQ ID NO: 82), and CDR3 (SEQ ID NO: 83);
(VH-2-7) the amino acid sequence of SEQ ID No: 95, or an amino acid sequence comprising one or a few amino acid substitutions (e.g., conservative substitutions), insertions, or deletions in regions of the amino acid sequence of SEQ ID No: 95 other than CDR1 (SEQ ID NO: 89), CDR2 (SEQ ID NO: 90), and CDR3 (SEQ ID NO: 91).

11. The antibody or antibody derivative according to claim 1 or 2, wherein the amino acid sequence of the light chain variable region VL domain of the half-molecule having the binding ability to CD39 is selected from the group consisting of;
(VL-2-1) the amino acid sequence of SEQ ID No: 48, or an amino acid sequence comprising one or a few amino acid substitutions (e.g., conservative substitutions), insertions, or deletions in regions the amino acid sequence of SEQ ID No: 48 other than CDR1 (SEQ ID NO: 44), CDR2 (SEQ ID NO: 45), and CDR3 (SEQ ID NO: 46);
(VL-2-2) the amino acid sequence of SEQ ID No: 56, or an amino acid sequence comprising one or a few amino acid substitutions (e.g., conservative substitutions), insertions, or deletions in regions the amino acid sequence of SEQ ID No: 56 other than CDR1 (SEQ ID NO: 52), CDR2 (SEQ ID NO: 53), and CDR3 (SEQ ID NO: 54);
(VL-2-3) the amino acid sequence of SEQ ID No: 124, or an amino acid sequence comprising one or a few amino acid substitutions (e.g., conservative substitutions), insertions, or deletions in regions the amino acid sequence of SEQ ID No: 124 other than CDR1 (SEQ ID NO: 60), CDR2 (SEQ ID NO: 61), and CDR3 (SEQ ID NO: 62);
(VL-2-4) the amino acid sequence of SEQ ID No: 72, or an amino acid sequence comprising one or a few amino acid substitutions (e.g., conservative substitutions), insertions, or deletions in regions the amino acid sequence of SEQ ID No: 72 other than CDR1 (SEQ ID NO: 68), CDR2 (SEQ ID NO: 69), and CDR3 (SEQ ID NO: 70);
(VL-2-5) the amino acid sequence of SEQ ID No: 80, or an amino acid sequence comprising one or a few amino acid substitutions (e.g., conservative substitutions), insertions, or deletions in regions the amino acid sequence of SEQ ID No: 80 other than CDR1 (SEQ ID NO: 76), CDR2 (SEQ ID NO: 77), and CDR3 (SEQ ID NO: 78);
(VL-2-6) the amino acid sequence of SEQ ID No: 88, or an amino acid sequence comprising one or a few amino acid substitutions (e.g., conservative substitutions), insertions, or deletions in regions the amino acid sequence of SEQ ID No: 88 other than CDR1 (SEQ ID NO: 84), CDR2 (SEQ ID NO: 85), and CDR3 (SEQ ID NO: 86);
(VL-2-7) the amino acid sequence of SEQ ID No: 96, or an amino acid sequence comprising one or a few amino acid substitutions (e.g., conservative substitutions), insertions, or deletions in regions the amino acid sequence of SEQ ID No: 96 other than CDR1 (SEQ ID NO: 92), CDR2 (SEQ ID NO: 93), and CDR3 (SEQ ID NO: 94).

12. The antibody or antibody derivative according to claim 1 or 2, wherein the half-molecule having the binding ability to CD39 antigen of the antibody derivative is selected from the following variable regions of the half-molecules which constitute the single chain Fv antibodies (scFv):
(2-1) an amino acid sequence in which the heavy chain variable region of (VH-2-1) and the light chain variable region of (VL-2-1) are connected via a linker;
(2-2) an amino acid sequence in which the heavy chain variable region of (VH-2-2) and the light chain variable region of (VL-2-2) are connected via a linker;
(2-3) an amino acid sequence in which the heavy chain variable region of (VH-2-3) and the light chain variable region of (VL-2-3) are connected via a linker;
(2-4) an amino acid sequence in which the heavy chain variable region of (VH-2-4) and the light chain variable region of (VL-2-4) are connected via a linker;
(2-5) an amino acid sequence in which the heavy chain variable region of (VH-2-5) and the light chain variable region of (VL-2-5) are connected via a linker;
(2-6) an amino acid sequence in which the heavy chain variable region of (VH-2-6) and the light chain variable region of (VL-2-6) are connected via a linker;
(2-7) an amino acid sequence in which the heavy chain variable region of (VH-2-7) and the light chain variable region of (VL-2-7) are connected via a linker.

13. The antibody or antibody derivative according to claim 1 or 2, wherein the antibody derivative is selected from a modified antibody selected from a humanized antibody, a chimeric antibody, a single chain Fv antibody (a scFv antibody), Fab, Fab', F(ab')2, Fc effector function modified antibody, IgG1LALA, IgGl N297A, IgG4_S228P or functional fragments thereof.

14. The antibody or antibody derivative according to claim 13, wherein the enhancement of immune activity is activation of T cells selected from the group consisting of removal of immunosuppressive signals, relieving T cell exhaustion, proliferation of T cells, increase in cytotoxicity of T cells against cancer cells, promotion of T cell cytokine secretion, enhancement of tumor infiltration of activated T cells, promotion of dendritic cell (DC) maturation, enhancement of dendritic cell-mediated activation of antigen-specific T cells, and enhancement of dendritic cell-mediated tumor cytotoxic activity of T cells.

15. The antibody or antibody derivative according to claim 13, wherein the antibody or antibody derivative induces cytotoxicity against cancer cells but not against normal cells.

16. The antibody or antibody derivative according to claim 13, wherein the cancer cell is selected from the group consisting of blood cancer, melanoma, breast cancer, lung cancer, colon cancer, gastric cancer, pancreatic cancer, and liver cancer.

17. A pharmaceutical composition for treating cancer, comprising a heterodimeric antibody or antibody derivative that is a combination of two different half-molecules, one of which has a binding ability to the Tim-3 antigen and the other of which has a binding ability to the CD39 antigen.

18. The pharmaceutical composition according to claim 17, wherein the cancer is selected from the group consisting of blood cancer, melanoma, breast cancer, lung cancer, colon cancer, gastric cancer, pancreatic cancer, and liver cancer.

19. A method for measuring cytotoxicity against cancer cells, comprising
a step of bringing cancer cells collected from a subject into contact in vitro with a heterodimeric antibody or antibody derivative that is a combination of two different half-molecules, one of which has a binding ability to the Tim-3 antigen and the other of which has a binding ability to the CD39 antigen, and
a step of measuring whether the cell viability of the cancer cells is decreased or a step of measuring whether the secretion of immune-activating substances is enhanced under culture conditions.

20. The method according to claim 19, wherein whether cell viability of cancer cells is decreased or whether immune cells derived from peripheral blood lymphocytes are activated, is measured in the presence of peripheral blood lymphocytes from the same subject.

21. The method according to claim 19 or 20, wherein the enhancement of cytotoxicity against the cancer cells in vitro when a heterodimeric antibody or antibody derivative that is a combination of two different half-molecules, one of which has a binding ability to the Tim-3 antigen and the other of which has a binding ability to the CD39 antigen is administered to the subject is measured based on the in vitro cytotoxicity against cancer cells collected from the subject.

22. The method according to claim 19 or 20, wherein the enhancement of cytotoxicity against cancer cells when a heterodimeric antibody or antibody derivative that is a combination of two different half-molecules, one of which has a binding ability to the Tim-3 antigen and the other of which has a binding ability to the CD39 antigen is administered to the subject is measured based on the enhancement of secretion of an immune activating substance in vitro.

23. A measurement kit comprising a heterodimeric antibody or antibody derivative that is a combination of two different half-molecules, one of which has a binding ability to the Tim-3 antigen and the other of which has a binding ability to the CD39 antigen, for measuring in vitro cytotoxicity against cancer cells from a subject, secretion of immune activating substances against the cancer cells, or activation of immune cells against cancer cells of the antibody or antibody derivative.
